# EUROPEAN PATENT APPLICATION

(11) **EP 4 847 041 A2**
(43) Date of publication of application: **02.12.2026**
(21) Application number: 26210097.7
(22) Date of filing: 15.04.2020
(51) Int. Cl.: A61K 39/395

(54) **INTEGRIN ALPHA10 AND AGGRESSIVE CANCER FORMS**

(30) Priority: 15.04.2019 EP 19169233
(62) Document number(s) of the earlier application(s) in accordance with Art. 76 EPC: 20718667.7 / 3 956 360
(71) Applicant: Targinta AB, 22381 Lund (SE)
(72) Inventor: LUNDGREN ÅKERLUND, Evy, Bjärred (SE); CHMIELARSKA MASOUMI, Katarzyna, Lund (SE); HUANG, Xiaoli, Arlöv (SE); THORÉN, Matilda, Ramlösa (SE)
(74) Representative: Holm, Peter Joakim

(57) **Abstract**

The present invention relates to a composition for treatment of aggressive cancer forms including aggressive breast cancer, aggressive lung cancer, aggressive prostate cancer and aggressive pancreatic cancer, as well as metastatic cancer, comprising an antibody specifically binding to an integrin alpha10 polypeptide, or a fragment thereof. The present invention also relates to methods for diagnosis and treatment of said aggressive cancer forms. The present invention also relates to methods for predicting survival rate of individuals affected by said aggressive cancer forms.

## Description

### Technical field

The present invention relates to a composition for treatment of aggressive cancers, including aggressive breast cancer, aggressive lung cancer, aggressive prostate cancer and aggressive pancreatic cancer, as well as metastatic cancer comprising an antibody specifically binding to an integrin alpha10 polypeptide, or a fragment thereof. The present invention also relates to methods for diagnosis and treatment of said aggressive cancer forms. The present invention also relates to methods for predicting survival rate of individuals affected by said aggressive cancer forms.

### Background

Cancer is a complex disease and progresses within a dynamically evolving extracellular matrix (ECM) that controls virtually every aspect of the tumor and tumor-associated cells. The main cell adhesion receptors for components of the ECM, the integrins, are family of 24 transmembrane heterodimers generated from a combination of 18 alpha integrin and 8 beta integrin subunits. Different tissue types typically express a unique set of integrins on their cell surface. Altered integrin expression patterns have been linked to many types of cancer (Moschos et al. 2007). Alterations in integrins signaling are involved in nearly all steps of carcinogenesis, ranging from switches in the utilization of αβ heterodimers, to aberrant expression of integrins, and constitutive activation of downstream effectors of integrin signaling and interactions with other signaling pathways.

Aggressive cancer forms are tumors that proliferate quickly and/or migrate to distant sites and other tissues rapidly, hence they may be invasive tumors. Moreover, aggressive cancer forms are usually associated with a poor survival prognosis.

The most common classification of breast cancer subtypes based on histological categorization indicates that the most common histological breast cancer subtype is invasive ductal carcinoma, representing 80% of invasive breast cancers, followed by invasive lobular carcinoma, representing approximately 10% of invasive breast cancers (Xiaofeng et al. 2015). The expression (or lack of expression) of specific protein markers is associated with aggressiveness of breast cancer, in particular hormone receptor status [expression of estrogen receptor (ER) and progesterone receptor (PR)] and human epidermal growth factor receptor 2 (HER2) (Arpino et al. 2015; Hariri et al. 2019). Triple-negative breast cancer (TNBC, ER-/ PR-/HER2-), is more likely to exhibit an aggressive behavior and is associated with an unfavorable prognosis compared with other subtypes of breast cancer (Rakha et al. 2007; Malorni et al. 2012). Once metastasized, TNBC has a high predisposition to involve the critical visceral organs such as lung, liver and brain, eventually leading to a significantly shorter median overall survival than in other subtypes (Bianchini et al. 2016). Six subtypes of TNBC have been identified, basal-like (BL1 and BL2), an immunomodulatory (IM), a mesenchymal (M), a mesenchymal stem-like (MSL), and a luminal androgen receptor (LAR) subtype (Ma et al. 2018). Therefore, developing optimal therapeutic strategies for the treatment of early TNBC is crucial to alleviate the burden of TNBC. There is overlap between histological and molecular classification. For example, invasive ductal carcinoma can have different molecular expression profiles and so be triple negative, HER2 positive, luminal A or luminal B. Triple negative basal like tumors are further characterized by having: more than 50% TP53 mutated, being highly proliferative due to loss of RB1, being associated with BRCA-1 mutation, being highly aneuploid, and having a unique expression of any one of cytokeratin 5, 6 or 17. In addition to TNBC, other aggressive breast cancer forms exist. Inflammatory breast cancer (IBC) is a rare and aggressive form of breast cancer. IBC tends to grow and spread quickly, with symptoms worsening within days or even hours. Thus, the development of novel targeted therapies for aggressive types of breast cancer is needed and is of paramount importance for improving the associated survival prognosis.

Prostate cancer is the second most common cancer in men and the fourth most common cancer overall worldwide. The five-year survival for the patients diagnosed with local or regional cancers is nearly 100%. However, the patients diagnosed with distant metastasis have only 28% of five-year survival. Healthy prostate epithelium contains luminal epithelial cells, basal cells and a small component of neuroendocrine (NE) cells that are scattered throughout the prostate. The majority of the prostate cancers are classified as adenocarcinomas characterized by an absence of basal cells and uncontrolled proliferation of malignant tumor cells with features of luminal differentiation including glandular formation and the expression of androgen receptor (AR) and prostate-specific antigen (PSA). Interestingly, every single case of prostatic adenocarcinoma also contains a small population (usually ~1%) of NE tumor cells. The NE cells in adenocarcinoma share many important features with those in the benign prostate. For example, in contrast to the non-NE luminal-type tumor cells, the NE cells in benign prostate and adenocarcinoma do not express AR and PSA (Einstein et al. 2019). A minority of the prostatic epithelial malignancies are variant forms including ductal type adenocarcinoma, mucinous (colloid) carcinoma, signet ring cell carcinoma, and small cell (neuroendocrine) carcinoma (SCNC). Similar to the NE cells in benign prostate and prostatic adenocarcinoma, the tumor cells in SCNC lack the expression of AR and PSA, which explains the clinical observation that such tumors, unlike adenocarcinomas, do not respond to hormonal therapy that stops androgen production and inhibits AR function. In contrast to the majority of prostatic, SCNC is highly aggressive, usually presenting with locally advanced disease or distant metastasis, and the patients usually die within months of the diagnosis (Saad et al. 2019).

Lung cancer is the leading cause of cancer deaths worldwide in both men and women (GLOBOCAN 2018). The 5-year survival rate for patients with localized tumors in the lung is 56%. However, most patients are diagnosed at a later stage and then the 5-year survival rate for patients with distant tumors is only 5% (SEER Cancer Statistics Review, 2015). Histologically lung cancer is divided into two main types, small cell lung cancer (SCLC) and non-small cell lung cancer (NSCLC). The majority of the tumors are classified as NSCLC, about 85%, compared to 15% of the tumors that are classified as SCLC. NSCLC includes a heterogeneous group of tumors, and is further subdivided into three major histological subtypes; adenocarcinoma, squamous cell carcinoma and large cell carcinoma. Adenocarcinoma and squamous cell carcinoma are the main subtypes of NSCLC. NSCLC is relatively insensitive to chemotherapy and radiation therapy compared to SCLC. In recent years, more specific therapies targeting molecular subgroups have shown promising results. However, only a small percentage of the patients identified by biomarkers correlated with therapy response (Bombardelli et al. 2016). Hence, there is a need for novel biomarkers that allow early detection of NSCLC and SCLC, amongst other lung tumors, as well as novel therapeutic targets.

Pancreatic cancer can be classified into two major groups: exocrine tumors and neuroendocrine tumors. Exocrine tumors, accounting for 94% of all pancreatic cancer, start in the exocrine cells where the digestive enzymes are made. This group comprises ductal adenocarcinoma (the most common type of pancreatic cancer representing 90% of all pancreatic cancer), acinar cell carcinoma, intraductal papillary-mucinous neoplasm, and more. Neuroendocrine tumors, also called islet cell tumors, attribute about 6% of pancreatic cancer. In general, pancreatic cancer develops in a relatively symptom-free manner and is usually advanced at the time of diagnosis. Due to the late diagnosis and poor responsiveness to chemotherapy and radiation therapy, the 5-year survival rate for pancreatic cancer patients is less than 5% (Milena et al. 2016). Therefore, it is highly needed to develop new methods for diagnosis and treatment of this aggressive disease.

Sarcoma is a wide-ranging family of cancers from cells of mesenchymal origin that can differentiate in several tissue linages; adipose, muscle, fibrous, cartilage and bone. Sarcomas account for 1% of all cancer diagnoses and cancer related deaths. The prevalence is higher in childhood and adolescence, and in these groups sarcomas accounts for 19-21% of the cancer related deaths. Due to the great histological and molecular heterogeneity this group of malignancies are particularly difficult to diagnose. Compared to carcinomas these tumors are very rare and usually grow locally with invasion of adjacent tissues. With localized and early stage tumor the long-term survival is good after surgical resection, but the tumors have a high risk of recurrence even after 10 to 15 years. Current therapy strategies for soft tissue sarcomas involve surgery, radiation and chemotherapy but there are limitations due to toxicity and modest responses. The current up to 5-year survival rate of 60% is a reflection of age, tumor type, stage and histological grade, but the survival is considerably reduced to 10-17% in high risk patients with metastasizing tumors. A classification of sarcoma cancer forms is found in WHO classification of tumors of soft tissue and bone (2013).

Hence, there is a need for new tools that may allow early diagnosis of aggressive tumors such as those discussed herein above, as well as novel therapeutic targets and therapies, so that their prognosis and survival rate can be improved.

### Summary

The present inventors have surprising found that several of the most aggressive cancer forms are characterized by having a high expression of integrin alpha10 on their cell surface. The inventors have thus found that it is possible to rapidly detect the presence of an aggressive cancer form in an individual based on the expression level of integrin alpha10 in cells of the tissue suspected of being affected by cancer. Hence, a more specific diagnosis is possible at an earlier stage than with current diagnostic tools, which results in early intervention and improved prognosis for the individual under examination.

Moreover, the present inventors have identified integrin alpha10 as a novel therapeutic target for aggressive cancer forms and their metastatic forms. In fact, they have found that it is possible to block proliferation and migration of tumor cells belonging to an aggressive cancer form by contacting said cells with an antibody specifically binding to an integrin alpha10 polypeptide or fragment thereof. It is also possible to induce cell death by contacting the tumor cells with a suitable antibody drug conjugate comprising an antibody specifically binding to an integrin alpha10 polypeptide or fragment thereof. Additionally, the inventors have found that it is possible to reduce the growth of a tumor by administering, and thereby by contacting, said tumor cells with an antibody specifically binding to an integrin alpha10 polypeptide or fragment thereof.

Hence, one aspect of the present disclosure relates to an antibody or antigen-binding fragment thereof, wherein the antibody or antigen-binding fragment thereof is specifically binding to an integrin alpha10 polypeptide, for use in the treatment and/or prevention of an aggressive cancer form selected from the group consisting of aggressive breast cancer, aggressive lung cancer, aggressive prostate cancer and aggressive pancreatic cancer, or a metastasis of any one of said cancer forms.

Another aspect of the present disclosure relates to an antibody or antigen-binding fragment thereof, wherein the antibody or antigen-binding fragment thereof is specifically binding to an integrin alpha10 polypeptide, for use in the diagnosis of an aggressive cancer selected from the group consisting of aggressive breast cancer, aggressive lung cancer, aggressive prostate cancer and aggressive pancreatic cancer, or a metastasis of any one of said aggressive cancer forms.

A further aspect of the present disclosure relates to a method of treating an aggressive cancer, wherein said aggressive cancer form is selected from the group consisting of aggressive breast cancer, aggressive lung cancer, aggressive prostate cancer and aggressive pancreatic cancer, or wherein said aggressive cancer form is a metastasis, the method comprising administering a pharmaceutically effective amount of an antibody or antigen-binding fragment thereof specifically binding to an integrin alpha10 polypeptide, to a subject in the need thereof.

An even further aspect of the present disclosure relates to a method for detecting an aggressive cancer cell in a subject, said method comprising the steps of:
a. providing a tissue suspected of comprising cancer cells of the subject;
b. analyzing in the tissue the presence of an antigen comprising an integrin alpha10 polypeptide or a fragment thereof,
c. determining the expression level of the integrin alpha10 antigen, and
d. comparing said expression level determined in c. with a control level,

wherein said control level is the average expression level of the antigen observed in healthy and/or benign cells of the same tissue type as the isolated sample,
wherein an expression level of the antigen higher than the control level is indicative of the presence of an aggressive cancer form in the subject, and wherein said aggressive cancer form is selected from the group consisting of aggressive breast cancer,
aggressive lung cancer, aggressive prostate cancer and aggressive pancreatic cancer, or a metastasis of any one of said aggressive cancer forms.

A further aspect of the present disclosure relates to a method for detecting an aggressive cancer cell in a subject, said method comprising the steps of:
a. providing a tissue suspected of comprising cancer cells of the subject;
b. optionally analyzing in the tissue the presence of one or more cells having a cancer morphology,
c. analyzing in the tissue the presence of an antigen comprising an integrin alpha10 polypeptide or a fragment thereof,
d. optionally determining the expression level of the integrin alpha10 antigen,
wherein presence of one or more cells having a cancer morphology in combination with expression of integrin alpha10 antigen are indicative of the presence of an aggressive cancer form in the subject, and wherein said aggressive cancer form is selected from the group consisting of aggressive breast cancer, aggressive lung cancer, aggressive prostate cancer and aggressive pancreatic cancer, or a metastasis of any one of said aggressive cancer forms.

Another aspect of the present disclosure relates to a method for diagnosis of an aggressive cancer form in a subject, the method comprising the steps of:
a. providing a tissue suspected of comprising cancer cells of the subject;
b. analyzing in the tissue the presence of an antigen comprising an integrin alpha10 polypeptide or a fragment thereof,
c. determining the expression level of the integrin alpha10 antigen, and
d. comparing the expression level determined in c. with a control level, wherein said control level is the average expression level of the antigen; and

wherein an expression level of the antigen higher than the control level is indicative of the presence of an aggressive cancer form in a sample, wherein said aggressive cancer form is selected from the group consisting of aggressive breast cancer,
aggressive lung cancer, aggressive prostate cancer and aggressive pancreatic cancer, or a metastasis of any one of said aggressive cancer forms, or a metastasis of any one of said aggressive cancer forms,
thereby diagnosing an aggressive cancer form in a subject.

A further aspect of the present disclosure relates to a method for diagnosis of an aggressive cancer form in a subject, the method comprising the steps of:
a. providing a tissue suspected of comprising cancer cells of the subject;
b. optionally analyzing in the tissue the presence of one or more cells having a cancer morphology;
c. analyzing in the sample the presence of an antigen comprising an integrin alpha10 polypeptide or a fragment thereof,
d. optionally determining the expression level of the integrin alpha10 antigen, and

wherein presence of one or more cells having a cancer morphology in combination with expression of integrin alpha10 antigen are indicative of the presence of an aggressive cancer form in a sample, wherein said aggressive cancer form is selected from the group consisting of aggressive breast cancer, aggressive lung cancer, aggressive prostate cancer and aggressive pancreatic cancer, or a metastasis of any one of said aggressive cancer forms,
thereby diagnosing an aggressive cancer form in a subject

A further aspect of the present disclosure relates to a method for classification of a triple negative breast cancer tumor sample of a subject, said method comprising:
a. providing a breast tissue suspected of comprising cancer cells of the subject;
b. isolating breast cancer cells characterized as being ER negative, PR negative and HER2 negative;
c. determining in the isolated cells an expression level of an antigen comprising an integrin alpha10 polypeptide or a fragment thereof, and
d. comparing the expression level determined in c. to a control level,
   wherein said control level is the average expression level of the antigen observed in healthy and/or benign breast tissue;

wherein an expression level of the antigen in the breast cancer cells higher than a control level, and an expression status of ER negative, PR negative and HER2 negative are indicative of a basal-like triple negative breast cancer or a luminal triple negative breast cancer,
thereby classifying the triple negative breast cancer tumor sample as belonging to a basal-like triple negative breast cancer tumor or to a luminal triple negative breast cancer tumor.

In one embodiment, the luminal triple negative breast cancer is a luminal androgen receptor triple negative breast cancer.

A further aspect of the present disclosure relates to a method for determining a prognosis for an aggressive cancer form for a subject, the method comprising:
a. providing a cancer tumor tissue of the subject;
b. analyzing in the sample the presence of an antigen comprising an integrin alpha10 polypeptide or a fragment thereof,
c. determining the expression level of the integrin alpha10 antigen,
d. comparing the expression level determined in c. to a control level,
   wherein the control level is the average expression level of the antigen observed in healthy and/or benign tissue of the same tissue type as the sample;
e. determining an unfavorable prognosis of the aggressive cancer form when the expression level of the integrin alpha10 antigen is higher than the control level,
wherein the aggressive cancer form selected from the group consisting of aggressive breast cancer, aggressive lung cancer, aggressive prostate cancer and aggressive pancreatic cancer, or a metastasis of any one of said aggressive cancer forms.

Another aspect of the present disclosure relates to a method for determining a prognosis for an aggressive cancer form for a subject, the method comprising:
a. providing a cancer tumor tissue of the subject;
b. optionally analyzing in the tissue the presence of one or more cells having a cancer morphology;
c. analyzing in the sample the presence of an antigen comprising an integrin alpha10 polypeptide or a fragment thereof,
d. optionally determining the expression level of the integrin alpha10 antigen, and comparing the determined expression level to a control level, wherein the control level is the average expression level of the antigen observed in healthy and/or benign tissue of the same tissue type as the sample;
e. determining an unfavorable prognosis of the aggressive cancer form when one or more cells having a cancer morphology are present in the tissue in combination with expression of the integrin alpha10 antigen, and/or
   the expression level of the integrin alpha10 antigen is higher than the control level,
wherein the aggressive cancer form selected from the group consisting of aggressive breast cancer, aggressive lung cancer, aggressive prostate cancer and aggressive pancreatic cancer, or a metastasis of any one of said aggressive cancer forms.

An further aspect of the present disclosure relates to a method of preventing metastasis from a primary aggressive cancer selected from the group consisting of aggressive breast cancer, aggressive lung cancer, aggressive prostate cancer and aggressive pancreatic cancer, the method comprising administering a therapeutically effective amount of an antibody or antigen-binding fragment thereof, wherein the antibody is specific for integrin alpha10 polypeptide, to a patient in the need thereof.

A further aspect of the present disclosure relates to a method of inhibiting integrin alpha10-mediated signaling of at least one cancer cell, the method comprising contacting the at least one cancer cell with an effective amount of an antibody or antigen-binding fragment thereof specific for integrin alpha10 polypeptide, wherein the at least one cancer cell is selected from the group consisting of an aggressive breast cancer cell, an aggressive lung cancer cell, an aggressive prostate cancer cell, an aggressive pancreatic cancer cell, and a metastatic tumor cell.

An even further aspect of the present disclosure relates to a method of inhibiting cellular functions of at least one cancer cell, the method comprising contacting the at least one cancer cell with an effective amount of an antibody or antigen-binding fragment specific for integrin alpha10 polypeptide,
wherein the at least one cancer cell is selected from the group consisting of an aggressive breast cancer cell, an aggressive lung cancer cell, an aggressive prostate cancer cell, an aggressive pancreatic cancer cell, and a metastatic tumor cell.

An even further aspect of the present disclosure relates to the use of an antibody or antigen-binding fragment thereof, wherein the antibody is specific for integrin alpha10 polypeptide, for the manufacture of a medicament for the treatment and/or prevention of an aggressive cancer selected from the group consisting of aggressive breast cancer, aggressive lung cancer, aggressive prostate cancer and aggressive pancreatic cancer, or a metastasis of any one of said cancer forms.

### Description of Drawings

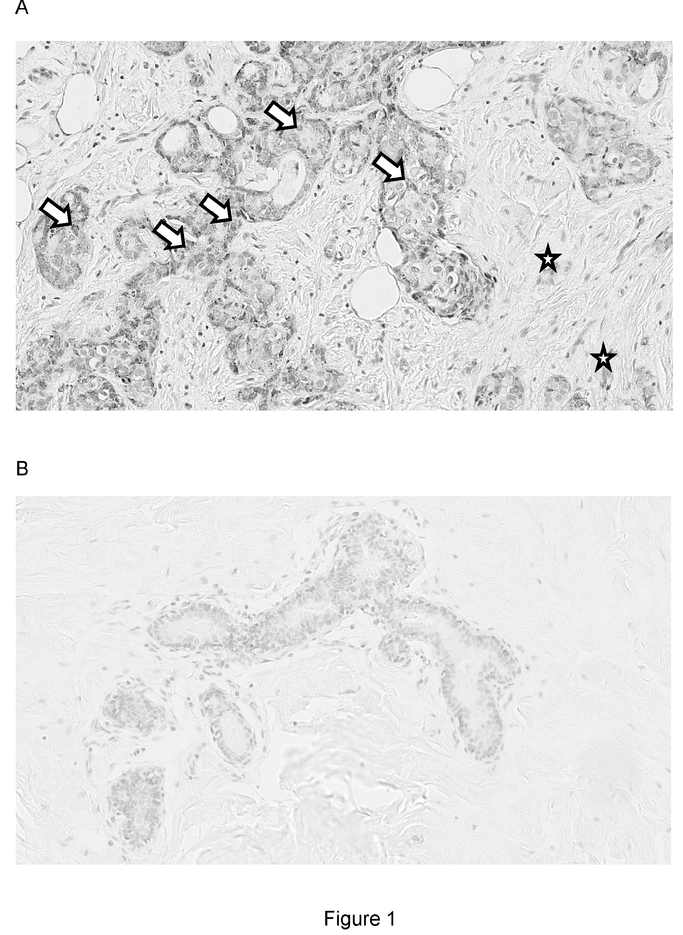

Figure 1: **Integrin alpha10 is expressed by invasive ductal carcinoma (IDC) cells in breast cancer tissue.** Immunohistochemical analysis using an antibody directed against integrin alpha10. It was shown that integrin alpha10 is specifically and strongly expressed on IDC cells (arrows) and on few cells in the stroma (stars) in breast carcinoma tissue (A). The expression of integrin alpha10 in morphologically unaffected benign breast tissue was weak (B).

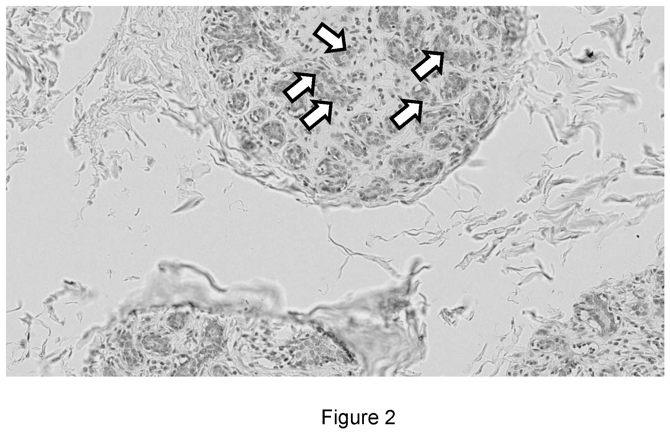

Figure 2: **Integrin alpha10 is expressed in triple negative breast cancer tumor tissue.** Immunohistochemical analysis using an antibody directed against integrin alpha10. It was shown that integrin alpha10 is specifically and strongly expressed on triple negative breast cancer cells (arrows).

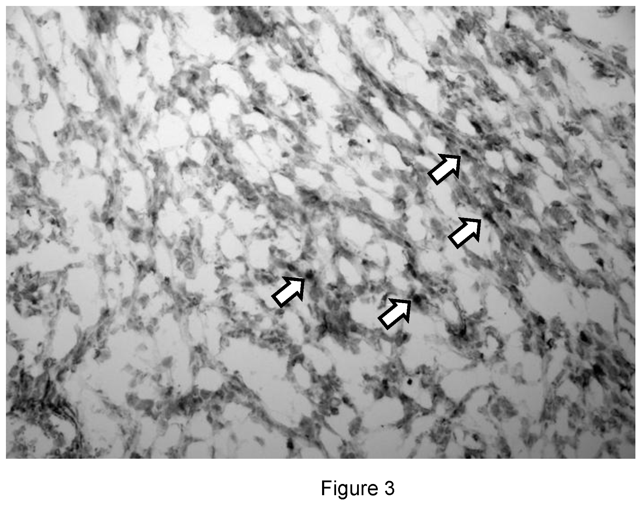

Figure 3: **Integrin alpha10 is expressed by cells in undifferentiated pleomorphic sarcoma tissue.** Immunohistochemical analysis using an antibody directed against integrin alpha10. It was shown that integrin alpha10 is specifically and strongly expressed on undifferentiated pleomorphic sarcoma cells in undifferentiated pleomorphic sarcoma tissue (arrows).

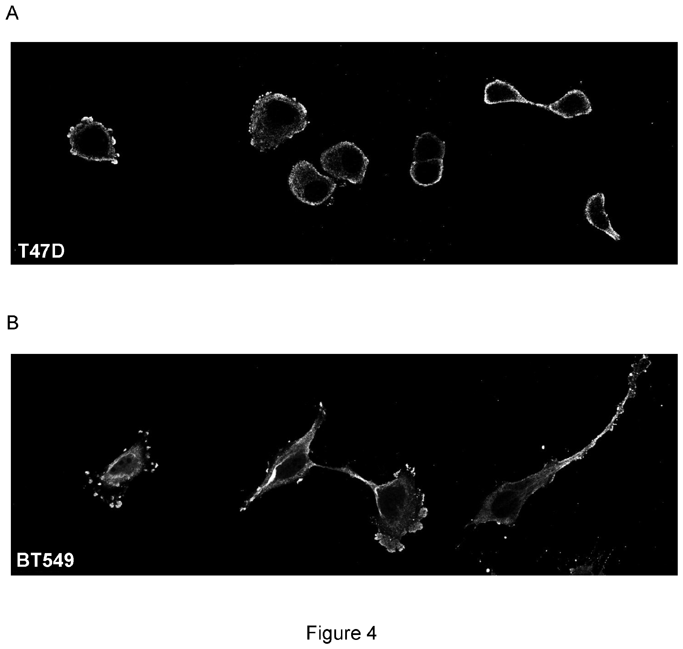

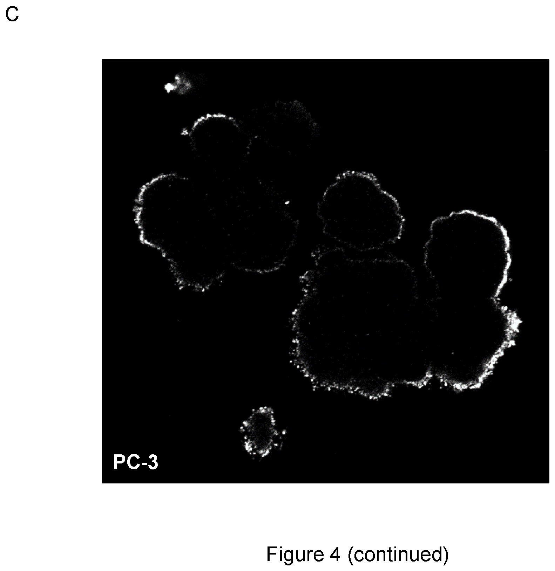

Figure 4: **Integrin alpha10 is expressed by aggressive cancer cell lines.** Immunofluorescence staining for integrin alpha10 on T47D and BT549 cells (aggressive breast cancer, monolayer culture) and in PC-3 cells (aggressive prostate cancer, sphere culture). Confocal images of T47D (A), BT549 (B) and PC-3 cells (C) showed staining of integrin alpha10 on the cell membrane.

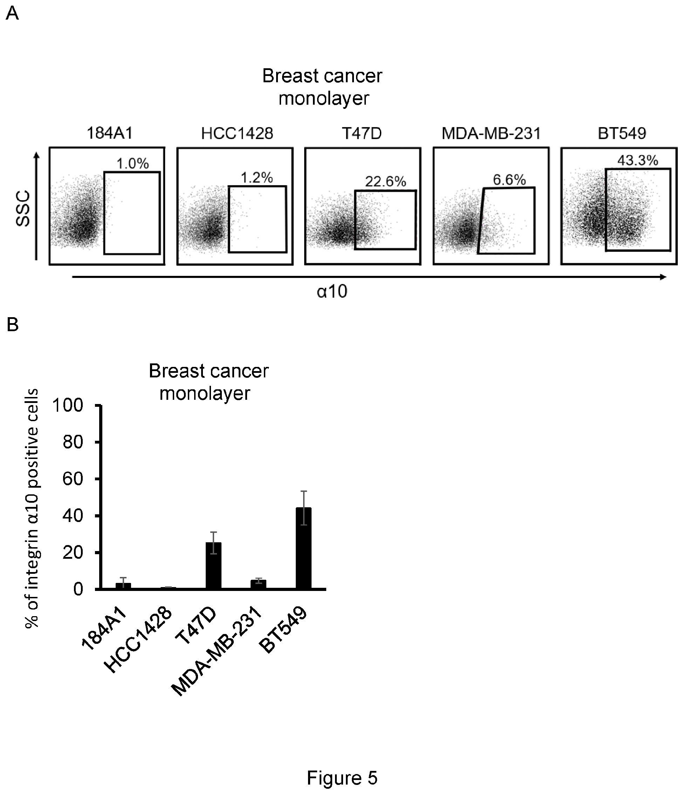

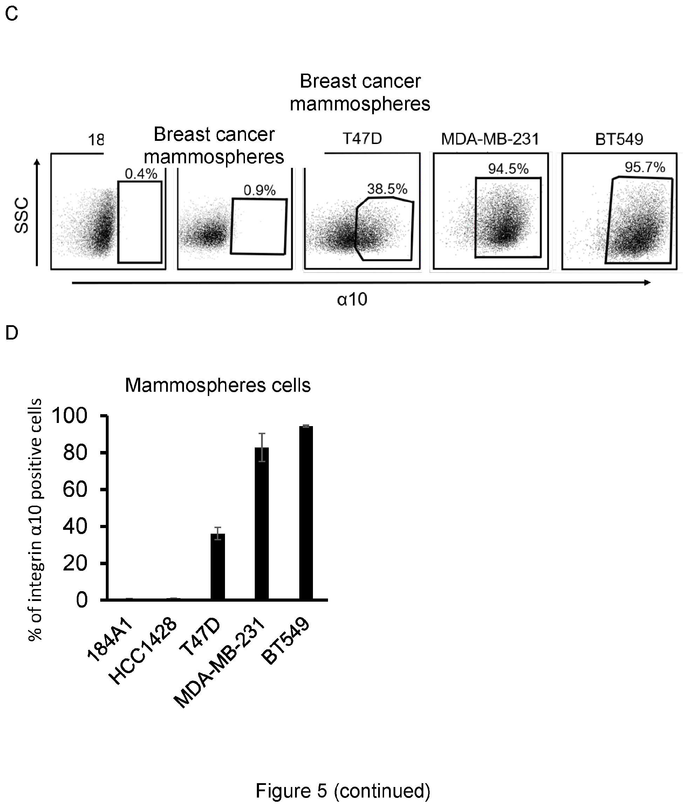

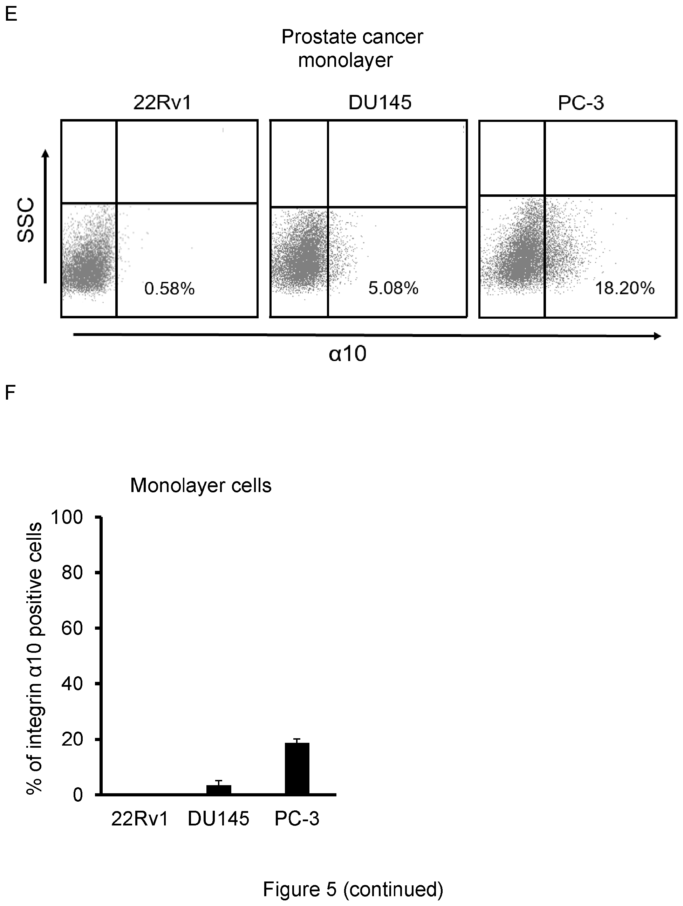

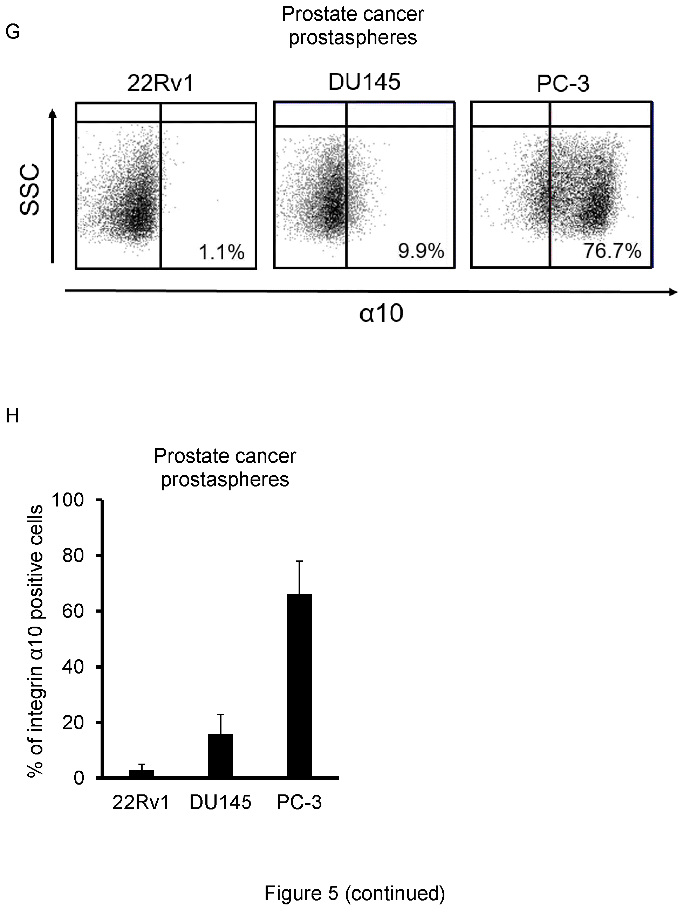

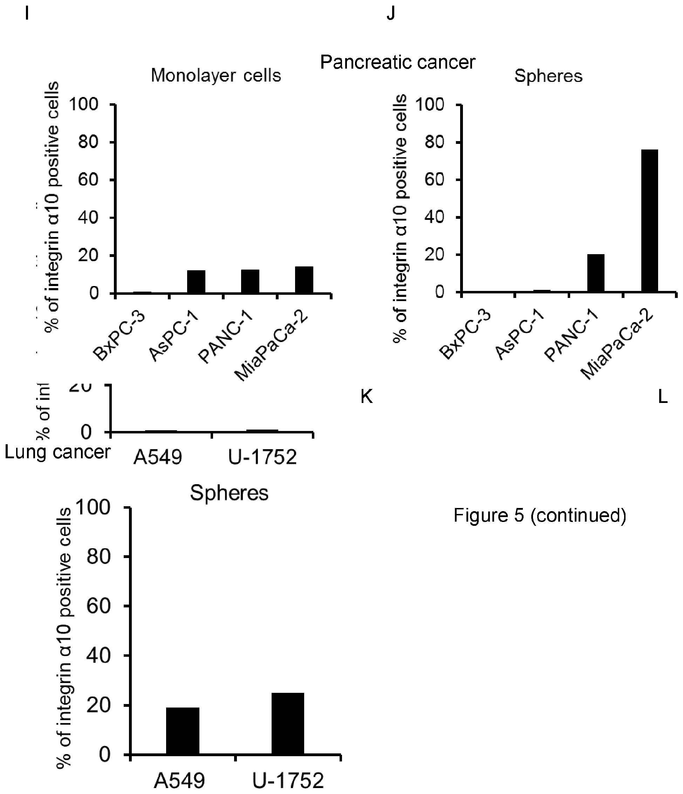

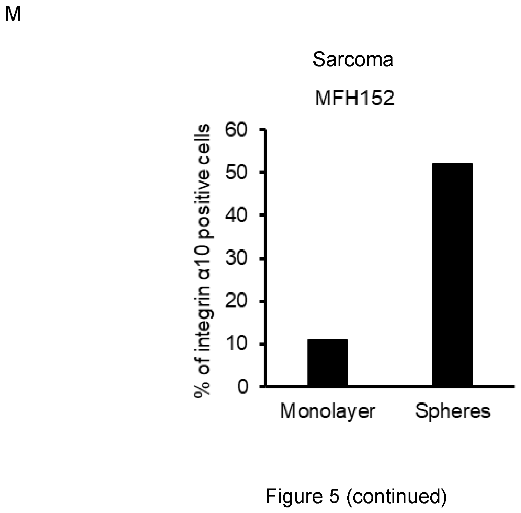

Figure 5: **Integrin alpha10 is expressed by aggressive cancer cell lines in monolayer and especially in sphere culture.**
   **A-D: Analysis of the expression of integrin alpha10 on breast cancer cells in monolayer (A and B) and in sphere-like structures (mammospheres) (C and D).** (A) As judged by flow cytometry scatter plots, the number of integrin alpha10 positive cells is high in aggressive breast cancer cell lines BT549 and T47D compared to less aggressive cancer cell lines 184A1, HCC1428 and MDA-MB-231 showing low number of integrin alpha10 positive cells. (B) Summary of the percentage of positive cells for integrin alpha10 for each cell line from n = 5 independent experiments. (C) Flow cytometry scatter plots showing that expression of integrin alpha10 is highly increased in triple negative cell lines MDA-MB-231 and BT549 cells grown as mammospheres, compared to monolayer cultures, whereas 184A1, HCC1428 and T47D showed a slightly increased or no increased expression of integrin alpha10 in comparison to the monolayer culture condition. (D) Summary of the percentage of positive cells for integrin alpha10 for each cell line from n = 5 independent experiments.
   **E-H: Analysis of the expression of integrin alpha10 on prostate cancer cells in monolayer (E and F) and in sphere-like structures (prostaspheres) (G and H).**
   The most aggressive prostate cancer cell line PC-3 showed the highest integrin alpha10 expression. The sphere culture condition drastically increased integrin alpha10 expression in PC-3 cells but not in less aggressive cell lines 22Rv1 or DU145. The representative flow cytometry plots show the proportion of integrin alpha10 positive cells in each cell line cultured as monolayer (E) and as spheres (G). Summary of the average proportion of integrin alpha10 positive cells in all prostate cancer cell lines from different experiments culture as monolayer (F) and spheres (H). Data represent mean ± SD for n ≥ 3.
   **I-J: Analysis of the expression of integrin alpha10 on pancreatic cells in monolayer (I) and in sphere-like structures (J).**
   The sphere culture condition drastically increased protein expression level of integrin alpha10 in the most invasive cell line MiaPaCa-2 cells (grade III) but not in less invasive cell lines BxPC-3 and AsPC-1 (grade II).
   **K-L: Analysis of the expression of integrin alpha10 on lung cancer cells in monolayer (K) and in sphere-like structures (L).**
   Integrin alpha10 protein expression was highly increased in aggressive lung cancer cell lines grown as spheres (K) compared to monolayer culture condition (L).
   **M: Analysis of the expression of integrin alpha10 on sarcoma cells in monolayer and in sphere-like structures (M).**
   The sphere culture condition drastically increased integrin alpha10 expression in undifferentiated pleomorphic sarcoma cancer cells.

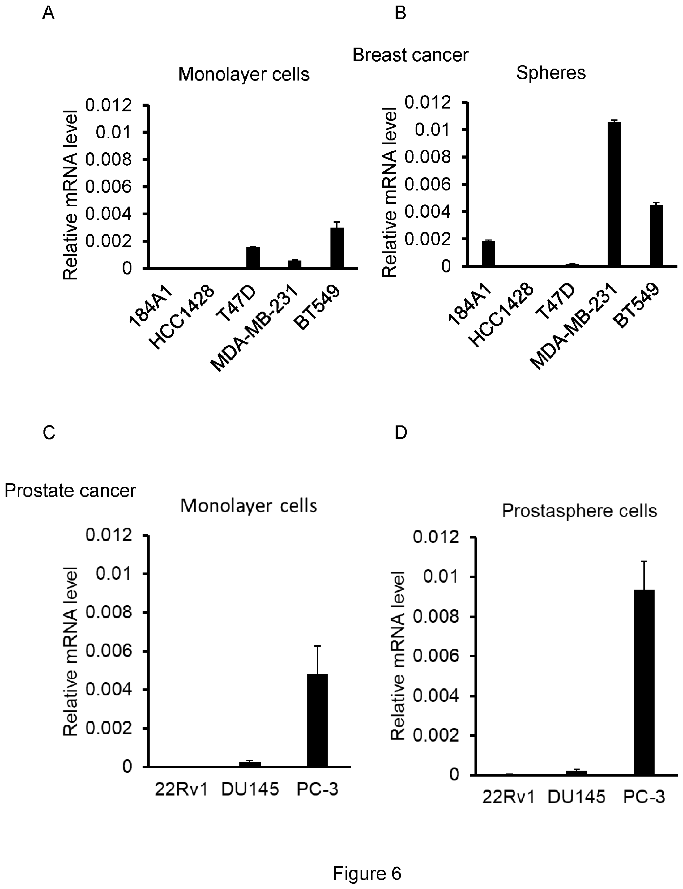

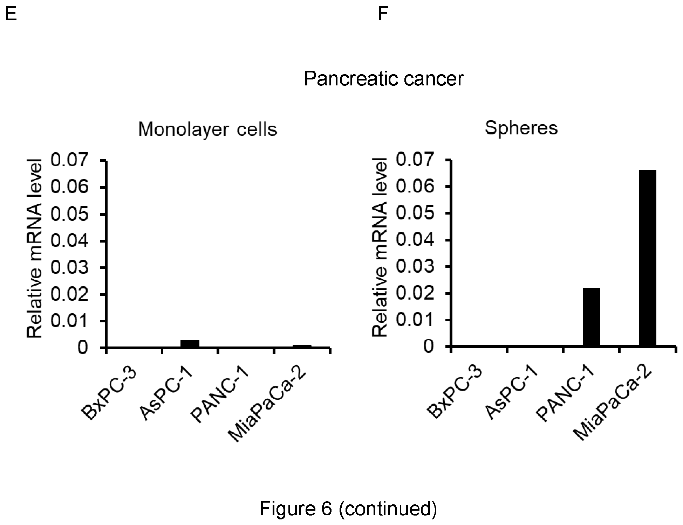

Figure 6: **Integrin alpha 10 *(ITGA10)* is expressed by aggressive cancer cell lines in monolayer and especially in three dimensional (3D) cultures (spheres) (mRNA analysis).**
   **A-B: Increased expression of integrin alpha10 in breast cancer cells cultured as mammospheres.**
   *ITGA10* expression is highest in the most aggressive cancer cell lines; e.g. triple-negative breast cancer: BT549, MDA-MB-231 and lowest in the least aggressive cell line T47D, especially when cancer cells were cultured as spheres.
   **C-D: Increased expression of integrin alpha10 in prostate cancer cells cultured as prostaspheres.**
   *ITGA10* expression is the highest in the most aggressive cell line PC-3 and lowest in the least aggressive cell line 22RV1, especially when cancer cells were cultured as spheres.
   **E-F: Increased expression of integrin alpha10 in invasive pancreatic cell line grade III.** The sphere culture condition drastically increased mRNA level of integrin alpha10 in the most invasive cell lines MiaPaCa-2 cells and PANC-1 (grade III) but not in less invasive cell line BxPC-3 and AsPC-1 (grade II).
   The data represents mean ± SD for n ≥ 3.

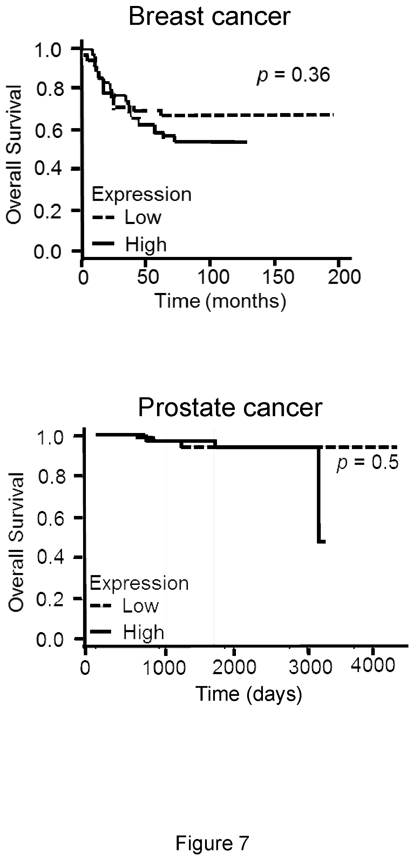

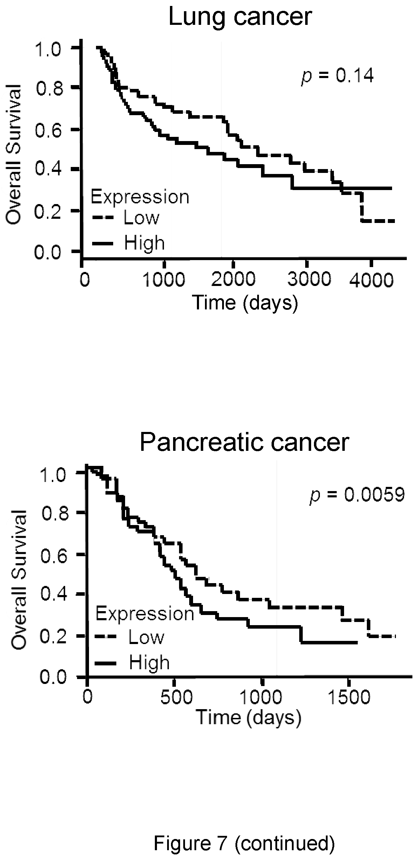

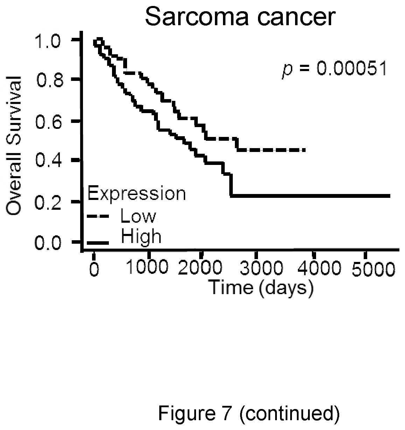

Figure 7: **High expression of *ITGA10* associates with poor overall survival in patients with different aggressive cancer indications.**
   Overall survival curves for *ITGA10* gene expression for patients with different aggressive cancers. Patients were divided into high (line) and low (dashed line) *ITGA10* expression groups based on the median cut off for the survival analysis and log-rank test. *P*-value represents log-rank testing of the difference in overall survival. For all indicated cancer forms, survival is lower in the patient group expressing high *ITGA10*, compared to the patient group expressing low *ITGA10*.

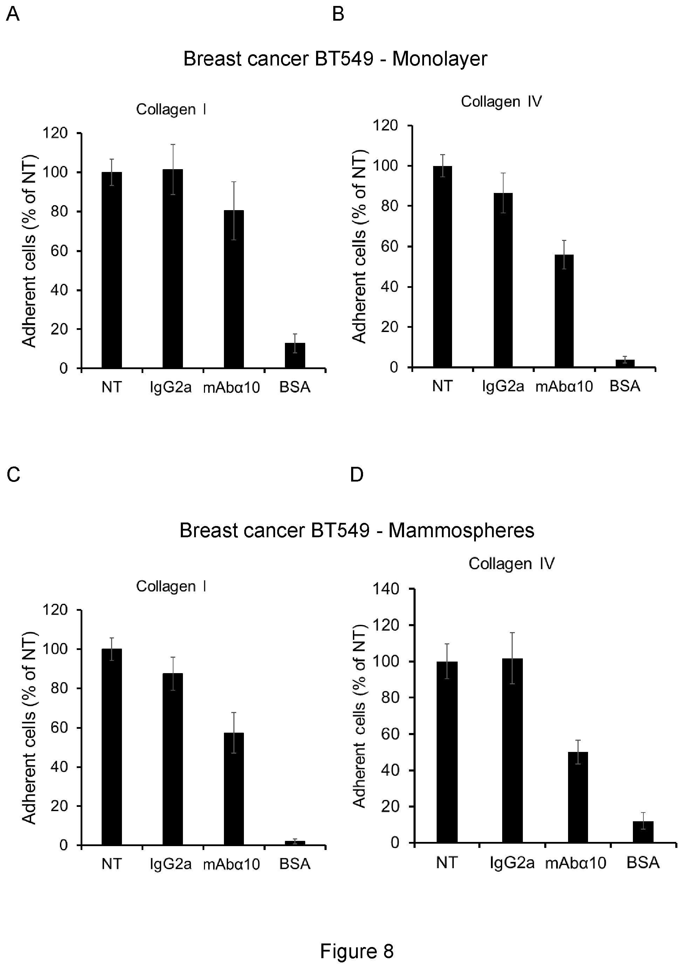

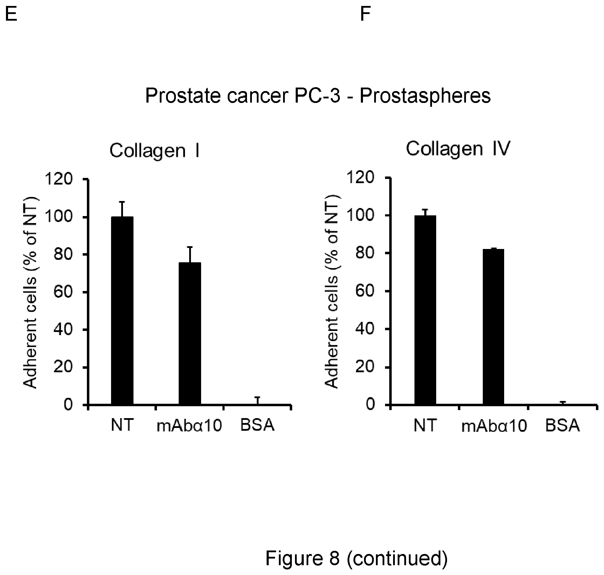

Figure 8: **Monoclonal integrin alpha10 antibody blocks adhesion of aggressive cancer cells to collagen**.
   **A-D: Triple negative breast cancer cells.** BT549 were cultured as monolayer (A and B) and as mammospheres (C and D). Cells were pre-incubated for 30 min with the monoclonal anti-integrin alpha10 blocking antibody mAbα10 (5 µg/ml) or the IgG2a isotype control antibody (5 µg/ml). Cells were then allowed to adhere to dishes coated with collagen type I (A and C), collagen type IV (B and D) or bovine serum albumin (BSA) as a control in the presence or absence of the antibodies. Data represent averages of triplicate measurements, and error bars represent the standard deviation at each data point. Cell adhesion is shown relative to the adhesion of non-treated cells (NT), which was set to 100%.
   **E-F: Aggressive prostate cancer cells.** PC-3 cells were pre-incubated for 30 min with a monoclonal anti-integrin alpha10 function blocking antibody mAbα10 (5 µg/ml). Controls were non-treated cells. Cells were then subjected to the adhesion assay and allowed to adhere to collagen I (E) and IV (F) in the presence or absence of the antibody. Data represent averages of triplicate measurements, and error bars represent the standard deviation. Cell adhesion is shown relative to the adhesion of non-treated (NT) cells, which was set to 100%.

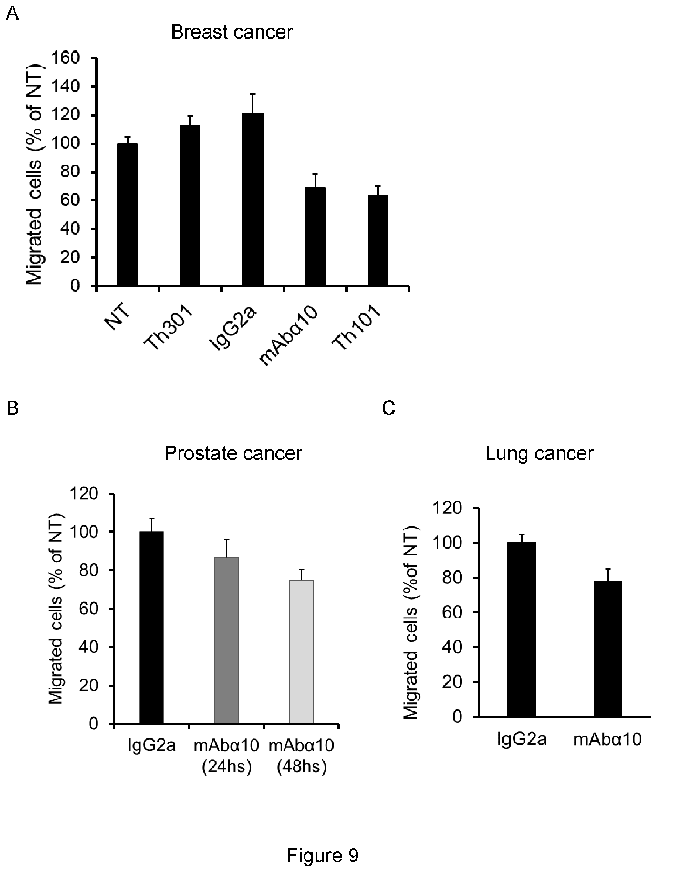

Figure 9: **Reduced migration after blocking integrin alpha10 with a monoclonal antibody**.
   **A: Breast cancer cells:** Breast cancer cells BT549 were pre-incubated for 30 min with monoclonal anti-integrin alpha10 blocking antibodies mAbα10 (mouse) or Th101 (human), or control antibodies IgG2a (mouse) or Th301 (human), both at 5 µg/ml. Cells were then subjected to a migration assay using the transwell assay, where chambers were coated with collagen type IV. Cell migration is shown relative to the migration of control cells (NT=non-treated), which was set to 100%.
   **B: Prostate cancer cells**: Decreased migration of prostate cancer cells by blocking integrin alpha10. The figure shows the effect of the anti-integrin alpha10 antibody mAbα10 on PC-3 cell migration in transwell assays. PC3 cells were seeded into transwell chambers coated with collagen type I in 24-well plates and incubated with monoclonal anti-integrin mAbα10 (5 µg/ml).
      Cell migration (assayed for 24 or 48 hours) is shown relative to the migration of cells incubated with isotope control antibody IgG2a, which was set to 100%.
   **C: Lung cancer cells:** Lung cancer cells were seeded into transwell chambers that were set into 24-well plates and incubated with monoclonal anti-integrin mAbα10. Cell migration is shown relative to the migration of cells incubated with isotope control antibody IgG2a, which was set to 100%.

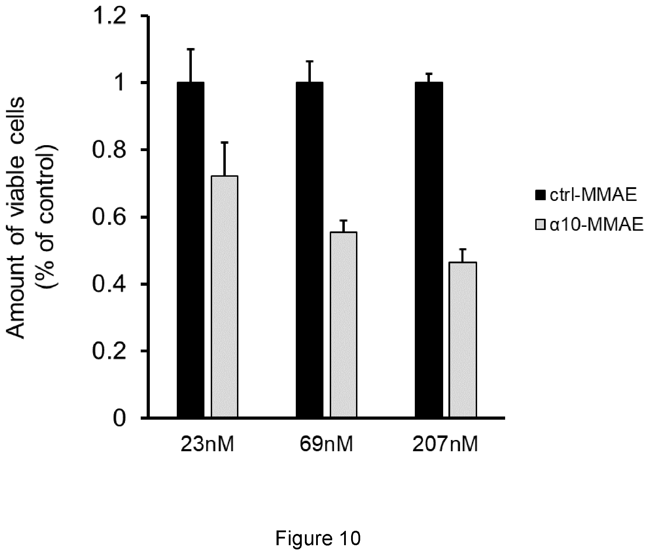

Figure 10: **Integrin alpha 10 antibody conjugated with the drug MMAE (anti alpha 10-MMAE) reduces the viability of breast cancer cells**. BT549 cells were incubated with increasing concentrations of the ADC (anti-alpha 10-MMAE) or the negative control (anti- ctrl-MMAE) for four days. Cell viability was determined by WST-1 colorimetric assay. The percentage of cell viability was calculated by comparing with the negative control anti ctrl-MMAE set to 100%. The results show that anti alpha 10-MMAE reduces cell viability on BT549.

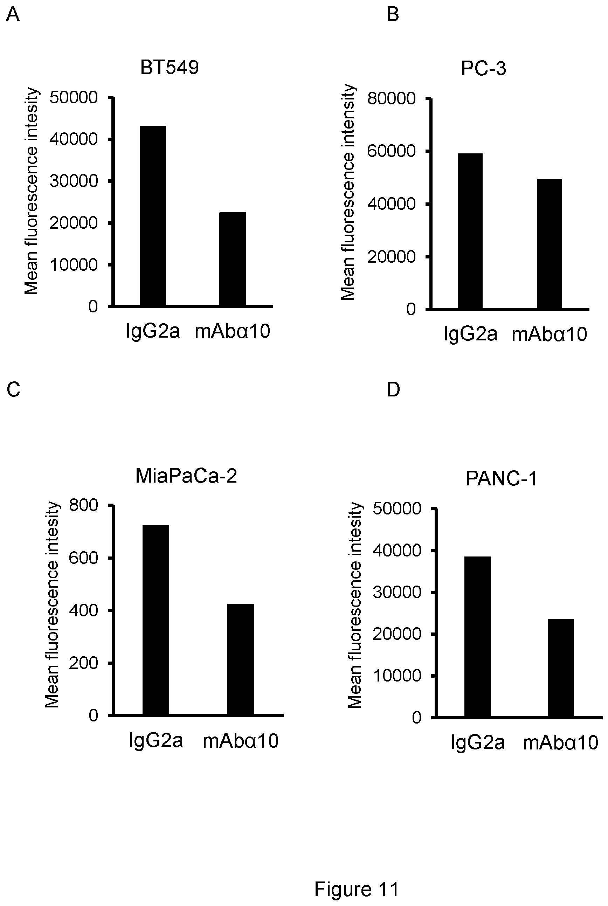

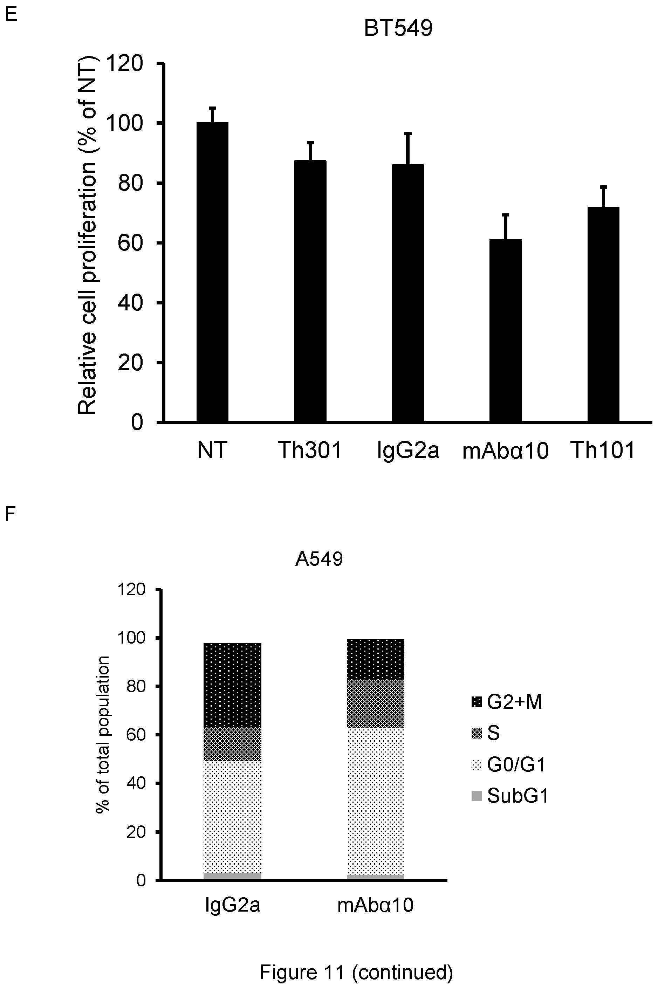

Figure 11: I**ntegrin alpha10 antibody reduces cell proliferation of breast, prostate, pancreatic and lung cancer cells as spheres. (A)** Breast (BT549), **(B)** prostate (PC-3) and **(C and D)** pancreatic (MiaPaCa-2 and PANC-1) cancer cells were treated with 5 µg/ml of integrin alpha10 antibody mAbα10, or control antibody (IgG2a), at the same time of seeding the cells in sphere culture conditions. The antibody was added again every second day for 14 days. BrdU was then added to the spheres, incubated for 24 hours and analyzed by flow cytometry to determine proliferation. The data is shown as mean fluorescence intensity.
   **(E)** Similarly, breast cancer cells were incubated with monoclonal anti-integrin alpha10 antibodies, mAbα10 or Th101, or negative control antibodies (Th301, IgG2a).
   Proliferation, as judged by BrdU incorporation, is shown relative to the cells incubated without an antibody (NT=non-treated), which was set to 100%.
   **(F)** The cell proliferation is inhibited by the treatment of integrin alpha10 antibody (mAbα10) by arresting the cell cycle in G0/G1 phase compared to the treatment of control antibody (IgG2a) in lung cancer. Lung cancer cells (A549) were seeded as spheres and treated with 5 µg/ml of integrin alpha 10 antibody (mAb 10) or control antibody (IgG2a). The antibody was added again every second day for 14 days. The cells were stained with BrdU and 7-AAD and the cell cycle was analyzed by flow cytometry. The data is shown as the percent of total population.

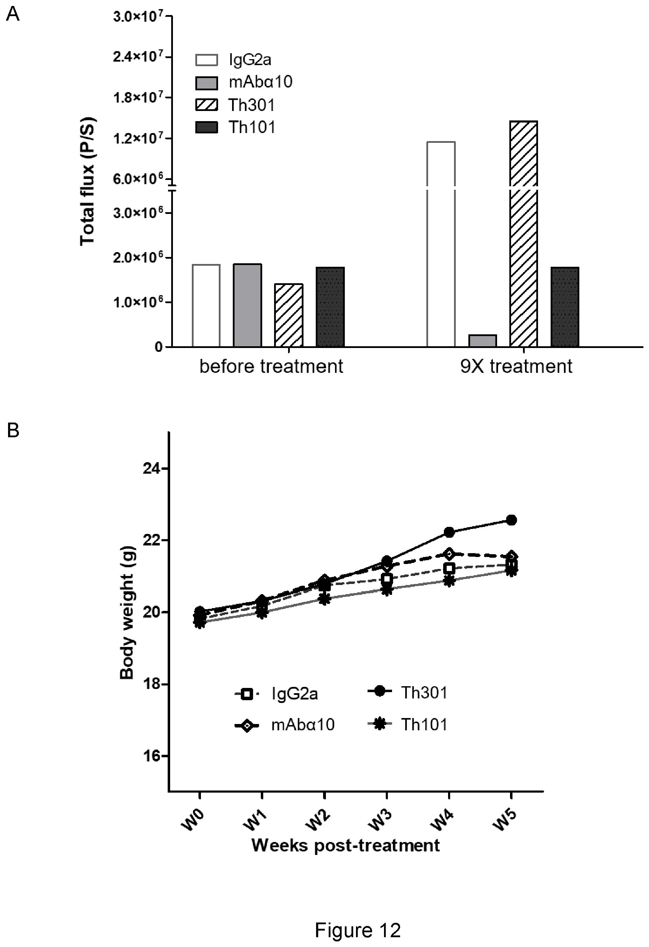

Figure 12: **Treatment with integrin alpha10 antibodies suppresses growth of breast cancer tumors.** The total flux readout (corresponding to the tumor size) was decreased in mice treated with the integrin alpha10 antibodies mAbα10 or Th101 compared to the mice treated with negative control antibodies (IgG2a and Th301).
   **(A)** Box plot showing the tumor growth progression based on total flux (= size of tumor) readouts in different experimental groups (isotype control antibody IgG2a vs. mAbα10 and negative control antibody Th301 vs. Th101) after 9 times of treatment (9X) with the antibodies. Aggressive breast cancer BT549 cells were infected with lentivirus-expressing luciferase and GFP (BT549 Luc/GFP). Animals were injected with 2 x 10⁶ BT549 Luc/GFP cells into the right flank of the mouse. Tumor growth was measured weekly via bioluminescent imaging using IVIS-CT spectrum. Photon flux (photons/s) for each mouse and a combined average is shown over the 9 weeks (9X) period following tumor cell injection. Data reported as means ± SEM of total photon flux (photons per second).
   **(B)** The weight of the mice was recorded each week during the study period. There was no sign of sickness or reduced growth rate observed before the onset of the treatment.

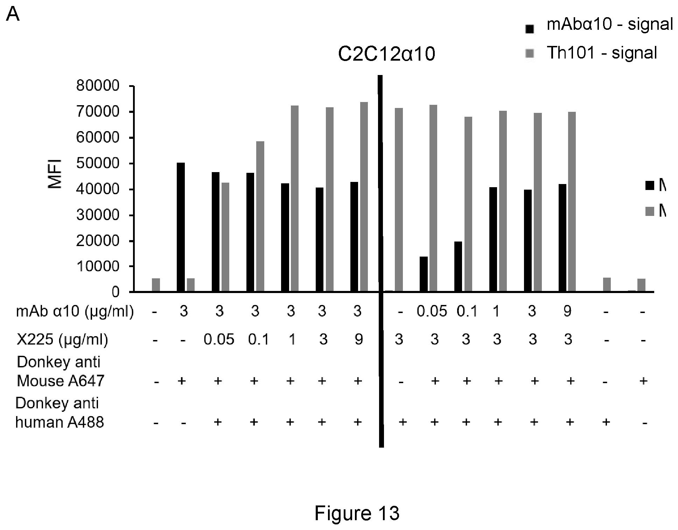

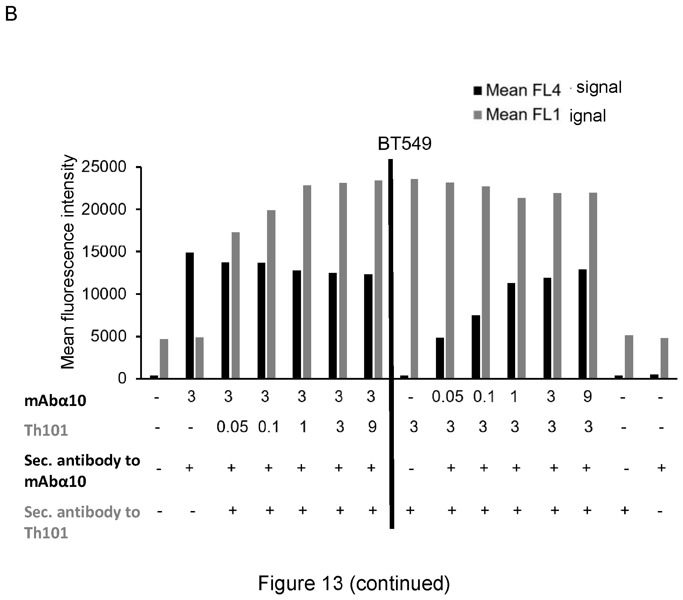

Figure 13: **Monoclonal antibody mAbα10 and Th101 bind different epitopes on integrin alpha10**
   The binding competition assay of the antibody mAbα10 and Th101 was performed in an integrin alpha10 overexpressing C2C12 cell line - C2C12α10 **(A)** and triple-negative breast cancer cell line BT549 **(B)** and analyzed via flow cytometry. Cells were incubated with antibodies at the indicated concentrations (µg/ml) for 30 min and then incubated with secondary antibodies, for another 30 min. Data are expressed in mean fluorescence intensity (MFI) of 100 000 cells. Grey bars: fluorescent intensity of donkey anti-human Alexa 488 which detects the binding intensity of antibody TH101 (human anti-integrin alpha10); Black bars: fluorescent intensity of donkey anti-mouse Alexa 647 which detects the binding intensity of antibody mAbα10 (mouse anti-integrin alpha10). Left part of the figure: With increasing concentrations (0 to 9 µg/ml) of antibody Th101 added to the reaction wells (grey bars), the signal representing antibody mAbα10 (at constant concentration of 3 µg/ml) remained stable (black bars).
   Right part of the figure: With increasing concentrations (0 to 9 µg/ml) of antibody mAbα10 added to the reaction wells (black bars), the signal representing antibody Th101 (at constant concentration of 3 µg/ml) remained stable (grey bars).

### Definitions

"Integrin alpha10" or "Integrin alpha10 subunit" or "Integrin alpha10 polypeptide" as used herein refers to the alpha10 subunit of the heterodimeric protein integrin alpha10 beta1. This denotation does not exclude the presence of the beta1 subunit bound to the alpha10 subunit thus forming the integrin alpha10 beta1 heterodimer. "Alpha" and "α", as well as "alpha10" and "alpha 10" are equivalent terms.

"Integrin alpha10" as used herein may refer to the alpha10 subunit of the heterodimeric protein integrin alpha10 beta1 as well as to the polynucleotide transcript encoding the alpha10 subunit of the heterodimeric protein integrin alpha10 beta1, and fragments thereof.

"Anti-integrin alpha10 antibody" or "Integrin alpha10 antibody" or "Anti-integrin alpha10 subunit antibody" as used herein refers to an antibody capable of recognizing and binding to at least the alpha10 integrin of the heterodimeric protein integrin alpha10 beta1. These antibodies may be antibodies that recognize an epitope of the heterodimeric protein integrin alpha10 beta1, wherein the epitope comprises amino acid residues of both the alpha10 and the beta1 integrin polypeptides. With respect to a monoclonal antibody, "mAba10" and "mAbα10" are equivalent terms.

As used herein, the singular forms "a", "an" and "the" include plural referents unless the context clearly states otherwise. Thus, for example, reference to "an antibody" includes a plurality of such antibodies.

"Subject" as used herein denotes a mammal, such as a rodent, a feline, a canine, an equine and a primate. Preferably a subject according to the invention is a human.

A "sample" as used herein encompasses any subject and a variety of sample types obtained from any subject. Examples of samples useful in the disclosed methods include but are not limited to a subject, a liquid tissue sample such as blood, or a solid tissue sample such as biopsy material or tissue cultures or cells derived there from and the progeny thereof. For example, biological samples include cells obtained from a tissue sample collected from a subject. Thus, samples encompass clinical samples, cells in culture, cell supernatants, cell lysates, and tissue samples, e.g. tissue samples from breast tissue, lung tissue, prostate tissue, pancreatic tissue, ovaries tissue, bone tissue, cartilage tissue, fat tissue, muscle tissue and connective tissue.

An "aggressive cancer form" as used herein refers to a tumor that proliferates quickly and/or migrates to distant sites and other tissues rapidly. An aggressive cancer form may be an invasive tumor and/or a tumor that has a tendency to metastasize and/or a high-grade tumor and/or a highly proliferating tumor. Aggressive cancer forms are usually associated with a poor survival prognosis. Examples of aggressive cancer forms are triple negative breast cancer, inflammatory breast cancer, squamous cell lung carcinoma, lung adenocarcinoma, small-cell lung carcinoma, prostate cancer, pancreatic cancer, ovarian cancer and sarcoma. Different cancer types can display different degrees of aggressivity.

A "cancer" as used herein refers to any malignant and/or invasive growth or tumor caused by abnormal cell growth. As used herein "cancer" refers to tumors named for the type of cells that form them. A cancer or tumor consists of tumor cells or cancer cells. Part of a cancer or tumor might be stroma cells, for example connective tissue cells such as fibroblasts. Examples of solid tumors include but are not limited to sarcomas and carcinomas. The term "cancer" includes but is not limited to a primary cancer that originates at a specific site in the body, a metastatic cancer that has spread from the place in which it started to other parts of the body, a recurrence from the original primary cancer after remission, and a second primary cancer that is a new primary cancer in a person with a history of previous cancer of different type from latter one.

"Detection", "detect", "detecting" as used herein includes qualitative and/or quantitative detection (measuring levels) with or without reference to a control, and further refers to the identification of the presence, absence or quantity of a given target, specifically the target of an integrin alpha 10 subunit.

"Inhibition" as used herein means that the presence of the antibody of the invention inhibits, in whole or in part, the binding of ligands to the receptor and/or the disablement of a signal the receptor would elicit upon ligand binding. This includes for example down-stream signalling having effect on cellular behaviour and processes. "Inhibition" and "blocking" are herein used as equivalent terms.

"ADCC activity" or "Antibody-dependent cellular cytotoxicity activity" as used herein refers to an activity to damage a target cell (e.g., tumour cell) by activating an effector cell via the binding of the Fc region of an antibody to an Fc receptor existing on the surface of an effector cell such as a killer cell, a natural killer cell, an activated macrophage or the like. An activity of antibodies of the present invention includes ADCC activity. ADCC activity measurements and antitumor experiments can be carried out in accordance using any assay known in the art.

### Detailed description

The present inventors have surprisingly found that the integrin alpha10 polypeptide (Uniprot: O75578) encoded by the gene *ITGA10* is overexpressed in tissue obtained from biopsies of aggressive tumors, in particular obtained from triple negative breast cancer, inflammatory breast cancer, squamous cell lung carcinoma, lung adenocarcinoma, small-cell lung carcinoma, prostate cancer, pancreatic cancer, ovarian cancer and sarcoma. Moreover, the present inventors have also found that integrin alpha10 polypeptide (Uniprot: O75578) encoded by the gene *ITGA10* is overexpressed in metastasis derived from the above cancers.

Based on this findings the inventors have developed methods and tools for detection and/or diagnosis and/or treatment and/or prevention of a cancer form, said cancer form being any one of breast cancer, lung cancer, prostate cancer, pancreatic cancer and sarcoma, or said cancer form being a metastasis of the above mentioned cancer forms.

In one embodiment, the said cancer form is an aggressive cancer form.

### Integrin alpha10 polypeptide

Integrins are heterodimers consisting of an alpha and a beta polypeptide. The integrin alpha10 beta1 heterodimer may be detected by anti-integrin alpha10-specific antibodies and integrin alpha10 binding peptides and proteins.

In one embodiment of the present disclosure, the integrin alpha10 polypeptide is a part of an integrin alpha10 beta1 heterodimer.

In one embodiment of the present disclosure, the integrin alpha10 polypeptide is expressed on the surface of the cells.

The integrin alpha10 beta1 was identified as a collagen type II binding receptor on chondrocytes in 1998 (Camper et al., 1998). Immunohistochemical analysis during development and in adult tissues has demonstrated a restricted localization of the marker to cartilage-containing tissues (Camper et al. 1998, Camper et al., 2001). Knockout mice lacking the marker have disorganized growth plates, decreased collagen in the matrix and shorter long-bones, further supporting its cell structural importance (Bengtsson et al., 2005). The amino acid sequence, variants, isoforms and sequence annotations can be found in Uniprot accession no O75578 (ITA10_HUMAN).

Integrin alpha10 beta1 is the most abundant collagen-binding integrin in cartilaginous tissues and its expression pattern is distinct from that of other collagen-binding integrins. *In vitro* and *in vivo* studies have identified integrin alpha10 beta1 as a unique phenotypic marker for chondrocyte differentiation and a crucial mediator of cell-matrix interactions required for proper cartilage development (Lundgren Åkerlund and Aszòdi, 2014).

Moreover, integrin alpha10 beta1 is present on mesenchymal stem cells (MSCs) and treatment of cultured MSCs with fibroblast growth factor-2 (FGF-2) increases expression of integrin alpha10 beta1 and improves in vitro chondrogenesis in aggregate cultures. Thus, integrin alpha10 beta1 is a cell surface biomarker of MSCs with chondrogenic potential (Varas et al., 2007).

Several different mouse brain structures, including whole brain, have earlier been analyzed for integrin alpha10 expression and it has been shown that there is no or low expression of integrin alpha10 in any of the healthy brain tissue (WO 99/51639). However, it has recently been found that the protein integrin alpha10 beta1 is expressed in tissue obtained from malignant neoplasms of the CNS, as well as in the sub ventricular zone (SVZ), a stem cell niche in the brain (WO 2016/133449).

The restricted distribution of the integrin alpha10 in healthy tissues makes it an excellent biomarker for disease. Accordingly, the present disclosure concerns detection of the antigen integrin alpha10 polypeptide, e.g. with antibodies directed specifically to SEQ ID NO: 1 (integrin alpha10), SEQ ID NO: 2 (the extracellular domain of integrin alpha10) or SEQ ID NO: 3 (the extracellular I-domain of integrin alpha10).

In one embodiment of the present disclosure, integrin alpha10 is a naturally occurring variant of integrin alpha10 polypeptide, an isoform of integrin alpha10 polypeptide or a splice variant of an integrin alpha10 polypeptide.

In one embodiment the variant of the integrin alpha10 antigen is at least 70% identical to SEQ ID NOs: 1, 2 or 3, e.g. a variant which is at least 75% identical to SEQ ID NOs: 1, 2 or 3, such as a variant which is at least 80% identical to SEQ ID NOs: 1, 2 or 3, e.g. a variant which is at least 85% identical to SEQ ID NOs: 1, 2 or 3, such as a variant which is at least 90% identical to SEQ ID NOs: 1, 2 or 3, e.g. a variant which is at least 95% identical to SEQ ID NOs: 1, 2 or 3, such as a variant which is at least 96% identical to SEQ ID NOs: 1, 2 or 3, e.g. a variant which is at least 97% identical to SEQ ID NOs: 1, 2 or 3, e.g. a variant which is at least 98% identical to SEQ ID NOs: 1, 2 or 3, e.g. a variant which is at least 99% identical to SEQ ID NOs: 1, 2 or 3, such as a variant which is at least 99.5% identical to SEQ ID NOs: 1, 2 or 3.

In one embodiment the fragment of integrin alpha10 comprises at least 100 consecutive amino acids of SEQ ID NO: 1, preferably at least 200 consecutive amino acids of SEQ ID NO: 1, preferably at least 300 consecutive amino acids of SEQ ID NO: 1, preferably at least 400 consecutive amino acids of SEQ ID NO: 1, preferably at least 500 consecutive amino acids of SEQ ID NO: 1, preferably at least 600 consecutive amino acids of SEQ ID NO: 1, preferably at least 700 consecutive amino acids of SEQ ID NO: 1, preferably at least 800 consecutive amino acids of SEQ ID NO: 1, preferably at least 900 consecutive amino acids of SEQ ID NO: 1, preferably at least 1000 consecutive amino acids of SEQ ID NO: 1.

Integrin alpha10 can also be detected on nucleotide level by analyzing a sample for the presence of e.g. mRNA transcripts which upon translation generates an integrin alpha10 antigen as defined herein above.

### Pharmaceutical compositions and administration thereof

In one embodiment of the present disclosure relates to compositions, such as pharmaceutical compositions, comprising:
a. an antibody specifically binding to an integrin alpha10 polypeptide or a fragment thereof, or
b. a polynucleotide specifically binding to a polynucleotide transcript which encodes an integrin alpha10 polypeptide or a fragment or variant thereof,
wherein said compositions are for use in the diagnosis and/or treatment of a cancer form as defined herein.

In one embodiment of the present disclosure, the composition for use in the diagnosis and/or treatment of an aggressive cancer form as defined herein comprises a pharmaceutically effective amount of an antibody specifically binding to an integrin alpha10 polypeptide or a fragment thereof.

In one embodiment of the present disclosure, the composition for use in the diagnosis and/or treatment of an aggressive cancer form as defined herein comprises pharmaceutically effective amount of a polynucleotide specifically binding to a polynucleotide transcript which encodes an integrin alpha10 polypeptide or a fragment or variant thereof.

A pharmaceutically effective amount as referred to herein is typically an amount of anti-integrin alpha10 antibody or of polynucleotide specifically binding to a polynucleotide transcript which encodes an integrin alpha10 polypeptide, which induces the desired response in an individual receiving said pharmaceutical composition.

Antibodies specifically binding to an integrin alpha10 polypeptide or their fragment, as well as polynucleotides specifically binding to a polynucleotide transcript which encodes an integrin alpha10 polypeptide, as well as their fragments and variants, are described in detail herein.

In one embodiment of the present disclosure, the composition for use in the diagnosis and/or treatment of an cancer form as defined herein comprises a pharmaceutically effective amount of an antibody specifically binding to an integrin alpha10 polypeptide or a fragment thereof, wherein the antibody or fragment thereof is conjugated to an additional moiety.

In one embodiment of the present disclosure, the composition for use in the diagnosis and/or treatment of an aggressive cancer form as defined herein comprises a pharmaceutically effective amount of a polynucleotide specifically binding to a polynucleotide transcript which encodes an integrin alpha10 polypeptide or a fragment or variant thereof, wherein the polynucleotide or fragment or variant thereof is conjugated to an additional moiety.

For example, the additional moiety may be a detectable moiety. An antibody specifically binding to an integrin alpha10 polypeptide or a fragment thereof and conjugated to a detectable moiety may be useful in detecting integrin alpha10 expression on a cell and so determining that said cell may be a malignant cell and/or tumor-associated cell. For example, the additional moiety may be a cytotoxic moiety. An antibody specifically binding to an integrin alpha10 polypeptide or a fragment thereof and conjugated to a cytotoxic moiety, such as an antibody drug conjugate (ADC) comprising an antibody specifically binding to an integrin alpha10 polypeptide or a fragment thereof, may be useful in specifically directing a certain cytotoxic moiety and/or drug to a cell expressing integrin alpha10 and being a malignant cell and/or tumor-associated cell.

For example, the additional moiety may comprise a biological response modifier. Biological response modifiers are substances that modify immune responses by either enhance an immune response or suppress it. Biological response modifiers may be endogenous, such as moieties usually produced naturally within the body, or exogenous.

In one embodiment the additional moiety may comprise a biological response modifier, such as a cytokine, a lymphokine, an interferon or combinations thereof.

### Detectable moieties

The compositions for use of the present disclosure may comprise a detectable moiety, e.g. the antibody specifically binding to an integrin alpha10 polypeptide or a fragment thereof and/or the polynucleotide specifically binding to a polynucleotide transcript which encodes an integrin alpha10 polypeptide or a fragment or variant thereof, may be covalently bound to a detectable moiety.

In one embodiment the detectable moiety selected from the group consisting of a fluorophore, an enzyme or a radioactive tracer or radioisotope. In one embodiment the detectable moiety is a radioactive tracer selected from a positron emitter and a gamma emitter. In one embodiment the radioisotope is selected from the group consisting of 99mTc, ¹¹¹In, ⁶⁷Ga, ⁶⁸Ga, ⁷²As, ⁸⁹Zr, ¹²³I and ²⁰¹Tl.

In one embodiment the antibody comprises a pair of detectable and cytotoxic radionuclides, such as ⁸⁶Y/⁹⁰Y or ¹²⁴I/²¹¹At.

In one embodiment the radioisotope is capable of simultaneously acting in a multimodal manner as a detectable moiety and also as a cytotoxic moiety.

In one embodiment the detectable moiety comprises or consists of a paramagnetic isotope, such as one selected from the group consisting of ¹⁵⁷Gd, ⁵⁵Mn, ¹⁶²Dy, ⁵²Cr and ⁵⁶Fe. In one embodiment the detectable moiety is detectable by an imaging technique such as SPECT, PET, MRI, optical or ultrasound imaging.

In one embodiment the cytotoxic moiety and/or detectable moiety is joined to the antibody or antigen-binding fragment thereof indirectly, via a linking moiety.

### Cytotoxic moieties

The compositions for use of the present disclosure may comprise a cytotoxic moiety, e.g. the antibody specifically binding to an integrin alpha10 polypeptide or a fragment thereof and/or the polynucleotide specifically binding to a polynucleotide transcript which encodes an integrin alpha10 polypeptide or a fragment or variant thereof, may be covalently bound to a cytotoxic moiety.

In one embodiment the cytotoxic moiety is selected from a group consisting of a toxin, a chemotherapeutic agent and a radioactive agent, or combinations thereof.

In one embodiment the cytotoxic moiety is a toxin. For example, in one embodiment the cytotoxic moiety is a toxin selected from the group selected from microtubule toxins, DNA toxins and transcription toxins.

In one embodiment the cytotoxic moiety is a microtubule toxin selected from the group consisting of Auristatin-based toxins, Maytansinoid-based toxins, Tubulysins-based toxins and Eribulin.

In one embodiment the cytotoxic moiety is a DNA toxin selected from the group consisting of DNA minor-groove binding agents, DNA minor-groove binding alkylating agents, DNA alkylating agents and DNA-cleaving agents. For example, in one embodiment the cytotoxic moiety is a DNA toxin selected from the group consisting of Pyrrolobenzodiazepine (PBD), Duocarmycin, Duocarmycin analogues, Indolino-benzodiazepine, Calicheamicins, Irinotecan and Exatecan derivatives.

In one embodiment the cytotoxic moiety is a transcription toxin, such as a ribosome inactivating protein, such as an RNA polymerase II inhibiting agent.

In one embodiment the cytotoxic moiety is a transcription toxin selected from the group consisting of Doxorubicin, Doxorubicin derivatives and Amanitin.

In one embodiment the cytotoxic moiety is a transcription toxin selected from the group consisting of shiga and shiga-like toxins; type I ribosome inactivating proteins, type II ribosome inactivating proteins and saporin, or combinations thereof. For example, the type I ribosome inactivating protein may be trichosanthin and/or luffin. For example, the type II ribosome inactivating protein may be ricin, agglutinin and/or abrin.

In one embodiment the cytotoxic moiety is a chemotherapeutic agent. For example, in one embodiment the chemotherapeutic agent may be an alkylating agent, an antimetabolite, an anti-microtubule agent, a topoisomerase inhibitor or a cytotoxic antibiotic. For example, in one embodiment the chemotherapeutic agent may be selected from the group consisting of Anthracyclines, Taxanes and Platinum agents. For example, in one embodiment the chemotherapeutic agent may be selected from the group consisting of Cisplatin, Paclitaxel, albumin-bound Paclitaxel, Docetaxel, Cyclophosphamide, Eribulin, Epirubicin, Doxorubicin, Carboplatin, Gemcitabine, Bleomycin, Fluorouracil, Cyclophosphamide, Vinorelbine, Capecitabine, Ixabepilone and Ixabepilone, or combinations thereof.

### Formulations

The compositions for use of the present disclosure may be pharmaceutical compositions suitable for parenteral administration. Such compositions preferably include aqueous and non-aqueous sterile injection solutions which may contain wetting or emulsifying reagents, anti-oxidants, pH buffering agents, bacteriostatic compounds and solutes which render the formulation isotonic with the body fluid, preferably the blood, of the individual; and aqueous and non-aqueous sterile suspensions which may include suspending agents or thickening agents. The pharmaceutical composition may be presented in unit-dose or multi-dose containers, for example, sealed ampoules and vials and may be stored in a freeze-dried condition requiring only the addition of the sterile liquid carrier immediately prior to use.

In one embodiment, the composition for use of the present disclosure further comprises at least one pharmaceutically acceptable diluent, carrier or excipient.

Preferably, the composition of the present invention comprises one or more suitable pharmaceutical excipients, which could be non-sterile or sterile, for use with cells, tissues or organisms, such as pharmaceutical excipients suitable for administration to an individual. Such excipients may include, but are not limited to, saline, buffered saline, dextrose, water, glycerol, ethanol and combinations of these excipients in various amounts. The formulation should suit the mode of administration.

Preferably, the pharmaceutical compositions of the present invention are prepared in a form which is injectable, either as liquid solutions or suspensions; furthermore solid forms suitable for solution in or suspension in liquid prior to injection are also within the scope of the present invention. The preparation may be also be emulsified or encapsulated in liposomes.

The anti-integrin alpha10 polypeptide antibody, or the polynucleotide specifically binding to a polynucleotide transcript which encodes an integrin alpha10 polypeptide, may be administered alone or in combination with other compounds, either simultaneously or sequentially in any order.

Administration could for example be parenteral via injection or infusion. Parenteral injection could for example be intraventricular, intrathecal, intratumoural, intravenous, intramuscular, intradermal or subcutaneous injection. Preferably, said administration is parenterally by injection or infusion.

### Clinical conditions

In one embodiment of the present disclosure, the cancer form to be treated and/or prevented and/or detected and/or diagnosed and/or classified and/or determined a prognosis for and/or prevented from metastasizing is selected from the group consisting of breast cancer, lung cancer, prostate cancer, pancreatic cancer, ovarian cancer and sarcoma.

In another embodiment of the present disclosure, the cancer form to be treated and/or prevented and/or detected and/or diagnosed and/or classified and/or determined a prognosis for and/or prevented from metastasizing is an aggressive cancer form selected from the group consisting of aggressive breast cancer, aggressive lung cancer, aggressive prostate cancer, aggressive pancreatic cancer, ovarian cancer and aggressive sarcoma.

An "aggressive cancer form" as used herein refers to a tumor that proliferates quickly and/or migrates to distant sites and other tissues rapidly. An aggressive cancer form may be an invasive tumor and/or a tumor that has a tendency to metastasize and/or a high-grade tumor and/or a highly proliferating tumor. Aggressive cancer forms are usually associated with a poor survival prognosis. Examples of aggressive cancer forms are triple negative breast cancer, inflammatory breast cancer, squamous cell lung carcinoma, lung adenocarcinoma, small-cell lung carcinoma, prostate cancer, pancreatic cancer, ovarian cancer and sarcoma. Different cancer types can display different degrees of aggressivity.

In one embodiment of the present disclosure, the aggressive breast cancer is selected from the group consisting of triple negative breast cancer and inflammatory breast cancer.

In one embodiment of the present disclosure, the aggressive cancer form is a triple negative breast cancer form, and said triple negative breast cancer is selected from the group consisting of basal-like 1 breast cancer, basal-like 2 breast cancer, claudin-low breast cancer, metaplastic breast cancer (MBC), interferon-rich breast cancer, immunomodulatory breast cancer, mesenchymal breast cancer, mesenchymal stem-like breast cancer, luminal androgen receptor breast cancer and unstable breast cancer.

The person of skills in the art knows what the characteristics of aggressive breast cancer are, see for example Arpino et al. 2015; Dai et al. 2016 and Ma et al. 2018.

In one embodiment of the present disclosure, the aggressive breast cancer form is a triple negative breast cancer form and it has morphological features of invasive ductal carcinoma.

In one embodiment of the present disclosure, the aggressive breast cancer form is a triple negative breast cancer form and it has morphological features of basal-like triple negative breast cancer.

In one embodiment of the present disclosure, the aggressive breast cancer form is a basal-like breast cancers form and it has morphological features of invasive ductal carcinoma.

In one embodiment of the present disclosure, the aggressive cancer form is prostate cancer, and the prostate cancer is small cell (neuroendocrine) carcinoma (SCNC).

In one embodiment of the present disclosure, the aggressive cancer form is lung cancer, and the lung cancer is squamous cell lung carcinoma, lung adenocarcinoma, small-cell lung carcinoma or large cell lung carcinoma.

In one embodiment of the present disclosure, the aggressive cancer form is lung cancer, and the lung cancer is squamous cell lung carcinoma.

In one embodiment of the present disclosure, the aggressive cancer form is pancreatic cancer, and the aggressive pancreatic cancer is wherein the aggressive pancreatic cancer is a neuroendocrine tumor.

In one embodiment of the present disclosure, the aggressive cancer form is pancreatic cancer, and the aggressive pancreatic cancer is a grade I, grade II or grade III pancreatic cancer.

In one embodiment of the present disclosure, the aggressive sarcoma is selected from the group consisting of undifferentiated pleomorphic sarcoma, myxofibrosarcoma, dedifferentiated liposarcoma, atypical lipomatous tumor, myxoinflammatory fibroblastic sarcoma, low grade fibromyxoid sarcoma, sclerosing epithelioid fibrosarcoma, pseudimyogenic hemangioendothelioma and mesenchymal chondrosarcoma.

In one embodiment of the present disclosure, the aggressive cancer form is a metastasis, for example, the aggressive cancer form may be a metastasis of any one of aggressive breast cancer, aggressive lung cancer, aggressive prostate cancer, aggressive pancreatic cancer, ovarian cancer and aggressive sarcoma.

In one embodiment of the present disclosure, the aggressive cancer form is a metastasis, for example, a metastasis of any one of triple negative breast cancer and inflammatory breast cancer.

In one embodiment of the present disclosure, the aggressive cancer form is a metastasis, for example, a metastasis of triple negative breast cancer, such as of a triple negative breast cancer selected from the group consisting of basal-like 1 breast cancer, basal-like 2 breast cancer, claudin-low breast cancer, metaplastic breast cancer (MBC), interferon-rich breast cancer, immunomodulatory breast cancer, mesenchymal breast cancer, mesenchymal stem-like breast cancer, luminal androgen receptor breast cancer and unstable breast cancer.

In one embodiment of the present disclosure, the aggressive cancer form is a metastasis, for example, a metastasis of an aggressive lung cancer. In one embodiment of the present disclosure, the aggressive cancer form is a metastasis, for example, a metastasis of squamous cell lung carcinoma, lung adenocarcinoma, small-cell lung carcinoma or large cell lung carcinoma.

In one embodiment of the present disclosure, the aggressive cancer form is a metastasis, for example, a metastasis of an aggressive pancreatic cancer.

In one embodiment of the present disclosure, the aggressive cancer form is a metastasis, for example, a metastasis of an aggressive prostate cancer.

In one embodiment of the present disclosure, the aggressive cancer form is a metastasis, for example, a metastasis of an aggressive sarcoma.

### Treatment of aggressive cancer forms

In one aspect the invention concerns the use of a composition comprising:
a. an antibody specifically binding to an integrin alpha10 polypeptide or a fragment thereof, or
b. a polynucleotide specifically binding to a polynucleotide transcript which encodes an integrin alpha10 polypeptide or a fragment or variant thereof,
for the manufacture of a medicament for the treatment of an aggressive cancer form, wherein said aggressive cancer form is selected from the group consisting of aggressive breast cancer, aggressive lung cancer, aggressive prostate cancer, aggressive pancreatic cancer and aggressive sarcoma, or wherein said aggressive cancer form is a metastasis.

One aspect of the present disclosure relates to a method of treating an aggressive cancer form, wherein said aggressive cancer form is selected from the group consisting of aggressive breast cancer, aggressive lung cancer, aggressive prostate cancer, aggressive pancreatic cancer and aggressive sarcoma, or wherein said aggressive cancer form is a metastasis, the method comprising administering a pharmaceutically effective amount of a composition comprising:
a. an antibody specifically binding to an integrin alpha10 polypeptide or a fragment thereof, or
b. a polynucleotide specifically binding to a polynucleotide transcript which encodes an integrin alpha10 polypeptide or a fragment or variant thereof,
to a subject in the need thereof.

One aspect of the present disclosure relates to a method of inhibiting integrin alpha10-mediated signaling of at least one cancer cell, the method comprising contacting the at least one cancer cell with a composition comprising an effective amount of:
a. an antibody or antigen-binding fragment specific for integrin alpha10 polypeptide; and/or
b. a polynucleotide transcript which encodes an integrin alpha10 polypeptide or a fragment or variant thereof,
wherein the at least one cancer cell is selected from the group consisting of an aggressive breast cancer cell, an aggressive lung cancer cell, an aggressive prostate cancer cell, an aggressive pancreatic cancer cell, an aggressive sarcoma cell and a metastatic tumor cell.

It is understood by the person skilled in the art that cellular signaling includes molecular mechanisms whereby cells detect and respond to external stimuli. Cell signaling also includes transcriptional and translational controls and mechanisms as well as signal transduction mechanisms.

One aspect of the present disclosure relates to a method of inhibiting cellular functions of at least one cancer cell, the method comprising contacting the at least one cancer cell with an effective amount of a composition comprising:
a. an antigen comprising an integrin alpha10 polypeptide or a fragment thereof; and/or
b. a polynucleotide transcript which encodes an integrin alpha10 polypeptide or a fragment or variant thereof,
wherein the at least one cancer cell is selected from the group consisting of an aggressive breast cancer cell, an aggressive lung cancer cell, an aggressive prostate cancer cell, an aggressive pancreatic cancer cell, an aggressive sarcoma cell and a metastatic tumor cell.

In one embodiment the present invention relates to a method for inhibiting the growth and/or proliferation of a cell expressing integrin alpha10 comprising administering a composition comprising an effective amount of:
a. an antibody specifically binding to an integrin alpha10 polypeptide or a fragment thereof, or
b. a polynucleotide specifically binding to a polynucleotide transcript which encodes an integrin alpha10 polypeptide or a fragment or variant thereof.

In one embodiment of the present disclosure, inhibiting at least one cancer cell is selected from the group consisting of:
a. inhibiting proliferation of the at least one cancer cell;
b. inhibiting self-renewal of the at least one cancer cell;
c. inhibiting anchorage-independent growth of the at least one cancer cell;
d. inhibiting migration of the at least one cancer cell;
e. inhibiting invasion of the at least one cancer cell;
f. inhibiting adhesion of the at least one cancer cell; and/or
combinations thereof.

In another embodiment, inhibiting at least one cancer cell by i) an antibody specifically binding to an integrin alpha10 polypeptide or ii) a polynucleotide specifically binding to a polynucleotide transcript which encodes an integrin alpha10 polypeptide or a fragment or variant thereof, inhibits anchorage-dependent growth of the at least one cancer cell.

In one embodiment, the present disclosure relates to a method of inhibiting at least one cancer cell, wherein the cancer cell is in an aggressive and/or metastatic tumor, and wherein said inhibiting is at least one of:
a. growth of the aggressive and/or metastatic tumor;
b. proliferation of the aggressive and/or metastatic tumor;
c. migration of the aggressive and/or metastatic tumor;
d. invasion of the aggressive and/or metastatic tumor;
e. initiation of new aggressive and/ metastatic tumors;
f. infiltration of new aggressive and/ metastatic tumors; and
combinations thereof.

The cell may express one or more further markers as defined herein. In one embodiment said cell is a malignant cells and/or tumor-associated cell. In another embodiment said cell is a cancer associated fibroblast (CAFs), a stromal cell, a stem cells and/or a stem-like cell. Said method may be performed in vitro or in vivo.

In one embodiment of the present disclosure, the composition comprising an effective amount of:
a. an antibody specifically binding to an integrin alpha10 polypeptide or a fragment thereof, or
b. a polynucleotide specifically binding to a polynucleotide transcript which encodes an integrin alpha10 polypeptide or a fragment or variant thereof.

Is capable of inducing cell death and/or inhibiting the growth and/or inhibiting proliferation and/or inhibiting migration of cells expressing an integrin alpha10.

In one embodiment of the present disclosure, the treatment is initiated upon detection of an integrin alpha10 polypeptide and/or polynucleotide transcript in a cancer cell in a tumor of said subject.

In one embodiment of the present disclosure, the composition disclosed herein for treatment of an aggressive cancer form is administered to an individual in need thereof in combination with radiation therapy and/or surgical removal of cancer. For example, in one embodiment the composition disclosed herein for treatment of an aggressive cancer form is administered to an individual thereof prior to radiation therapy and/or surgical removal of cancer. Alternatively, or in addition, in one embodiment the composition disclosed herein for treatment of an aggressive cancer form is administered to an individual thereof after radiation therapy and/or surgical removal of cancer.

In one embodiment of the present disclosure, the treatment is prophylactic, ameliorative or curative.

In one embodiment of the present disclosure, the methods disclosed herein target an antigen comprising an integrin alpha10 polypeptide or a fragment thereof that is expressed on the surface of the cells.

The person skilled in the art will appreciate the possibility to prevent metastasis derived from an aggressive cancer.

One aspect of the present disclosure relates to a method of preventing metastasis from a primary cancer form selected from the group consisting of breast cancer, lung cancer, prostate cancer, pancreatic cancer and sarcoma, the method comprising administering a therapeutically effective amount of:
a) an antibody or antigen-binding fragment thereof, wherein the antibody or antigen binding fragment is specific for integrin alpha10 polypeptide; and/or
b) a polynucleotide transcript which encodes an integrin alpha10 polypeptide or a fragment or variant thereof,
to a patient in need thereof.

It will be appreciated by the person skilled in the art that a method of preventing metastasis from a primary cancer can be administered upon detection of the primary cancer.

### Detection and diagnosis of aggressive cancer forms

One aspect of the present disclosure relates to an agent comprising or consisting of an antibody with specificity for an integrin alpha10 polypeptide, or a fragment thereof, for use in detecting cells associated with an aggressive cancer form of a mammal, wherein the cells express an integrin alpha10 polypeptide, and wherein said aggressive cancer form is selected from the group consisting of aggressive breast cancer, aggressive lung cancer, aggressive prostate cancer, aggressive pancreatic cancer and aggressive sarcoma, or a metastasis of any one of said aggressive cancer forms.

An aspect of the present disclosure relates to a composition comprising:
a) an antibody or antigen-binding fragment thereof, specifically binding to an integrin alpha10 polypeptide, or
b) a polynucleotide specifically binding to a polynucleotide transcript which encodes an integrin alpha10 polypeptide or a fragment or variant thereof,
for use in the diagnosis of a cancer form selected from the group consisting of breast cancer, lung cancer, prostate cancer, pancreatic cancer, and sarcoma, or a metastasis of any one of said cancer forms.

It will be appreciated by the person skilled in the art that the process of detecting a biological marker for a disease, e.g. integrin alpha10 as in the present invention, or diagnosing a disease, e.g. by analyzing the expression of integrin alpha10 as in the present invention, can include comparing the tissue to be analyzed to a healthy, non-malignant or non-affected tissue. For example, a breast cancer sample can be compared to an unaffected area in the same tissue sample or to a healthy breast tissue sample. A lung cancer sample can be compared to an unaffected area in the same tissue sample or to a healthy lung tissue sample. A prostate cancer sample can be compared to an unaffected area in the same tissue sample or to a healthy prostate tissue sample. A pancreatic cancer sample can be compared to an unaffected area in the same tissue sample or to a healthy pancreatic tissue sample. A sarcoma sample can be compared to an unaffected area in the same tissue sample or to a healthy connective tissue sample.

In the process of diagnosing a cancer, the person skilled in the art will appreciate the possible usefulness to compare the level of integrin alpha10 polypeptide or polynucleotide of a cancer cell to a reference cell.

In one embodiment, the diagnosed cancer comprises cells which display equal or higher levels of i) the integrin alpha10 antigen or ii) the polynucleotide transcript observed in healthy and/or benign tissue of the same type.

In another embodiment, the diagnosed cancer comprises cells which display equal or higher levels of i) the integrin alpha10 antigen or ii) the polynucleotide transcript observed in a less aggressive cancer type of the same tissue type, compared to the diagnosed cancer type.

In another embodiment, the diagnosed cancer comprises cells which display equal or higher expression levels of i) the integrin alpha10 antigen or ii) the polynucleotide transcript than in a reference cell line.

The person skilled in the art will appreciate that reference cell lines might be of use in a standardized diagnosis procedure due to the genotypic and phenotypic stability of these cell lines compared to primary cells. Reference cell lines might be established from healthy tissue or from cancer tissue, wherein the cancer might be a more or less aggressive cancer type, depending on its tendency to grow and metastasize. Established cell lines need to be immortalized to be able to be propagated in cell culture. A non-malignant cell line as used herein is understood as an established cell line which does not show signs of malignancy, and which is similar in its phenotype to healthy tissue cells.

Reference cell lines might be, for example, the cell lines depicted in table 1.

**Table1. Overview over cell lines used in the present study, and their aggressivity**

| | **Non-malignant** | **Less aggressive** | **Aggressive** |
|---|---|---|---|
| **Breast cancer** | 184A1 | HCC1428 | BT549 |
| | | T47D | MDA-MB-231 |
| **Prostate cancer** | | 22Rv1 | PC-3 |
| | | DU145 | |
| **Pancreatic cancer** | | BxPC-3 | PANC-1 |
| | | AsPC-1 | MiaPaCa-2 |
| **Lung cancer** | | | A549 |
| | | | U-1752 |
| **Sarcoma cancer** | | | MFH152 |

In one embodiment the reference cell line used during the diagnosis procedure is derived from healthy tissue, such as selected from the group consisting of a reference cell line derived from healthy breast tissue, a reference cell line derived from healthy prostate tissue, a reference cell line derived from healthy lung tissue, a reference cell line derived from healthy pancreas tissue and a reference cell line derived from healthy connective tissue.

In one embodiment the reference cell line used during the diagnosis procedure of breast cancer is derived from healthy tissue, such as the reference cell line 184A1 derived from healthy breast cancer tissue.

In another embodiment the reference cell line used during the diagnosis procedure is derived from a less aggressive cancer type of the same tissue type, compared to the diagnosed cancer type, such as selected from the group consisting of a reference cell line derived from less aggressive breast cancer, a reference cell line derived from less aggressive prostate cancer, a reference cell line derived from less aggressive lung cancer, a reference cell line derived from less aggressive pancreatic cancer, and a reference cell line derived from less aggressive sarcoma.

In one embodiment the reference cell line used during the diagnosis procedure of an aggressive cancer is selected from the group consisting of cell line HCC1428 derived from a less aggressive breast cancer, cell line T47D derived from a less aggressive breast cancer, cell line 22Rv1 derived from a less aggressive prostate cancer, cell line DU145 derived from a less aggressive prostate cancer, cell line BxPC-3 derived from a less aggressive pancreatic cancer and cell line AsPC-1derived from a less aggressive pancreatic cancer.

An aspect of the present disclosure relates to a method for detecting an aggressive cancer cell in a subject, said method comprising the steps of:
a. providing a tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen and/or ii) the polynucleotide transcript, and
d. comparing said expression level determined in c. with a control level,

wherein said control level is the average level observed in healthy and/or benign cells of the same tissue type as the isolated sample,
wherein an expression level of i) the antigen and/or ii) the polynucleotide transcript higher than the control level is indicative of the presence of an aggressive cancer form in the subject, and wherein said aggressive cancer form is selected from the group consisting of aggressive breast cancer, aggressive lung cancer, aggressive prostate cancer, aggressive pancreatic cancer and aggressive sarcoma, or a metastasis of any one of said aggressive cancer forms.

Another aspect of the present disclosure relates to a method for diagnosis of a cancer form in a subject, the method comprising the steps of:
a. providing a tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of the i) integrin alpha10 antigen and/or ii) polynucleotide transcript, and
d. comparing the expression level determined in c. with a control level, wherein said control level is the average expression level of i) the antigen or ii) the polynucleotide transcript observed in healthy and/or benign tissue of the same type; and

wherein an expression level of i) the antigen and/or ii) the polynucleotide transcript higher than the control level is indicative of the presence of an cancer form in a sample, wherein said cancer form is selected from the group consisting of breast cancer, lung cancer, prostate cancer, pancreatic cancer and sarcoma, or a metastasis of any one of said cancer forms,
thereby diagnosing a cancer form in a subject.

Yet another aspect of the present disclosure relates to a method for diagnosis of an cancer form in a subject, the method comprising the steps of:
a. providing a tissue suspected of comprising cancer cells of the subject;
b. analyzing in the tissue the presence of one or more cells having a cancer morphology;
c. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,

optionally determining the expression level of the i) integrin alpha10 antigen and/or ii) polynucleotide transcript, and
wherein presence of one or more cells having a cancer morphology in combination with expression of integrin alpha10 i) antigen and/or ii) polynucleotide transcript are indicative of the presence of an cancer form in a sample, wherein said cancer form is selected from the group consisting of breast cancer, lung cancer, prostate cancer, pancreatic cancer and sarcoma, or a metastasis of any one of said cancer forms, thereby diagnosing an cancer form in a subject.

In one embodiment of the present disclosure, analyzing presence of an antigen comprising an integrin alpha10 polypeptide or a fragment thereof in step b. comprises contacting the tissue suspected of comprising aggressive cancer cells with a composition of the present disclosure. For example, in one embodiment of the present disclosure, the tissue suspected of comprising cancer cells may be put in contact with a composition comprising or consisting of an antibody with specificity for an integrin alpha10 polypeptide.

In addition to analyzing in the sample the presence of an antigen comprising an integrin alpha10 polypeptide or a fragment thereof; and/or a polynucleotide transcript which encodes an integrin alpha10 polypeptide or a fragment or variant thereof, and determining whether the integrin alpha10 expression level is higher than a control level, the method for diagnosis of an aggressive cancer form in a subject and/or the method for detecting an aggressive cancer cell in a subject may further comprise a step of morphologically characterizing the sample as comprising cancer cells belonging to an aggressive cancer forms, wherein said aggressive cancer form is selected from the group consisting of aggressive breast cancer, aggressive lung cancer, aggressive prostate cancer, aggressive pancreatic cancer and aggressive sarcoma, or a metastasis of any one of said aggressive cancer forms.

Morphological characterization of cancer tissues or of tissues suspected of being cancerous, is known to the person of skills in the art and is currently used to detect cancer cells and diagnose presence of cancer. However, morphological characterization alone may only be useful in distinguishing a cancer cell from a healthy and/or benign cell of the same tissue type, and it is not sufficient to determine whether a cancer cell belongs to an aggressive cancer form, such as for diagnosing the presence of an aggressive cancer form in a subject.

Hence, in one aspect, the present disclosure relates to a method for detecting an aggressive cancer cell in a subject, said method comprising the steps of:
a. providing a tissue suspected of comprising cancer cells of the subject;
b. analyzing in the tissue the presence of one or more cells having a cancer morphology,
c. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
d. optionally determining the expression level of the i) integrin alpha10 antigen and/or ii) polynucleotide transcript,
wherein presence of one or more cells having a cancer morphology in combination with expression of integrin alpha10 i) antigen and/or ii) polynucleotide transcript are indicative of the presence of an aggressive cancer form in the subject, and wherein said aggressive cancer form is selected from the group consisting of aggressive breast cancer, aggressive lung cancer, aggressive prostate cancer, aggressive pancreatic cancer and aggressive sarcoma, or a metastasis of any one of said aggressive cancer forms.

In one embodiment, the present disclosure relates to a method for detecting the presence of a breast cancer cell in a subject, or for diagnosis of breast cancer in a subject, wherein said breast cancer is an aggressive breast cancer form selected from the group consisting of triple negative breast cancer and inflammatory breast cancer, the method comprising the steps of:
a. providing breast tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign breast tissue; and
wherein an expression level of i) the antigen and/or ii) the polynucleotide transcript higher than the control level is indicative of the presence of breast cancer, thereby detecting presence of a breast cancer cell and/or diagnosing breast cancer in a subject.

In one embodiment, the present disclosure relates to a method for detecting the presence of lung cancer in a subject, or for diagnosis of lung cancer in a subject, the method comprising the steps of:
a. providing a lung tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign lung tissue; and
wherein an expression level of i) the antigen and/or ii) the polynucleotide transcript higher than the control level is indicative of the presence of lung cancer, thereby detecting and/or diagnosing squamous lung cancer in a subject.

In one embodiment, the present disclosure relates to a method for detecting the presence of squamous lung cell carcinoma in a subject, or for diagnosis of squamous lung cell carcinoma in a subject, the method comprising the steps of:
a. providing a lung tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign lung tissue; and
wherein an expression level of i) the antigen and/or ii) the polynucleotide transcript higher than the control level is indicative of the presence of squamous lung cell carcinoma, thereby detecting and/or diagnosing squamous lung cell carcinoma in a subject.

In one embodiment, the present disclosure relates to a method for detecting the presence of lung adenocarcinoma in a subject, or for diagnosis of lung adenocarcinoma in a subject, the method comprising the steps of:
a. providing a lung tissue suspected of comprising cancer cells of the subject;
b. analyzing in the tissue the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign lung tissue; and
wherein an expression level of i) the antigen and/or ii) the polynucleotide transcript higher than the control level is indicative of the presence of lung adenocarcinoma, thereby detecting and/or diagnosing lung adenocarcinoma in a subject.

In one embodiment, the present disclosure relates to a method for detecting the presence of a prostate cancer cell in a subject, or for diagnosis of prostate cancer in a subject, the method comprising the steps of:
a. providing a prostate tissue suspected of comprising cancer cells of the subject;
b. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign prostate tissue; and
wherein an expression level of i) the antigen and/or ii) the polynucleotide transcript higher than the control level is indicative of the presence of prostate cancer, thereby detecting and/or diagnosing prostate cancer in a subject.

In one embodiment, the present disclosure relates to a method for detecting the presence of a pancreatic cancer cell in a subject, or for diagnosis of pancreatic cancer in a subject, the method comprising the steps of:
a. providing a pancreatic tissue suspected of comprising cancer of the subject;
b. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign pancreatic tissue; and
wherein an expression level of i) the antigen and/or ii) the polynucleotide transcript higher than the control level is indicative of the presence of pancreatic cancer, thereby detecting and/or diagnosing pancreatic cancer in a subject.

In one embodiment, the present disclosure relates to a method for detecting the presence of a sarcoma cell in a subject, or for diagnosis of sarcoma in a subject, the method comprising the steps of:
a. providing a connective tissue suspected of comprising cancer of the subject;
b. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of i) the antigen or ii) the polynucleotide transcript, and
d. comparing the expression level determined in c. with a control level, wherein said control level is the average expression level of i) the antigen or ii) the polynucleotide transcript observed in healthy and/or benign connective tissue; and
wherein an expression level of i) the antigen and/or ii) the polynucleotide transcript higher than the control level is indicative of the presence of sarcoma, thereby detecting and/or diagnosing sarcoma in a subject.

The present inventors have found that the expression level of integrin alpha10 is useful in distinguishing between subtypes of aggressive breast cancer, such as between subtypes of triple negative breast cancer.

Thus, one aspect of the present disclosure relates to a method for classification of a triple negative breast cancer tumor sample of a subject, said method comprising:
a. providing a sample of a breast tissue from the subject;
b. isolating breast cancer cells characterized as being ER negative, PR negative and HER2 negative;
c. determining in the isolated cells an expression level of:
   i. an antigen comprising an integrin alpha10 polypeptide or a fragment thereof; and/or
   ii. a polynucleotide transcript which encodes an integrin alpha10 polypeptide or a fragment or variant thereof, and
d. comparing the expression level determined in c. to a control level, wherein the control level is the average expression level of the antigen comprising an integrin alpha10 polypeptide observed in healthy and/or benign breast tissue;

wherein an expression level of i) the antigen and/or ii) the polynucleotide transcript in the breast cancer cells higher than a control level, and an expression status of ER negative, PR negative and HER2 negative are indicative of a basal-like triple negative breast cancer or a luminal triple negative breast cancer,
thereby classifying the triple negative breast cancer tumor sample as belonging to a basal-like triple negative breast cancer tumor or to a luminal triple negative breast cancer tumor.

In alternative one embodiment, the luminal triple negative breast cancer is a luminal androgen receptor triple negative breast cancer.

In one embodiment, the method for classification of a triple negative breast cancer tumor sample of the present disclosure further comprises analyzing the expression level of claudin in said sample, wherein an expression level of i) the antigen and/or ii) the polynucleotide transcript in the breast cancer cells higher than a control level, and an expression status of ER negative, PR negative, HER2 negative and low levels of claudin are indicative of a basal-like triple negative breast cancer,
thereby classifying the triple negative breast cancer tumor sample as belonging to a basal-like triple negative breast cancer tumor.

Claudin-low breast tumors are a subtype of triple negative breast tumors. These tumors exhibit low expression of many of the claudin genes, including claudin 3, claudin 4, and claudin 7. Other important features of claudin-low tumors is that they almost always have an intense immune cell infiltrate, and they also have stem cell features and features of epithelial mesenchymal transition (EMT).

In one embodiment, the method for classification of a triple negative breast cancer tumor sample of the present disclosure further comprising classifying the triple negative breast cancer tumor sample as belonging to a type 2 basal-like triple negative breast cancer tumor when the expression level of i) the integrin alpha10 antigen and/or ii) the polynucleotide transcript is higher than a control level.

In one embodiment, the method for classification of a triple negative breast cancer tumor sample of the present disclosure, further comprises determining expression of one or more polypeptides selected from the groups consisting of cytokeratin 7, cytokeratin 8, cytokeratin 18 and cytokeratin 19, and wherein expression of integrin alpha10 and one or more polypeptides selected from the groups consisting of cytokeratin 7, cytokeratin 8, cytokeratin 18 and cytokeratin 19 is indicative of luminal triple negative breast cancer.

In one embodiment, the method for classification of a triple negative breast cancer tumor sample of the present disclosure, further comprises determining expression of one or more polypeptides selected from the groups consisting of cytokeratin 5/6, cytokeratin 14, cytokeratin 17, p63, EGFR and c-kit/CD117, and expression of integrin alpha10 and one or more polypeptides selected from the groups consisting of cytokeratin 5/6, cytokeratin 14, cytokeratin 17, p63, EGFR, 34BE12 and c-kit/CD117 is indicative of basal-like triple negative breast cancer.

The present inventors have surprisingly found that a high level of integrin alpha10 expression in cells belonging to an aggressive cancer form as defined herein directly correlate and is indicative of a poor prognosis.

Hence, one aspect of the present disclosure relates to a method for determining a prognosis for an aggressive cancer form for a subject, the aggressive cancer form selected from the group consisting of aggressive breast cancer, aggressive lung cancer, aggressive prostate cancer, aggressive pancreatic cancer and aggressive sarcoma, or a metastasis of any one of said aggressive cancer forms, the method comprising:
a. providing a cancer tumor tissue of the subject;
b. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
c. determining the expression level of the i) integrin alpha10 antigen and/or ii) polynucleotide transcript,
d. comparing the expression level determined in c. to a control level, wherein the control level is the average expression level of the integrin alpha10 i) antigen and/or ii) polynucleotide transcript observed in healthy and/or benign tissue of the same tissue type as the sample;
e. determining an unfavorable prognosis of the aggressive cancer form when the expression level of i) the antigen and/or ii) the polynucleotide transcript is higher than the control level.

Another aspect of the present disclosure relates to a method for determining a prognosis for an aggressive cancer form for a subject, the method comprising:
a. providing a cancer tumor tissue of the subject;
b. analyzing in the tissue the presence of one or more cells having a cancer morphology;
c. analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
d. optionally determining the expression level of the i) integrin alpha10 antigen and/or ii) polynucleotide transcript,
e. determining an unfavorable prognosis of the aggressive cancer form when one or more cells having a cancer morphology are present in the tissue in combination with expression of the integrin alpha10 i) antigen and/or ii) polynucleotide transcript,
wherein the aggressive cancer form selected from the group consisting of aggressive breast cancer, aggressive lung cancer, aggressive prostate cancer, aggressive pancreatic cancer and aggressive sarcoma, or a metastasis of any one of said aggressive cancer forms.

The method for determining a prognosis for an aggressive cancer form for a subject may be applied to any one of the aggressive cancer forms described herein, see for example the section "Clinical conditions".

In one embodiment, the present disclosure relates to a method for determining a prognosis for an aggressive cancer form for a subject, wherein the prognosis is overall survival rate or recurrence free survival rate.

In one embodiment of the present disclosure, the methods disclosed herein target an antigen comprising an integrin alpha10 polypeptide or a fragment thereof that is expressed on the surface of the cells.

The methods of the present disclosure may be conducted *in vivo* or *in vitro*.

In one embodiment, the method for detecting an aggressive cancer cell in a subject as disclosed herein is conducted *in vivo*. In one embodiment, the method for diagnosis of an aggressive cancer form in a subject as disclosed herein is conducted *in vitro,* and the tissue is a tissue sample obtained from the subject.

In one embodiment, the method for diagnosis of an aggressive cancer form in a subject as disclosed herein is conducted *in vivo*. In one embodiment, the method for diagnosis of an aggressive cancer form in a subject as disclosed herein is conducted *in vitro,* and the tissue is a tissue sample obtained from the subject.

In one embodiment, the method for determining a prognosis for an aggressive cancer in a subject as disclosed herein is conducted *in vivo*. In one embodiment, the method for determining a prognosis for an aggressive cancer in a subject as disclosed herein is conducted *in vitro,* and the tissue is a tissue sample obtained from the subject.

In one embodiment of the present disclosure, the step of analyzing in the sample the presence of:
i. an antigen comprising an integrin alpha10 polypeptide or a fragment thereof;
   and/or
ii. a polynucleotide transcript which encodes an integrin alpha10 polypeptide or a fragment or variant thereof,
comprises imaging the tissue and/or tissue sample.

In one embodiment of the present disclosure, the step of determining the expression level of the i) integrin alpha10 antigen and/or ii) polynucleotide transcript comprises imaging the tissue and/or tissue sample.

Imaging may for example be conducted by administering to the subject and/or to the tissue sample a labelled moiety that is capable of binding to an antigen comprising an integrin alpha10 polypeptide or a fragment thereof. For example, imaging may be conducted by administering to the subject and/or to the tissue sample a labelled anti-integrin alpha10 antibody as defined herein.

### Antibodies directed against integrin alpha10 polypeptide

In one embodiment the composition for use in the diagnosis and/or treatment and or prevention of an aggressive cancer form of the present disclosure comprises an anti-integrin alpha10-specific antibody which binds to the integrin alpha10 polypeptide in an immunological reaction. Preferably, the antibody binds to the integrin alpha10 polypeptide extracellular domain, but in certain embodiments the anti-integrin alpha10 antibody has overlapping specificity for the entire integrin alpha10 beta1 heterodimeric complex. This may e.g. mean that the antibody of the present invention binds to an epitope covering both the alpha10 and beta1 polypeptides.

The antibodies and functional equivalents thereof may be produced by any suitable method known to the person skilled in the art.

In one embodiment the antibody of the invention is produced in a hybridoma cell line (e.g. the mAb 365 hybridoma cell line deposited at the Deutsche Sammlung von Mikroorganismen und Zellkulturen GmbH under the accession number DSM ACC2583), thus producing an antibody binding to the extracellular integrin alpha10 domain. For production of said hybridoma, a gene knockout mouse of the integrin alpha10beta1 may be used. The knockout mouse is described in WO 03/101497, included herein by reference.

In one embodiment the antibody of the invention is
a) a monoclonal antibody, produced by the hybridoma cell line deposited at the Deutsche Sammlung von Mikroorganismen und Zellkulturen GmbH under the accession number DSM ACC2583; or
b) an antibody which competes for binding to the same epitope as the epitope bound by the monoclonal antibody produced by the hybridoma deposited at the Deutsche Sammlung von Mikroorganismen und Zellkulturen GmbH under the accession number DSM ACC2583; or
c) a fragment of a) or b), wherein said fragment is capable of binding specifically to the extracellular I-domain of the integrin alpha10 polypeptide chain.

In another embodiment, the anti-integrin alpha10 antibody of the present invention has been identified by an antibody panning procedure.

The antibody of the present invention defined by the amino acid sequences of SEQ ID NO.s: 4-11, will hereby be denoted Th101. The Th101 antibody is disclosed herein by its 6 CDRs (complementary determining regions) (SEQ ID NO: 4 to 9), by its heavy chain variable region (SEQ ID NO: 10) and by its light chain variable region (SEQ ID NO: 11). The identification and production of the Th101 antibody, as employed in the examples, is described in WO 08/075038, included herein by reference.

In one embodiment, the anti-integrin alpha10 antibody of the present invention comprises:
a heavy chain variable region comprising
   a) a CDR-H1 comprising or consisting of the amino acid sequence of SEQ ID NO: 4;
   b) a CDR-H2 comprising or consisting of the amino acid sequence of SEQ ID NO: 5; and
   c) a CDR-H3 comprising or consisting of the amino acid sequence of SEQ ID NO: 6;
   and/or
a light chain variable region comprising
   d) a CDR-L1 comprising or consisting of the amino acid sequence of SEQ ID NO: 7;
   e) a CDR-L2 comprising or consisting of the amino acid sequence of SEQ ID NO: 8; and
   f) a CDR-L3 comprising or consisting of the amino acid sequence of SEQ ID NO: 9.
or a variant of any one of SEQ ID NO:s 4 to 9, wherein any one amino acid has been altered for another amino acid, with the proviso that no more than 3 amino acids have been so altered, for example wherein 2, or 1 amino acids have been so altered.

In another embodiment, the anti-integrin alpha10 antibody of the present invention comprises a heavy chain variable region comprising or consisting of the amino acid sequence of SEQ ID NO: 10.

In another embodiment, the anti-integrin alpha10 antibody of the present invention comprises a light chain variable region comprising or consisting of the amino acid sequence of SEQ ID NO: 11.

The integrin alpha10 antibody may be administrated to said mammal more than once, such as twice, for example 3 times, such as 3 to 5 times, for example 5 to 10 times, such as 10 to 20 times, for example 20 to 50 times, such as more than 50 times. It is also possible that different integrin alpha10 antigens are administered to the same mammal, either simultaneously of sequentially in any order.

In general, the integrin alpha10 antibody will be in an aqueous solution or suspension prior to administration. Furthermore, the integrin alpha10 antigen may be mixed with one or more other compounds. For example, the integrin alpha10 antigen may be mixed with one or more suitable adjuvants and/or with one or more carriers.

Adjuvants are any substance whose admixture with an administered antigen increases or otherwise modifies the immune response to said antigen. Suitable adjuvants are well known by those of skill in the art.

Carriers are scaffold structures, e.g. a polypeptide or a polysaccharide, to which an antigen is capable of being associated. A carrier may be present independently of an adjuvant. Suitable carriers are well known by those of skill in the art.

Methods of preparing monoclonal antibodies, mixtures of monoclonal antibodies or polyclonal antibodies are known in the art and are for example described in Antibodies: A Laboratory Manual, By Ed Harlow and David Lane, Cold Spring Harbor Laboratory Press, 1988*.*

In one embodiment the anti-integrin alpha10 antibody of the present invention is an antibody capable of inhibiting the biological and functional activity of an integrin alpha10 polypeptide.

In another embodiment the anti-integrin alpha10 antibody administered as a part of an antibody-drug complex (ADC). In an ADC, the antibody is linked to a moiety e.g. a disease modifying drug or toxin. Upon specifically binding to integrin alpha10 polypeptide, the ADC is internalized into the cell, and thereby the moiety is delivered into the cell.

In one embodiment the antibody comprised in the composition for use in the diagnosis and/or treatment of an aggressive cancer form of the present disclosure has an isotype selected from the group consisting of IgA, IgD, IgG, IgE and IgM. In a further embodiment the antibody is an IgG isotype, such as an IgG isotype selected from the group consisting of IgG1, IgG2 (e.g. IgG2a), IgG3 and IgG4.

In one embodiment the antibody specifically binding to an integrin alpha10 polypeptide according to the present disclosure is a single chain antibody.

The present disclosure contemplates both monoclonal and polyclonal antibodies and fragments thereof, antigen binding fragments and recombinant proteins thereof which are capable of binding integrin alpha10 polypeptide.

In one embodiment the antibody specifically binding to an integrin alpha10 polypeptide for use in diagnosis and/or treatment according to present disclosure is a polyclonal antibody.

In one embodiment the antibody specifically binding to an integrin alpha10 polypeptide according to the present disclosure is a humanized antibody or a human antibody.

In one embodiment the antibody specifically binding to an integrin alpha10 polypeptide according to the present disclosure is mouse antibody.

In one embodiment the antibody specifically binding to an integrin alpha10 polypeptide according to the present disclosure is a monoclonal antibody.

In one embodiment the antibody specifically binding to an integrin alpha10 polypeptide according to the present disclosure is a polyclonal antibody.

In one embodiment the antibody comprised in the composition for use in the diagnosis and/or treatment of an aggressive cancer form of the present disclosure is an antibody fragment. Antigen binding fragments of antibodies are fragments of antibodies retaining the ability to specifically bind to an antigen. Examples of antibody fragment of the present invention includes antibody fragments selected from the group consisting of a Fab-fragment, a Fab' fragment, a F(ab')₂ fragment and an Fv fragment, such as a single-chain variable fragment (scFv) and a single-domain antibody.

The antibody comprised in the composition for use in the diagnosis and/or treatment of an aggressive cancer form of the present disclosure may also be a chimeric antibody, i.e. an antibody comprising regions derived from different species. The chimeric antibody may for example comprise variable regions from one species of animal and constant regions from another species of animal. For example, a chimeric antibody can be an antibody having variable regions which derive from a mouse monoclonal antibody and constant regions which are human. Such antibodies may also be referred to as humanized antibodies. For example a chimeric humanized antibody may be fully human.

In one embodiment the antibody comprised in the composition for use in the diagnosis and/or treatment of an aggressive cancer form of the present disclosure is a heterospecific antibody such as a bispecific antibody, which is a protein or polypeptide, which comprises two different antigen binding sites with different specificities. For example, the bispecific antibody may recognize and bind to (a) an epitope on integrin alpha10 and (b) to another epitope on integrin alpha10. It may thus recognize and bind to two different epitopes within the same antigen. The term "heterospecific antibody" is intended to include any protein or polypeptide, which has more than two different antigen binding site with different specificities. Accordingly, the invention includes, but is not limited to, bispecific, trispecific, tetraspecific, and other multispecific antibodies which are directed to integrin alpha10 polypeptide.

In some embodiments, the antibody specifically binding to an integrin alpha10 polypeptide or a fragment thereof according to the present disclosure may be conjugated to a moiety, such as an additional moiety. The conjugation may improve and facilitate both treatment and diagnosis of an aggressive cancer form.

In one embodiment the antibody is covalently bound to a detectable moiety, such as a detectable moiety selected from the group consisting of a fluorophore, an enzyme, a radioactive tracer or a radioisotope. The integrin alpha10 antigen may also be detected by detecting a peptide, protein or polypeptide other than integrin alpha10 polypeptide, wherein said other peptide, protein or polypeptide is capable of specifically binding to an integrin alpha10 antigen. In one embodiment said peptide, protein or polypeptide is linked to an enzyme, a fluorophore or a radioactive tracer. The radioactive tracer may e.g. be selected from a positron emitter, or a gamma emitter. Conjugation of the antibody to a detectable moiety facilitates and improves detection of said antibody, which in turn may facilitate detection of integrin alpha10-expressing cells in a sample and so the diagnosis of an aggressive cancer form.

The person of skill in the art is capable of selecting the standard laboratory equipment for detection of the anti-integrin alpha10 antibodies, depending on the situation and physical state of the sample.

In one embodiment the person of skill in the art would conduct the detection step using flow cytometry such as Fluorescence-Activated Cell Sorting (FACS).

Typical immunological methods well known in the art include but are not limited to western blot, enzyme linked immunosorbent assay (ELISA), radioimmunoassay (RIA), immunohistochemistry (IHC), immunofluorescent assay (IF), fluorescence in situ hybridization (FISH).

Detecting integrin alpha10 can be achieved using methods well known in the art of detection and imaging, such as clinical imaging, such as conventional fluorescence microscopes, confocal microscope, 2-photon microscopes, stimulated emission depletion (STED) etc.

In one embodiment, the anti-integrin alpha10 antibody is a functional, blocking antibody, such as an antibody that is capable of inhibiting and/or blocking migration, and/or proliferation of cells expressing integrin alpha10.

### Molecular probes for detection of integrin alpha10

Analysis of the biological sample for the presence of an integrin alpha10 antigen or an integrin alpha10 encoding polynucleotide can also be carried out by using a molecular probe (protein or polynucleotide) capable of binding or hybridizing to integrin alpha10 mRNA, cDNA or protein to detect its expression in the biological sample or by using PCR, preferably Q-PCR.

In one embodiment the integrin alpha10 specific polynucleotide probes are linked to a detectable moiety optionally capable of emitting photons. By using this embodiment the subject to be diagnosed or investigated may be illuminated using a source of light capable of exciting said detectable moiety e.g. a fluorophore. Methods for detecting photons include but is not limited to PET-scan and SPECT-scan.

In certain embodiments the detectable moiety is selected from the group consisting of a fluorophore, an enzyme or a radioactive tracer.

A polynucleotide transcript may also be detected using PCR, preferably Q-PCR.

In a further embodiment the presence of integrin alpha10 in a biological sample is detected by using an integrin alpha10 nucleic acid probe which binds to integrin alpha10 RNA or cDNA in a hybridizing reaction.

Exemplary nucleic acid integrin-alpha10-specific targeting components include DNA-probes, antisense RNAs or RNAi, such as microRNAs, short interfering RNAs (siRNA) and short hairpin RNAs (shRNA).

Typical methods for detection of nucleic acids well known in the art include but are not limited to Northern blotting, Southern blotting, polymerase chain reaction (PCR), microarrays, in situ hybridization etc.

In a further embodiment the presence of integrin alpha10 in a biological sample is detected by using an integrin alpha10 binding peptide or protein. Such peptides or proteins can be made recombinant, chemically synthesized, or purified from a natural source.

In a further embodiment the presence of integrin alpha10 in a biological sample is detected *in vivo* by using an integrin alpha10-specific antibody, or an integrin alpha10 binding peptide or protein, or an integrin alpha10 nucleic acid probe which binds to integrin alpha10 RNA or cDNA in a hybridizing reaction.

Typical methods for detection of cell surface antigens and polynucleotides *in vivo* are well known in the art include but are not limited to positron emission tomography, x-ray computed tomography (CT), magnetic resonance imaging (MRI) and functional magnetic resonance imaging (fMRI), ultrasound and single-photon emission computed tomography (SPECT). In particular cell surface antigens can be imaged *in vivo* using immunolabelling with a radioactive tracer bound to an antibody or other specifically binding protein.

Preferably the antibodies used for *in vivo* imaging are antibody fragments such as Fab fragments, and single chain antibodies due to their smaller size and absence of effector function.

### Items

1. A composition comprising:
   a. an antibody or antigen-binding fragment thereof, specifically binding to an integrin alpha10 polypeptide, or
   b. a polynucleotide specifically binding to a polynucleotide transcript which encodes an integrin alpha10 polypeptide or a fragment or variant thereof,
   for use in the treatment and/or prevention of a cancer form selected from the group consisting of breast cancer, lung cancer, prostate cancer, pancreatic cancer and sarcoma, or a metastasis of any one of said cancer forms.
2. The composition for use according to any one of the preceding items, wherein the breast cancer is selected from the group consisting of triple negative breast cancer and inflammatory breast cancer.
3. The composition for use according to any one of the preceding items, wherein the triple negative breast cancer is selected from the group consisting of basal-like 1 breast cancer, basal-like 2 breast cancer, claudin-low breast cancer, metaplastic breast cancer (MBC), interferon-rich breast cancer, immunomodulatory breast cancer, mesenchymal breast cancer, mesenchymal stem-like breast cancer, luminal androgen receptor breast cancer and unstable breast cancer.
4. The composition for use according to any one of the preceding items, wherein the lung cancer is selected from the group consisting of squamous cell lung carcinoma, lung adenocarcinoma, large cell lung carcinoma and small-cell lung carcinoma.
5. The composition for use according to any one of the preceding items, wherein the prostate cancer is small cell neuroendocrine carcinoma (SCNC).
6. The composition for use according to any one of the preceding items, wherein the pancreatic cancer is a neuroendocrine tumor.
7. The composition for use according to any one of the preceding items, wherein the pancreatic cancer and/or neuroendocrine tumor is a grade I, grade II or grade III pancreatic cancer.
8. The composition for use according to any one of the preceding items, wherein the sarcoma is selected from the group consisting of undifferentiated pleomorphic sarcoma, myxofibrosarcoma, dedifferentiated liposarcoma, atypical lipomatous tumor, myxoinflammatory fibroblastic sarcoma, low grade fibromyxoid sarcoma, sclerosing epithelioid fibrosarcoma, pseudimyogenic hemangioendothelioma and mesenchymal chondrosarcoma.
9. The composition for use according to any one of the preceding items, wherein the integrin alpha10 polypeptide is expressed on the surface of a malignant cell and/or a tumor-associated cell.
10. The composition for use according to any one of the preceding items, wherein the antibody is a monoclonal antibody, polyclonal antibody, a chimeric antibody, a single chain antibody or fragment thereof.
11. The composition for use according to any one of the preceding items, wherein the antibody is a non-human antibody, a chimeric antibody, a bispecific antibody, a humanized antibody or a human antibody.
12. The composition for use according to any one of the preceding items, wherein the antibody is a mouse monoclonal antibody.
13. The composition for use according to any one of the preceding items, wherein the antibody is a human monoclonal antibody.
14. The composition for use according to any one of the preceding items, wherein the antibody has an isotype selected from the group consisting of IgA, IgD, IgG, IgE and IgM.
15. The composition for use according to any one of the preceding items, wherein the antibody is:
   a) a monoclonal antibody, produced by the hybridoma cell line deposited at the Deutsche Sammlung von Mikroorganismen und Zellkulturen GmbH under the accession number DSM ACC2583; or
   b) an antibody which competes for binding to the same epitope as the epitope bound by the monoclonal antibody produced by the hybridoma deposited at the Deutsche Sammlung von Mikroorganismen und Zellkulturen GmbH under the accession number DSM ACC2583; or
   c) a fragment of a) or b), wherein said fragment is capable of binding specifically to the extracellular I-domain of the integrin alpha10 polypeptide chain.
16. The composition for use according to any one of the preceding items, wherein the antibody or antigen-binding fragment thereof comprises:
   a heavy chain variable region comprising
      a) a CDR-H1 comprising or consisting of the amino acid sequence of SEQ ID NO: 4;
      b) a CDR-H2 comprising or consisting of the amino acid sequence of SEQ ID NO: 5; and
      c) a CDR-H3 comprising or consisting of the amino acid sequence of SEQ ID NO: 6;
      and/or
   a light chain variable region comprising
      d) a CDR-L1 comprising or consisting of the amino acid sequence of SEQ ID NO: 7;
      e) a CDR-L2 comprising or consisting of the amino acid sequence of SEQ ID NO: 8; and
      f) a CDR-L3 comprising or consisting of the amino acid sequence of SEQ ID NO: 9.
   or a variant of any one of SEQ ID NO:s 4 to 9, wherein any one amino acid has been altered for another amino acid, with the proviso that no more than 3 amino acids have been so altered, for example wherein 2, or 1 amino acids have been so altered.
17. The composition for use according to any one of the preceding items, wherein the antibody or antigen-binding fragment thereof comprises a heavy chain variable region comprising or consisting of the amino acid sequence of SEQ ID NO: 10.
18. The composition for use according to any one of the preceding items, wherein the antibody or antigen-binding fragment thereof comprises a light chain variable region comprising or consisting of the amino acid sequence of SEQ ID NO: 11.
19. The composition for use according to any one of the preceding items, wherein the antibody or antigen-binding fragment thereof and/or the polynucleotide or fragment or variant thereof is conjugated to an additional moiety.
20. The composition for use according to any one of the preceding items, wherein the additional moiety comprises a detectable moiety, such as a detectable moiety selected from the group consisting of a fluorophore, an enzyme and a radioactive tracer or radioisotope.
21. The composition for use according to any one of the preceding items, wherein the additional moiety comprises a cytotoxic moiety.
22. The composition for use according to any one of the preceding items, wherein the cytotoxic moiety is selected from a group consisting of a toxin, a chemotherapeutic agent and a radioactive agent, or combinations thereof.
23. The composition for use according to any one of the preceding items, wherein the cytotoxic moiety is a toxin.
24. The composition for use according to any one of the preceding items wherein said toxin is selected from the group selected from microtubule toxins, DNA toxins and transcription toxins.
25. The composition for use according to any one of the preceding items wherein said microtubule toxins are selected from the group consisting of Auristatin-based toxins, Maytansinoid-based toxins, Tubulysins-based toxins and Eribulin.
26. The composition for use according to any one of the preceding items wherein said DNA toxins are selected from the group consisting of DNA major-groove binding agents, DNA minor-groove binding alkylating agents, DNA alkylating agents and DNA-cleaving agents.
27. The composition for use according to any one of the preceding items wherein said DNA toxins are selected from the group consisting of Pyrrolobenzodiazepine (PBD), Duocarmycin, Duocarmycin analogues, Indolino-benzodiazepine, Calicheamicins, Irinotecan and Exatecan derivatives.
28. The composition for use according to any one of the preceding items wherein said transcription toxin is an RNA polymerase II inhibiting agent.
29. The composition for use according to any one of the preceding items wherein said transcription toxin is selected from the group consisting of Doxorubicin, Doxorubicin derivatives and Amanitin.
30. The composition for use according to any one of the preceding items, wherein the transcription toxin is selected from the group consisting of shiga and shiga-like toxins; type I ribosome inactivating proteins, type II ribosome inactivating proteins and saporin, or combinations thereof.
31. The composition for use according to any one of the preceding items, wherein the type I ribosome inactivating protein is trichosanthin and/or luffin.
32. The composition for use according to any one of the preceding items, wherein the type II ribosome inactivating protein is ricin, agglutinin and/or abrin.
33. The composition for use according to any one of the preceding items, wherein the additional moiety comprises a biological response modifier.
34. The composition for use according to any one of the preceding items, wherein the biological response modifier is a cytokine, a lymphokine, an interferon or combinations thereof.
35. The composition for use according to any one of the preceding items, wherein the chemotherapeutic agent is an alkylating agent, an antimetabolite, an anti-microtubule agent, a topoisomerase inhibitor or a cytotoxic antibiotic.
36. The composition for use according to any one of the preceding items, wherein the chemotherapeutic agent is selected from the group consisting of anthracyclines, taxanes and platinum agents.
37. The composition for use according to any one of the preceding items, wherein the chemotherapeutic agent is selected from the group consisting of cisplatin, paclitaxel, albumin-bound paclitaxel, docetaxel, cyclophosphamide, eribulin, epirubicin, doxorubicin, carboplatin, gemcitabine, bleomycin, fluorouracil, cyclophosphamide, vinorelbine, capecitabine, ixabepilone and ixabepilone, or combinations thereof.
38. The composition for use according to any one of the preceding items, wherein the composition further comprises at least one pharmaceutically acceptable diluent, carrier or excipient.
39. The composition for use according to any one of the preceding items, wherein the integrin alpha10 polypeptide is a naturally occurring variant of integrin alpha10 polypeptide, an isoform of integrin alpha10 polypeptide or a splice variant of an integrin alpha10 polypeptide.
40. The composition for use according to any of the preceding items, wherein the antibody and/or the polynucleotide is capable of inducing cell death and/or inhibiting growth and/or inhibiting proliferation and/or inhibiting migration of cells expressing an integrin alpha10 polypeptide and/or polynucleotide transcript.
41. The composition for use according to any of the preceding items, wherein the cells are malignant cells and/or tumor-associated cells.
42. The composition for use according to any of the preceding items, wherein the malignant cells or tumor-associated cells are cancer associated fibroblast (CAFs), stromal cells, stem cells and/or stem-like cells.
43. The composition for use according to any one of the preceding items, wherein the integrin alpha10 polypeptide is a part of an integrin alpha10 beta1 heterodimer.
44. The composition for use according to any of the preceding items, wherein the treatment is prophylactic, ameliorative or curative.
45. The composition for use according to any of the preceding items, wherein the treatment is initiated upon detection of an integrin alpha10 polypeptide and/or polynucleotide transcript in a cancer cell in a tumor of said subject.
46. The composition for use according to any of the preceding items, wherein the composition is administered to an individual in need thereof in combination with radiation therapy and/or surgical removal of cancer.
47. The composition for use according to any of the preceding items, wherein the composition is administered to an individual in need thereof prior to radiation therapy and/or surgical removal of cancer.
48. The composition for use according to any of the preceding items, wherein the composition is administered to an individual in need thereof after radiation therapy and/or surgical removal of cancer.
49. A composition comprising:
   a) an antibody or antigen-binding fragment thereof, specifically binding to an integrin alpha10 polypeptide, or
   b) a polynucleotide specifically binding to a polynucleotide transcript which encodes an integrin alpha10 polypeptide or a fragment or variant thereof,
   for use in the diagnosis of a cancer form selected from the group consisting of breast cancer, lung cancer, prostate cancer, pancreatic cancer, and sarcoma, or a metastasis of any one of said cancer forms.
50. The composition according to item 49 wherein the cancer comprises cells which display equal or higher levels of i) the integrin alpha10 antigen or ii) the polynucleotide transcript observed in healthy and/or benign tissue of the same type.
51. The composition according to item 49 wherein the cancer comprises cells which display equal or higher levels of i) the integrin alpha10 antigen or ii) the polynucleotide transcript observed in a less aggressive cancer type of the same tissue type, compared to the diagnosed cancer type.
52. The composition according to item 49, wherein the cancer comprises cells which display equal or higher expression levels of i) the integrin alpha10 antigen or ii) the polynucleotide transcript than in a reference cell line.
53. The composition according to item 52, wherein the reference cell line is derived from healthy tissue.
54. The composition according to any one of items 52 to 53, wherein the reference cell line is selected from the group consisting of a reference cell line derived from healthy breast tissue, a reference cell line derived from healthy prostate tissue, a reference cell line derived from healthy lung tissue, a reference cell line derived from healthy pancreas tissue and a reference cell line derived from healthy connective tissue.
55. The composition according to any one of items 52 to 54, wherein the reference cell line is the cell line 184A1 derived from healthy breast cancer tissue.
56. The composition according to item 52, wherein the reference cell line is derived from a less aggressive cancer type of the same tissue type, compared to the diagnosed cancer type.
57. The composition according to item 56, wherein the reference cell line is selected from the group consisting of a reference cell line derived from less aggressive breast cancer, a reference cell line derived from less aggressive prostate cancer, a reference cell line derived from less aggressive lung cancer, a reference cell line derived from less aggressive pancreatic cancer, and a reference cell line derived from less aggressive sarcoma.
58. The composition according to any one of items 56 to 57, wherein the reference cell line is selected from the group consisting of cell line HCC1428 derived from a less aggressive breast cancer, cell line T47D derived from a less aggressive breast cancer, cell line 22Rv1 derived from a less aggressive prostate cancer, cell line DU145 derived from a less aggressive prostate cancer, cell line BxPC-3 derived from a less aggressive pancreatic cancer and cell line AsPC-1derived from a less aggressive pancreatic cancer.
59. A method of treating a cancer form, wherein said cancer form is selected from the group consisting of breast cancer, lung cancer, prostate cancer, pancreatic cancer and sarcoma, or wherein said cancer form is a metastasis, the method comprising administering a pharmaceutically effective amount of a composition comprising:
   a. an antibody specifically binding to an integrin alpha10 polypeptide or a fragment thereof, or
   b. a polynucleotide specifically binding to a polynucleotide transcript which encodes an integrin alpha10 polypeptide or a fragment or variant thereof,
   to a subject in the need thereof.
60. A method for detecting a cancer cell in a subject, said method comprising the steps of:
   a. providing a tissue suspected of comprising cancer cells of the subject;
   b. analyzing in the tissue the presence of:
      i. an antigen comprising an integrin alpha10 polypeptide or a fragment thereof; and/or
      ii. a polynucleotide transcript which encodes an integrin alpha10 polypeptide or a fragment or variant thereof,
   c. determining the expression level of the i) integrin alpha10 antigen and/or ii) polynucleotide transcript, and
   d. comparing said expression level determined in c. with a control level,

   wherein said control level is the average expression level of i) the antigen or ii) the polynucleotide transcript observed in healthy and/or benign cells of the same tissue type as the isolated sample,
   wherein an expression level of i) the antigen and/or ii) the polynucleotide transcript higher than the control level is indicative of the presence of a cancer form in the subject, and wherein said cancer form is selected from the group consisting of breast cancer, lung cancer, prostate cancer, pancreatic cancer and sarcoma, or a metastasis of any one of said cancer forms.
61. A method for detecting a cancer cell in a subject, said method comprising the steps of:
   a. providing a tissue suspected of comprising cancer cells of the subject;
   b. analyzing in the tissue the presence of one or more cells having a cancer morphology,
   c. analyzing in the tissue the presence of:
      i. an antigen comprising an integrin alpha10 polypeptide or a fragment thereof; and/or
      ii. a polynucleotide transcript which encodes an integrin alpha10 polypeptide or a fragment or variant thereof,
   d. optionally determining the expression level of the i) integrin alpha10 antigen and/or ii) polynucleotide transcript,
   wherein presence of one or more cells having a cancer morphology in combination with expression of integrin alpha10 i) antigen and/or ii) polynucleotide transcript are indicative of the presence of a cancer form in the subject, and wherein said cancer form is selected from the group consisting of breast cancer, lung cancer, prostate cancer, pancreatic cancer and sarcoma, or a metastasis of any one of said cancer forms.
62. A method for diagnosis of a cancer form in a subject, the method comprising the steps of:
   a. providing a tissue suspected of comprising cancer cells of the subject;
   b. analyzing in the tissue the presence of:
      i. an antigen comprising an integrin alpha10 polypeptide or a fragment thereof; and/or
      ii. a polynucleotide transcript which encodes an integrin alpha10 polypeptide or a fragment or variant thereof,
   c. determining the expression level of the i) integrin alpha10 antigen and/or ii) polynucleotide transcript, and
   d. comparing the expression level determined in c. with a control level, wherein said control level is the average expression level of i) the antigen or ii) the polynucleotide transcript observed in healthy and/or benign tissue of the same type; and

   wherein an expression level of i) the antigen and/or ii) the polynucleotide transcript higher than the control level is indicative of the presence of an cancer form in a sample, wherein said cancer form is selected from the group consisting of breast cancer, lung cancer, prostate cancer, pancreatic cancer and sarcoma, or a metastasis of any one of said cancer forms,
   thereby diagnosing a cancer form in a subject.
63. A method for diagnosis of an cancer form in a subject, the method comprising the steps of:
   a. providing a tissue suspected of comprising cancer cells of the subject;
   b. analyzing in the tissue the presence of one or more cells having a cancer morphology;
   c. analyzing in the sample the presence of:
      i. an antigen comprising an integrin alpha10 polypeptide or a fragment thereof; and/or
      ii. a polynucleotide transcript which encodes an integrin alpha10 polypeptide or a fragment or variant thereof,
   d. optionally determining the expression level of the i) integrin alpha10 antigen and/or ii) polynucleotide transcript, and

   wherein presence of one or more cells having a cancer morphology in combination with expression of integrin alpha10 i) antigen and/or ii) polynucleotide transcript are indicative of the presence of an cancer form in a sample, wherein said cancer form is selected from the group consisting of breast cancer, lung cancer, prostate cancer, pancreatic cancer and sarcoma, or a metastasis of any one of said cancer forms,
   thereby diagnosing an cancer form in a subject.
64. The method according to any one of items 60 to 63, wherein step b. further comprises administering to said subject a composition according to any one of items 1 to 58, or a fragment thereof.
65. The method according to any one of items 60 to 64, further comprising morphologically characterizing the sample as comprising cancer cells belonging to a cancer form, wherein said cancer form is selected from the group consisting of breast cancer, lung cancer, prostate cancer, pancreatic cancer, and sarcoma, or a metastasis of any one of said cancer forms.
66. The method according to any one of items 60 to 65, wherein said method is for detecting the presence of breast cancer in a subject or for diagnosis of breast cancer in a subject, wherein said breast cancer is a breast cancer form selected from the group consisting of triple negative breast cancer and inflammatory breast cancer, wherein said method comprises comparing the expression level of the integrin alpha10 polypeptide determined in b. with a control level, wherein said control level is the average expression level of i) the antigen or ii) the polynucleotide transcript observed in healthy and/or benign breast tissue cells, and wherein a level of the antigen comprising an integrin alpha10 polypeptide higher than the control level is indicative of the presence of breast cancer in the subject.
67. The method according to any one of items 60 to 65, wherein said method is for detecting the presence of lung cancer in a subject, or for diagnosis of lung cancer in a subject, wherein said method comprises comparing the expression level of the integrin alpha10 polypeptide determined in b. with a control level, wherein said control level is the average expression level of i) the antigen or ii) the polynucleotide transcript observed in non-cancerous lung cells, and wherein a level of the antigen comprising an integrin alpha10 polypeptide higher than the control level is indicative of the presence of lung cancer in the subject.
68. The method according to any one of items 60 to 65, wherein said method is for detecting the presence of squamous lung cell carcinoma in a subject, or for diagnosis of squamous lung cell carcinoma in a subject, wherein said method comprises comparing the expression level of the integrin alpha10 polypeptide determined in b. with a control level, wherein said control level is the average expression level of i) the antigen or ii) the polynucleotide transcript observed in non-cancerous lung cells, and wherein a level of the antigen comprising an integrin alpha10 polypeptide higher than the control level is indicative of the presence of squamous lung cell carcinoma in the subject.
69. The method according to any one of items 60 to 65, wherein said method is for detecting the presence of lung adenocarcinoma in a subject, or for diagnosis of lung adenocarcinoma in a subject, wherein said method comprises comparing the expression level of the integrin alpha10 polypeptide determined in b. with a control level, wherein said control level is the average expression level of i) the antigen or ii) the polynucleotide transcript observed in non-cancerous lung cells, and wherein a level of the antigen comprising an integrin alpha10 polypeptide higher than the control level is indicative of the presence of lung adenocarcinoma in the subject.
70. The method according to any one of items 60 to 65, wherein said method is for detecting the presence of a prostate cancer cell in a subject, or for diagnosis of prostate cancer in a subject, wherein said method comprises comparing the expression level of the integrin alpha10 polypeptide determined in b. with a control level, wherein said control level is the average expression level of i) the antigen or ii) the polynucleotide transcript observed in non-cancerous prostate cells, and wherein a level of the antigen comprising an integrin alpha10 polypeptide higher than the control level is indicative of the presence of an form of prostate cancer in the subject.
71. The method according to any one of items 60 to 65, wherein said method is for detecting the presence of a pancreatic cancer cell in a subject, or for diagnosis of pancreatic cancer in a subject, wherein said method comprises comparing the expression level of the integrin alpha10 polypeptide determined in b. with a control level, wherein said control level is the average expression level of i) the antigen or ii) the polynucleotide transcript observed in non-cancerous pancreas cells, and wherein an expression level of the integrin alpha10 polypeptide higher than the control level is indicative of the presence of an form of pancreatic cancer in the subject.
72. The method according to any one of items 60 to 65, wherein said method is for detecting the presence of sarcoma in a subject, or for diagnosis of sarcoma in a subject, wherein said method comprises comparing the expression level of the integrin alpha10 polypeptide determined in b. with a control level, wherein said control level is the average expression level of i) the antigen or ii) the polynucleotide transcript observed in non-cancerous sarcoma cells, and wherein a level of the antigen comprising an integrin alpha10 polypeptide higher than the control level is indicative of the presence of sarcoma in the subject.
73. A method for classification of a triple negative breast cancer tumor sample of a subject, said method comprising:
   a. providing a breast tissue suspected of comprising cancer cells of the subject;
   b. isolating breast cancer cells characterized as being ER negative, PR negative and HER2 negative;
   c. determining in the isolated cells an expression level of:
      i. an antigen comprising an integrin alpha10 polypeptide or a fragment thereof; and/or
      ii. a polynucleotide transcript which encodes an integrin alpha10 polypeptide or a fragment or variant thereof, and
   d. comparing the expression level determined in c. to a control level, wherein said control level is the average expression level of i) the antigen or ii) the polynucleotide transcript observed in healthy and/or benign breast tissue;

   wherein an expression level of i) the antigen and/or ii) the polynucleotide transcript in the breast cancer cells higher than a control level, and an expression status of ER negative, PR negative and HER2 negative are indicative of a basal-like triple negative breast cancer or a luminal triple negative breast cancer,
   thereby classifying the triple negative breast cancer tumor sample as belonging to a basal-like triple negative breast cancer tumor or to a luminal triple negative breast cancer tumor.
74. The method according to item 73, wherein expression of integrin alpha10 and one or more polypeptides selected from the group consisting of cytokeratin 7, cytokeratin 8, cytokeratin 18 and cytokeratin 19 is indicative of luminal triple negative breast cancer.
75. The method according to item 73, wherein expression of integrin alpha10 and one or more polypeptides selected from the group consisting of cytokeratin 5/6, cytokeratin 14, cytokeratin 17, p63, EGFR, 34BE12 and c-kit/CD117 is indicative of basal-like triple negative breast cancer.
76. A method for determining a prognosis for a cancer form for a subject, the method comprising:
   a. providing a cancer tumor tissue of the subject;
   b. analyzing in the sample the presence of:
      i. an antigen comprising an integrin alpha10 polypeptide or a fragment thereof; and/or
      ii. a polynucleotide transcript which encodes an integrin alpha10 polypeptide or a fragment or variant thereof,
   c. determining the expression level of the i) integrin alpha10 antigen and/or ii) polynucleotide transcript,
   d. comparing the expression level determined in c. to a control level, wherein the control level is the average expression level of i) the antigen and/or ii) the polynucleotide transcript observed in healthy and/or benign tissue of the same tissue type as the sample;
   e. determining an unfavorable prognosis of the cancer form when the expression level of the integrin alpha10 i) antigen and/or ii) polynucleotide transcript is higher than the control level,
   wherein the cancer form is selected from the group consisting of breast cancer, lung cancer, prostate cancer, pancreatic cancer and sarcoma, or a metastasis of any one of said cancer forms.
77. A method for determining a prognosis for a cancer form for a subject, the method comprising:
   a. providing a cancer tumor tissue of the subject;
   b. optionally analyzing in the tissue the presence of one or more cells having a cancer morphology;
   c. analyzing in the sample the presence of:
      i. an antigen comprising an integrin alpha10 polypeptide or a fragment thereof; and/or
      ii. a polynucleotide transcript which encodes an integrin alpha10 polypeptide or a fragment or variant thereof,
   d. optionally determining the expression level of the i) integrin alpha10 antigen and/or ii) polynucleotide transcript, and comparing the determined expression level to a control level, wherein the control level is the average expression level of i) the antigen and/or ii) the polynucleotide transcript observed in healthy and/or benign tissue of the same tissue type as the sample;
   e. determining an unfavorable prognosis of the cancer form when one or more cells having a cancer morphology are present in the tissue in combination with expression of the integrin alpha10 i) antigen and/or ii) polynucleotide transcript, and/or
      the expression level of the integrin alpha10 i) antigen and/or ii) polynucleotide transcript is higher than the control level,
   wherein the cancer form is selected from the group consisting of breast cancer, lung cancer, prostate cancer, pancreatic cancer and sarcoma, or a metastasis of any one of said cancer forms.
78. The method according to any one of items 76 to 77, wherein the prognosis is overall survival rate or recurrence free survival rate.
79. The method according to any one of items 60 to 78, wherein the step of analyzing in the sample the presence of:
   i. an antigen comprising an integrin alpha10 polypeptide or a fragment thereof; and/or
   ii. a polynucleotide transcript which encodes an integrin alpha10 polypeptide or a fragment or variant thereof,
   comprises imaging the tissue and/or tissue sample.
80. The method according to any one of items 60 to 79, wherein the step of determining the expression level of the i) integrin alpha10 antigen and/or ii) polynucleotide transcript comprises imaging the tissue and/or tissue sample.
81. A method of preventing metastasis from a primary cancer form selected from the group consisting of breast cancer, lung cancer, prostate cancer, pancreatic cancer and sarcoma, the method comprising administering a therapeutically effective amount of:
   a) an antibody or antigen-binding fragment thereof, wherein the antibody or antigen binding fragment is specific for integrin alpha10 polypeptide; and/or
   b) a polynucleotide transcript which encodes an integrin alpha10 polypeptide or a fragment or variant thereof,
   to a patient in need thereof.
82. The method according to item 81 wherein the a) antibody or antigen-binding fragment and/or b) polynucleotide transcript is administered upon detection of the primary cancer.
83. A method of inhibiting integrin alpha10-mediated signaling of at least one cancer cell, the method comprising contacting the at least one cancer cell with a composition comprising an effective amount of:
   a. an antibody or antigen-binding fragment specific for integrin alpha10 polypeptide; and/or
   b. a polynucleotide transcript which encodes an integrin alpha10 polypeptide or a fragment or variant thereof,
   wherein the at least one cancer cell is selected from the group consisting of an breast cancer cell, an lung cancer cell, an prostate cancer cell, an pancreatic cancer cell, an sarcoma cell and a metastatic tumor cell.
84. A method of inhibiting cellular functions of at least one cancer cell, the method comprising contacting the at least one cancer cell with a composition comprising an effective amount of:
   a. an antibody or antigen-binding fragment specific for integrin alpha10 polypeptide; and/or
   b. a polynucleotide transcript which encodes an integrin alpha10 polypeptide or a fragment or variant thereof,
   wherein the at least one cancer cell is selected from the group consisting of a breast cancer cell, a lung cancer cell, a prostate cancer cell, a pancreatic cancer cell, a sarcoma cell and a metastatic tumor cell.
85. The method according to any one of items 60 to 84, wherein the antigen comprising an integrin alpha10 polypeptide or a fragment thereof is expressed on the surface of the cells.
86. The method of inhibiting cellular functions of at least one cancer cell according to any one of items 84 to 85, wherein the at least one cancer cell is in a tumor and/or a metastatic tumor, and wherein inhibiting is selected from the group consisting of:
   a. inhibiting proliferation of the at least one cancer cell;
   b. inhibiting self-renewal of the at least one cancer cell;
   c. inhibiting anchorage-independent growth of the at least one cancer cell;
   d. inhibiting migration of the at least one cancer cell;
   e. inhibiting invasion of the at least one cancer cell;
   f. inhibiting viability of the at least one cancer cell;
   g. inhibiting adhesion of the at least one cancer cell; and/or combinations thereof.
87. The method of inhibiting cellular functions of at least one cancer cell according to any one of items 84 to 86, wherein the at least one cancer cell is in a tumor and/or a metastatic tumor, and inhibiting cellular functions of the at least one cancer cell inhibits at least one of:
   a. growth of the tumor and/or metastatic tumor;
   b. proliferation of the tumor and/or metastatic tumor;
   c. migration of the tumor and/or metastatic tumor;
   d. invasion of the tumor and/or metastatic tumor;
   e. spreading of new tumor and/or metastatic tumors;
   f. initiation of new tumor and/or metastatic tumors;
   g. infiltration of new tumor and/or metastatic tumors; and/or combinations thereof.
88. The method according to any one of items 83 to 87, wherein the at least one cancer cell is characterized by an expression level of:
   a. an antigen comprising an integrin alpha10 polypeptide or a fragment thereof; and/or
   b. a polynucleotide transcript which encodes an integrin alpha10 polypeptide or a fragment or variant thereof,
   higher than a control level, wherein the control level is the average expression level of a. the antigen and/or b. the polynucleotide observed in healthy and/or benign cells of the same tissue type as the cancer cell.
89. The method and/or composition according to any one of items 49 to 88, wherein the composition is according to any one of items 1 to 48.
90. The method according to any one of items 59 to 89, wherein the method further comprises administering to said subject a composition according to any one of items 1 to 48, or a fragment thereof.
91. The method according to any one of items 60 to 90, wherein the method is an *in vitro* method, and wherein the tissue is a tissue sample obtained from the subject.
92. The composition according to claim 49, wherein the diagnosis is an *in vitro* diagnosis.
93. Use of composition comprising:
   a. an antibody specifically binding to an integrin alpha10 polypeptide or a fragment thereof, or
   b. a polynucleotide specifically binding to a polynucleotide transcript which encodes an integrin alpha10 polypeptide or a fragment or variant thereof,
   for the manufacture of a medicament for the treatment and/or prevention of a cancer form selected from the group consisting of breast cancer, lung cancer, prostate cancer and pancreatic cancer, or a metastasis of any one of said cancer forms.
94. The composition, use or method according to any one of the preceding items 1 to 93, wherein the cancer form is an aggressive cancer form selected from the group consisting of aggressive breast cancer, aggressive lung cancer, aggressive prostate cancer, aggressive pancreatic cancer and aggressive sarcoma.

### Examples

### Example 1: Protein expression of integrin alpha10 in tissues from aggressive tumors including breast, lung, pancreatic tumor and sarcoma compared to expression in unaffected tissue regions as visualized by immunohistochemistry

### Material and methods:

Human material consisting of breast, lung, pancreatic and sarcoma tissue specimens was used. A standard protocol for immunohistochemistry (see Renshaw 2007, ISBN10: 1 904842 038, Scion Publishing Ltd, UK) was optimized (see below), using the HRP polymer conjugated secondary antibodies (DAKO Envision anti-rabbit, DK) followed by reaction with di-aminobenzidine and hydrogen peroxide.

For breast and pancreas tissues: Paraffin sections (4 or 8µm) were used.

Sections were de-paraffinized and rehydrated via immersion of slides in xylene followed by an ethanol series and water according to standard protocols. Optimization of the labelling protocol included treatment of the paraffin sections for antigen retrieval by immersing slides in an acidic buffered solution (citrate buffer: 10 mM Sodium citrate, 0.05% Tween 20, pH 6.0) followed by heat treatment (at 92-95°C). For lung and sarcoma tissues: Cryosections (8 µm), and the same protocol as described above, were used. Optimization of the labelling protocol included post-fixation of the cryosections with acetone (100%) at -20°C and quenching in 0.3% hydrogen peroxide, before blocking (PBS with 1% BSA and 0.05% Triton-X100). 3 x 3 min rinses in PBS were performed between pre-treatments and incubation steps. Triton-X100 (0.05%) was added to the primary antibody dilution buffer. 3 x 3 min rinses in PBS were performed between pre-treatments and incubation steps. Sections were counterstained with Mayer's Hematoxylin to visualize nuclei..

The primary antibody used was a polyclonal rabbit anti-integrin alpha10 antibody, at a concentration of 3 µg/ml.

### Results:

Integrin alpha 10 is specifically and strongly expressed in aggressive cancer forms including invasive ductal carcinoma (Figure 1A) and in triple negative breast cancer tissue (Figure 2), whereas negligible expression of integrin alpha10 was seen in morphologically unaffected breast tissue (Figure 1B). As expected, integrin alpha10 was localized on the cell membrane (arrows).

In addition, integrin alpha10 was distinctly expressed on squamous cell lung carcinoma cells (data not shown) and in pancreatic cancer cells and proliferating islet cells in pancreatic ductal adenocarcinoma tissue (data not shown) but not in the surrounding morphologically healthy tissue. Integrin alpha 10 was distinctly expressed on undifferentiated pleomorphic sarcoma cells (Figure 3).

### Conclusion:

The results demonstrate that integrin alpha10 is distinctly expressed in patient tissue obtained from several different cancer forms considered to be the most aggressive ones.

### Example 2: Integrin alpha10 expression in breast and prostate cancer cell lines visualized by immunofluorescence

### Material and methods:

Cells (originally obtained from the American Type Culture Collection-ATCC) of the triple negative breast cancer cell lines BT549, and the luminal A breast cancer cell line T47D were grown as monolayer and cells of the prostate cancer cell line PC-3 were grown as spheres on Ibidi µ-Slide 8 Well microscopy slides (Ibidi Labware, Germany). The immunofluorescence protocol described in Example 2 of PCT/EP2017/070838 was followed. The primary antibody used was an alternative mouse monoclonal antibody against integrin alpha10, used at 1,7 µg/ml. The secondary antibody was fluorophore conjugated antibody (anti-mouse AF488 or AF647 conjugated and/or anti-rabbit AF488 or RodRX, all made in donkey, from Jackson Immunoresearch, USA). All secondary antibodies were diluted 1: 200, in PBS containing 1% BSA, and incubated for 30 min.

### Results:

Immunofluorescence followed by confocal microscopy showed that integrin alpha10 was specifically and strongly expressed on cell membranes both in the two different breast cancer cell lines (Figure 4A-B) and in the prostate cancer cell line (Figure 4C).

### Conclusion:

The results demonstrate that integrin alpha10 is distinctly expressed in cell lines from different aggressive cancers.

### Example 3: Monolayer culturing of human non-malignant and cancer cell lines and flow cytometry analysis of integrin alpha10 protein expression

### Material and methods:

Breast cancer cell lines: 184A1, HCC1428, T47D, MDA-MB-231 and BT549; lung cancer cell lines: A549 (adenocarcinoma) and U-1752 (squamous cell lung carcinoma); prostate cancer cell lines: 22Rv1, Du145 and PC-3; pancreatic cancer cell lines: BxPC-3, AsPC-1, PANC-1 and MiaPaCa-2 were originally obtained from the American Type Culture Collection (ATCC).

The 184A1 cell line was established from healthy mammary tissue obtained from a reduction mammoplasty. Cells derived from the tissue were exposed to benzo(a)pyrene, and a transformed line was established. The line appears to be that the cells are immortal, but not malignant. All other cell lines are derived from malignant tumor and are malignant cells (see table below).

Immunostaining of cells was performed by incubating cells with an alternative mouse monoclonal anti-integrin 10 antibody (Alexa Fluor 647 conjugate), at a concentration of 1,0 µg/ml, for 30 min in dark at 4°C. After 30 min incubation with primary antibody, cells were washed twice with DPBS (Hyclone, SH3002802) containing 1% FBS and 0.1% sodium azide prior to flow cytometry analysis using a BD Accuri C6 flow cytometer.

**Table1. Overview over cell lines used in the present study, and their aggressivity**

| | **Non-malignant** | **Less aggressive** | **Aggressive** |
|---|---|---|---|
| **Breast cancer** | 184A1 | HCC1428 | BT549 |
| | | T47D | MDA-MB-231 |
| **Prostate cancer** | | 22Rv1 | PC-3 |
| | | DU145 | |
| **Pancreatic cancer** | | BxPC-3 | PANC-1 |
| | | AsPC-1 | MiaPaCa-2 |
| **Lung cancer** | | | A549 |
| | | | U-1752 |
| **Sarcoma cancer** | | | MFH152 |

### Results:

The results from breast cancer showed that integrin alpha10 is moderately expressed in T47D and highly expressed in triple negative cell line BT549 cultured as monolayer (Figure 5A and 5B). The aggressive prostate cancer cells PC-3 showed the highest expression of integrin alpha10 when cultured as monolayer (Figure 5E and 5F). In addition, the most invasive and high-grade pancreatic cancer cell lines (grade III), MiaPaCa-2 and PANC-1, also showed the highest integrin alpha10 expression (Figure 5I). In contrast, less aggressive pancreatic cancer cell lines, BxPC-3 and AsPC-1, showed no or less integrin alpha10 expression, especially evident in sphere culture (see Example 4, Figure 5J). Monolayer-cultured lung cancer cells showed very low expression (Figure 5K), which however increased when the cells were cultured as spheres (Figure 5L and Experiment 4).

### Conclusion:

The results demonstrate that integrin alpha10 expression is highest in the most aggressive cancer types. This suggests a correlation between integrin alpha10 and aggressiveness of different cancers, and thereby possibly with migration and invasiveness. This observation can readily be seen in monoculture systems, and is even more visible in the three-dimensional (3D) culture system (see Experiment 4). 3D cell culture models are better models than the traditional 2D monolayer culture due to improved cell-cell interactions, cell-ECM interactions, and cell populations and structures that resemble *in vivo* architecture.

### Example 4: Detection and analysis of integrin alpha10 protein in sphere formation assays

### Material and methods:

Breast cell lines 184A1, HCC1428, T47D, MDA-MB-231 and BT549 cells, or lung cancer cell lines A549 and U-1752, or prostate cancer cell lines 22Rv1, DU145 and PC-3 cells, or pancreatic cancer cell lines BxPC-3, AsPC-1, PANC-1 and MiaPACa-2, or sarcoma cell lines MFH152 were seeded in ultra-low attachment plates (CLS3471, Corning) in serum-free media to form non-adherent spheres (breast cancer: termed mammospheres, prostate cancer: termed prostasphere), with the ability to self-renew. Non-scaffold-based cultures are formed by cellular aggregates commonly known as spheroids or sphere. Plating medium for sphere formation consisted of DMEM/F12 (1:1) w/Glutamax (31331-08, Gibco) media, supplemented with B27 (12587-010, Gibco), 20 ng/ml human basic fibroblast growth factor (Miltenyi Biotec), 20 ng/ml human epidermal growth factor (Miltenyi Biotec) and 100 U/ml penicillin, 100 U/ml streptomycin. Cells were incubated in the incubator for about 10 days without moving, especially for the first 5 days. Immunostaining and flow cytometry analysis were performed as described in Example 3, using an alternative mouse monoclonal anti-integrin alpha10 antibody. 3D culture systems provide excellent in vitro models, allowing the study of cellular responses in a setting that resembles in vivo environments.

### Results:

The results from breast cancer experiments demonstrated that expression of integrin alpha10 was significantly increased when the aggressive triple negative breast cancer cells MDA-MB-231 and BT549 were cultured in in 3D conditions (spheres), which mimics tumor growth *in vitro* (Figure 5C-D). The non-malignant or less aggressive breast cell lines 184A1 and HCC1428, respectively, did not increase integrin alpha10 in sphere cultures. The aggressive cell line from prostate cancer, PC-3, also upregulated the protein expression of integrin alpha10 both (Figure 5G-H) when cells were grown in spheres (for comparison to monolayer cultures: Figure 5E-F). In agreement, the aggressive pancreatic cancer cells, PANC-1 and MiaPaCa-2 readily formed spheres and significantly increased protein expression of integrin alpha10 compared to monolayer cultures (Figure 5I-J). In contrast, the cell lines BxPC-3 and AsPC-1 did not increase expression integrin alpha10, and in addition the AsPC-1 did not form spheres in culture. The lung cancer cells A549 and U-1752 grown as spheres drastically increased the protein level of integrin alpha10 (Figure 5K-L). Similarly, the aggressive sarcoma cell line MFH152 readily formed spheres and significantly increased expression of integrin alpha10 at protein level compared to monolayer cultures (Figure 5M).

### Conclusion:

The results demonstrate that integrin alpha 10 expression is highest in the most aggressive cancer types. Sphere culture (3D cell culture), which is an excellent way to mimic tumor growth *in vivo*, further increases the integrin alpha10 expression in aggressive cancer cells. This provides additional support to the correlation between integrin alpha 10 and aggressive cancers.

### Example 5: Detection and analysis of integrin alpha10 mRNA (ITGA10) expression in human breast, prostate and pancreatic cancer cell lines cultured as monolayers or spheres

### Material and methods:

RNA extraction and quantitative PCR were conducted on different cell lines cultured either in monolayers or as spheres to mimic tumor growth. Total RNA was extracted from cells using RNeasy Plus mini kit (Qiagen) and reverse-transcribed into cDNA using a SuperScript cDNA Synthesis Kit (Life Technologies). For quantitative PCR, TaqMan Gene expression master mix (Life Technologies) and TaqMan probes (Thermo Fisher Scientific) were used: *GAPDH* (Mm99999915_g1) and *ITGA10* (Mm01265767_m1). Cycle threshold values of target genes were normalized to geometric mean of housekeeping genes *GAPDH* to get ΔCt. 2 to the power of -ΔCt (2^{-ΔCt}) was calculated for final analysis.

### Results:

The integrin alpha10 mRNA (*ITGA10*) level is higher in the more invasive and aggressive cancer cells, namely triple-negative breast cancer cells MDA-MB-231 and BT549 (Figure 6A-B), prostate cancer cells PC-3 (Figure 6C-D), and pancreatic cancer cells PANC-1 and MiaPaCa-2 (Figure 6E-F); compared to the less invasive and less aggressive cell lines, namely non-malignant breast cells 184A1 and breast cancer cells HCC1428 and T47D, prostate cancer cells 22RV1 and DU145, and pancreatic cancer cells BxPC-3 and AsPC-1, as shown in the respective figures. Furthermore, the integrin alpha10 (*ITGA10*) mRNA level significantly increased when the cells were cultured in sphere condition compared to monolayer condition (see Figures 6A vs. 6B, Figures 6C vs. 6D and Figures 6E vs. 6F).

### Conclusion:

The results demonstrate that integrin alpha10 mRNA expression is highest in the most aggressive cancer types. Sphere culture (3D cell culture) ,which is an excellent way to mimic tumor growth *in vivo*, further increases the integrin alpha10 expression in aggressive cancer cells. This provides additional support to the correlation between integrin alpha 10 and aggressive cancers.

### Example 6: Overall survival curves for ITGA10 gene expression in different cancer indications.

### Material and methods:

The overall survival curves for *ITGA10* gene expression in 255 triple-negative breast cancer patients (Gyorffy B et al, 2010) (A) was analyzed. The patients were divided into *ITGA10* low expression and *ITGA10* high expression cohorts by median cutoff. Kaplan-Meier plots were constructed and a log-rank test was used to determine differences among relapse free survival. The cutoff value used in the analysis was 224.

Overall survival curves for squamous cell lung carcinoma (Database GSE4573, 88 samples), prostate adenocarcinoma (Database TCGA-prostate adenocarcinoma, 413 samples ), localized pancreatic ductal adenocarcinoma (Database GSE211501, 102 samples), and sarcoma (Database TCGA-SARCOMA, 234 samples) were analyzed using PROGgeneV2 tool for high *ITGA10* expression and low *ITGA10* expression groups which were divide by the median value. The analysis allows the investigation of prognostic implications of gene expression associated with *ITGA10* in the corresponding microarray datasets. (Goswami CP et al, 2013)

### Results:

The survival curves show the overall survival differences between patients with high (line) or low (dashed line) *ITGA10* expression (Figure 7). Patients were divided into high and low *ITGA10* expression groups based on the median cut off for the survival analysis and log-rank test. P-value represents log-rank testing of the difference in overall survival. For all indicated cancer forms, survival is lower in the patient group expressing high *ITGA10*, compared to the patient group expressing low *ITGA10*.

### Conclusion:

High expression of *ITGA10* associates with lower overall survival in the indicated cancer types.

### Example 7: Inhibition on cell adhesion after blocking integrin alpha10 with antibodies

### Material and methods:

The day before the adhesion assay, 48-well plates were coated with collagen type I (Sigma, C7661-5 MG), collagen IV (Sigma, C5533-5MG) or bovine serum albumin (BSA). On the experiment day, plates were incubated with 0.25% BSA at 37 °C for 30 min to block non-specific binding. In parallel, carcinoma cells (breast cancer cells BT549 and prostate cancer cells PC-3) were harvested, suspended into a single cell suspension in HBSS. The cells were pre-incubated for 30 minutes in the presence or in the absence of the mouse monoclonal antibody mAbα directed to the integrin alpha10 polypeptide at the concentration of 10 µg/ml. As a negative control, the mouse monoclonal isotope control antibody IgG2a was as used. The cells were then allowed to attach for 60 min and unattached cells were removed by washing with HBSS. Adherent cells were fixed with 96% ethanol and stained by 0.1% crystal violet. Absorbance was measured by plate reader at a wavelength of 590 nm.

### Results:

Treatment of the breast BT549 cells with the function blocking monoclonal antibody against integrin alpha10 polypeptide significantly decreased cell adhesion to collagen I and IV for BT549 cells (Figure 8). This effect was seen when cells were cultured both in monolayer (Figure 8A-B) and as mammospheres (Figure 8C-D) and was compared to non-treated or IgG2a treated cells. Similarly, treatment with the function blocking monoclonal integrin alpha10 antibody significantly decreased cell adhesion to collagen I and IV of PC-3 cells cultured as spheres compared to the non-treated cells as control (Figure 8E-F). BSA-coated wells were used as a negative control because cell adhesion to extracellular matrix (ECM) is mediated through integrin receptors and cells will not attach to BSA-coated wells.

### Conclusion:

The results demonstrated that integrin alpha10 function blocking antibodies can block adhesion, and thereby likely affect critical tumor cell functions such as proliferation, migration and growth.

### Example 8: Reduced cell migration after blocking integrin alpha10 with a monoclonal antibody

### Material and methods:

Migration assays on breast cancer cells BT549, lung cancer cells A549 and prostate cancer PC-3 carcinoma cells were performed using Boyden Chambers containing polycarbonate filters with 8 µm pore size (Corning). Filters were coated with collagen collagen IV (Sigma, C5533-5 MG) for BT549 breast cancer cells and collagen type I (Sigma, C7661-5 MG) for PC-3 prostate cancer cells, or were uncoated for A549 lung cancer cells. For migration assays, a collagen working solution (0.01 mg/ml) was prepared from a stock solution (1 mg/ml) with PBS. The lower compartment was filled with 10% FBS medium as a chemoattractant and also contained monoclonal antibodies against integrin alpha10. Cancer cells were incubated with 5 µg/ml of the antibodies and incubated for 30 min before adding the cells to the upper compartment of the Boyden Chamber. The cells on the lower chambers were fixed after 24 h or 48 h and stained with crystal violet. OD 590 nm was measured in a plate reader (SpectraMax ABS, Molecular Devices).

Breast cancer cells migration assay: Cells were incubated with either mouse anti-integrin alpha10 monoclonal antibody (mAbα10) or human anti-integrin alpha10 Th101, binding to different epitopes of integrin alpha10 (see Example 12). As a negative control, cells were incubated with negative control antibody Th301 human IgG1 VH/Lambda or negative/isotope control antibody IgG2a (Mouse monoclonal IgG2a, κ), or any antibody treatment was omitted (NT=non-treated).

Prostate cancer cell migration assay: Cells were incubated with mouse anti-integrin alpha10 monoclonal antibody (mAbα10) and allowed to migrate for 24 or 48 hours. As a negative control, cells were incubated with isotope control antibody IgG2a.

Lung cancer migration assay: Cells were incubated with mouse anti-integrin alpha10 monoclonal antibody (mAbα10) for 24 or 48 hours. As a negative control, cells were incubated with isotope control antibody IgG2a.

### Results:

Breast cancer cells: Incubation with both monoclonal anti-integrin alpha10 antibodies mAbα10 or Th101 decreased migration of cells compared to non-treated cells or cells incubated with control antibodies (Figure 9A). Both anti-integrin alpha10 antibodies had a similar effect.

Prostate cancer cells: Incubation with the anti-integrin alpha10 antibody mAbα10 decreased migration of cells compared to cells incubated with the control antibody (Figure 9B). This effect was aggravated when the cells were assayed for 48 hours compared to 24 hours.

Lung cancer cells: Similarly, incubation with monoclonal anti-integrin alpha10 antibody mAbα10 decreased migration of cells compared cells incubated with the control antibody (Figure 9C).

### Conclusion:

The results demonstrate that monoclonal integrin alpha10 antibodies are capable of blocking integrin alpha10 and inhibit migration of breast and prostate cancer cells (Figure 9A and Figure 9B, respectively), and lung cancer cells (Figure 9C).

### Example 9: Decreased viability of breast cancer cells treated with integrin alpha10 antibody-drug conjugates

### Material and methods:

The breast cancer cells BT549 were cultured as monolayer in 96-well plates and treated with anti-integrin alpha10-MMAE ADC (antibody-drug conjugate or anti-control-MMAE ADC). The ADC is a conjugate of the alternative mouse monoclonal integrin alpha10 antibody (IgG1 (kappa)) or an isotype control IgG1 antibody (anti-ctrl) with the microtubule inhibitor Monomethyl auristatin E (MMAE). Cells were incubated at 37°C for 4 days with 23, 69 and 207 nM of the ADCs. The cell viability was determined by using the WST-1 assay (Roche, Mannheim, Germany), according to the manufacturer´s recommendation.

### Results:

The antibody-drug conjugate (ADC), composed of an integrin alpha10 antibody conjugated to the potent cytotoxin MMAE (anti-alpha 10-MMAE), induced cell death of the breast cancer cells (Figure 10). In contrast, no cell death was observed when the cells were treated with the isotype control IgG1 antibody conjugated to MMAE (anti-ctrl-MMAE). The higher the concentration of the antibody-drug conjugate, the lower the viability of the breast cancer cells.

### Conclusion:

The results demonstrate specific cytotoxic effect by the integrin alpha10 antibody conjugate.

### Example 10: Monoclonal alpha10 antibody treatment suppresses in vitro cell proliferation of aggressive breast, pancreatic and lung cancer cells

### Material and methods:

Sphere assay (Figure 11 A-D and F): Breast (BT549), prostate (PC-3), pancreatic (MiaPaCa-2 and PANC-1) and lung (A549) cancer cells were seeded in 6-well ultra-low attachment plates (CLS3471, Corning) in serum-free media to form non-adherent spheres. The cells were treated with either 10 µg/ml of monoclonal anti-integrin alpha10 antibody mAbα10 or control antibody (IgG2a) at the same time of seeding.

The antibody was added again every second day for 14 days. After 14 days of treatment, the BrdU (10 µM final concentration) was added to the spheres and the cells were incubated for 24 hours. The cells were then collected for the BrdU analysis according to the instruction from BD Pharmigen APC BrdU Flow Kits (Cat. No. 552598) through flow cytometry. The mean fluorescence intensity of BrdU staining was calculated. For lung cancer cells (A549), in addition of BrdU staining, 7-Aminoactinomycin D (7-AAD) was added to stain the total DNA. The cell cycle analysis was based on both BrdU and 7-AAD staining.

Monolayer assay (Figure 11 E): Adherent BT549 breast cancer cells were seeded in 96 well plates coated with collagen type IV and treated with monoclonal anti-integrin alpha10 antibodies (mAbα10 or Th101) to get final concentration of 5 µg/ml directly after cell seeding. After 24 hours of treatment with antibodies BrdU was added for 2.5 hours and proliferation was measured using the Cell Proliferation ELISA BrdU kit (Roche Diagnostics GmbH), according to the manufacturer's instructions.

Cell cycle analysis (Figure 11 F): The immunofluorescent staining of incorporated bromodeoxyuridine (BrdU) and flow cytometric analysis was used to determine the frequency and nature of individual cells that have synthesized DNA. In this method, BrdU (an analog of the DNA precursor thymidine) is incorporated into newly synthesized DNA by cells entering and progressing through the S (DNA synthesis) phase of the cell cycle. The incorporated BrdU is stained with specific anti-BrdU fluorescent antibodies. The levels of cell-associated BrdU are then measured by flow cytometry. Often, staining with a dye that binds to total DNA such as 7-aminoactinomycin D (7-AAD) is coupled with immunofluorescent BrdU staining. With this combination, two-color flow cytometric analysis permits the enumeration and characterization of cells that are actively synthesizing DNA (BrdU incorporation) in terms of their cell cycle position (i.e. G0/G1, S, or G2+M phase defined by 7-AAD staining intensities).

### Results:

Sphere assay: The results demonstrate that the function blocking monoclonal alpha10 antibody reduces proliferation of the aggressive breast (Figure 11A), prostate (Figure 11B) and pancreatic cancer cells (Figure 11C-D). This was compared to proliferation after treatment with the control antibody IgG2a, which in contrast showed no reduction in proliferation. The cell cycle analysis showed that treatment with monoclonal alpha10 antibody inhibited cell proliferation by arresting the cells in G0/G1 phase comparing to the control antibody IgG2a treatment in lung cancer cell line A549 (Figure 11F).

Monolayer assay: Figure 11E shows that anti-integrin alpha10 antibodies (mAbα10 and Th101) blocked cell proliferation of monolayer-cultured breast cancer cells, compared to incubation with control antibodies or no antibody.

### Conclusion:

These results confirm the involvement of the integrin alpha10 in cell proliferation processes, thereby indicating that an anti-integrin alpha10 antibody has potential as an anti-proliferative cancer treatment.

### Example 11: Treatment with integrin alpha10 antibodies suppress progression of breast tumor growth in vivo.

### Material and methods:

All *in vivo* experiments were performed in female NMRI-nu immunodeficient mice at the age 6-8 weeks (n = 5 per group) purchased from Janvier Labs (France). Animal welfare and experimental procedure were carried out in accordance with international standards and animals were maintained under specific pathogen-free (SPF) conditions.

All experimental procedures were approved by the Malmö and Lund Animal Ethics Committee (Sweden). For the induction of tumors, mice were inoculated with 2x10⁶ aggressive breast cancer BT549 cells in Matrigel by subcutaneous injection into the right flank regions. Two to three weeks post injection the tumor growth was monitored by non-invasive 2D bioluminescence (BLI) imaging, using IVIS-CT spectrum (PerkinElmer, MA, USA). Mice showing tumor growth signals were randomized into two groups (control and treatment) based on their average BLI signal intensity recorded in a defined region of interest (ROI) with average total flux (photons/s) values. Different antibodies against integrin alpha10 were used in this study: mouse monoclonal mAb α10 and human monoclonal antibody Th101. The concentration of each antibody injected to mice was calculated to 5 mg/kg and animal received antibodies via intraperitoneal injection up to the end points. Tumor growth was monitored using bioluminescence 2D and 3D microCT imaging. Briefly, mice were anesthetized with 3% isoflurane gas and injected intraperitoneally with 150 mg D-Luciferin/kg of the body weight in PBS prior to imaging. Acquisition of 2D images were taken sequentially with five intervals between different segments of exposures (Emission: open filter, f/stop: 1, binding: 8). BLI signal intensity was quantified in total flux (photons/s) after deducting the average background signal (Bkg) from measurement region of interest (ROI) using the live image analysis software (PerkinElmer, MA, USA). The weight of the mice was recorded each week before the treatment with antibodies. No sign of sickness or reduced growth rate could be observed.

### Results:

The results demonstrate that the total flux readout was decreased in mice treated with the integrin alpha10 antibodies mAbα10 or Th101 compared to the mice treated with negative control antibodies (IgG2a and Th301) (Figure 12A). By that it was shown that the tumour growth was decreased. The weight of animals was monitored from the onset of the treatment, and no sign of sickness nor weight loss in animals treated with respective antibodies could be observed. (Figure 12B).

### Conclusion:

The results show that different antibodies against integrin alpha10, here mAbα10 (=Ab365) or Th101, can reduce tumor growth *in vivo*, thereby suggesting the usefulness of an anti-integrin alpha10 antibody as an anti-proliferative cancer treatment.

### Example 12: Monoclonal antibody mAbα10 and Th101 bind different epitopes of integrin alpha10.

### Material and methods:

The binding competition assay of the antibody mouse monoclonal antibody mAbα10 and the human monoclonal antibody Th101 was performed in integrin alpha10 overexpressing C2C12α10 cells and triple-negative breast cancer BT549 cells. The cells were incubated with single or mixed antibodies at the indicated concentrations (µg/ml) for 30 min followed by two washed, and then stained with secondary antibodies for 30 min. Donkey anti-human Alexa 488 was used as a secondary antibody to the human monoclonal antibody Th101. Donkey anti-mouse Alexa 647 was used as a secondary antibody to the mouse monoclonal antibody mAba10. The binding of the antibodies was analyzed by flow cytometry, wherein the fluorescence signal of the secondary antibodies were detected.

Data are expressed in mean fluorescence intensity of 100 000 cells.

### Results:

The flow cytometry data show that the binding intensity of the antibody mAbα10 was not significantly changed by the increased concentrations of Th101 antibody (Figure 13 A-B). With increasing concentrations (0 to 9 µg/ml) of antibody Th101 added to the reaction wells (grey bars), the signal representing antibody mAbα10 (at constant concentration of 3 µg/ml) remained stable (black bars) (left part of the figures).

Vice versa, the binding intensity of Th101 was not affected by the increased concentrations of mAbα10 (Figure 13 A-B). With increasing concentrations (0 to 9 µg/ml) of antibody mAbα10 added to the reaction wells (black bars), the signal representing antibody Th101 ( a constant concentration of 3 µg/ml) remained stable (grey bars) (right part of the figures).

Similar results were obtained for both cell lines; C2C12α10 cells (Figure 13A) and BT549 cells (Figure 13B).

### Conclusion:

It the antibodies would bind to the same epitope, then increasing concentrations of one antibody would lead to less binding of the other antibody, due to a competition for the same binding site. Since this is not the case, the results suggest that the two monoclonal integrin alpha10 antibodies mAbα10 and Th101 bind different epitopes of integrin alpha10 antigen.

### Example 13: Sequences

### Sequence information of human integrin alpha10 polypeptide

SEQ ID NO: 1
   Human integrin alpha10 full length polypeptide
   See sequence list.
SEQ ID NO: 2
   Extracellular domain of human integrin alpha10
   See sequence list.
SEQ ID NO: 3
   I-domain of human integrin alpha10
   See sequence list.

### Sequence information of anti-integrin alpha10 antibody Th101

SEQ ID NO: 4:
   Variable heavy chain complementarity-determining region 1 (CDR-H1)
   FTFSDYGMN
SEQ ID NO: 5:
   Variable heavy chain complementarity-determining region 2 (CDR-H2)
   VISYDGSNKYYADSVKG
SEQ ID NO: 6:
   Variable heavy chain complementarity-determining region 3 (CDR-H3)
   GGNWGVFDY
SEQ ID NO: 7:
   Variable light chain complementarity-determining region 4 (CDR-L1)
   SGSSSNIGSNPVH
SEQ ID NO. 8:
   Variable light chain complementarity-determining region 5 (CDR-L2)
   ENNKRPS
SEQ ID NO. 9:
   Variable light chain complementarity-determining region 6 (CDR-L3)
   AAWDDSLSGQGV
SEQ ID NO. 10:
   Heavy chain variable region 
EVQLLESGGGLVQPGGSLRLSCAASGFTFSDYGMNWVRQAPGKGLEWVAVISYDGSNKYYADSV
KGRFTISRDNSKNTLYLQMNSLRAEDTAVYYCARGGNWGVFDYWGQGTLVTVSS
SEQ ID NO. 11:
   Light chain variable region

QSVLTQPPSASGTPGQRVTISCSGSSSNIGSNPVHWYQQLPGTAPKLLIYENNKRPSGVPDRFS
GSKSGTSASLAISGLRSEDEADYYCAAWDDSLSGQGVFGGGTKLTVLG

### References

Arpino G, Milano M, De Placido S. Features of aggressive breast cancer, The Breast. 2015;24(5): 594-600.
Bengtsson T, Aszodi A, Nicolae C, Hunziker EB, Lundgren-Akerlund E, Fässler R. Loss of alpha10beta1 integrin expression leads to moderate dysfunction of growth plate chondrocytes, J Cell Sci. 2005;118(Pt 5):929-36.
Bianchini G, Balko JM, Mayer IA, et al. Triple-negative breast cancer: challenges and opportunities of a heterogeneous disease. Nat Rev Clin Oncol 2016;13:674-90.
Bombardelli L and Berns A. The steady progress of targeted therapies, promising advances for lung cancer, Ecancermedicalscience 2016;10:638.
Camper L, Hellman U, Lundgren-Akerlund. Isolation, Cloning, and Sequence Analysis of the Integrin Subunit α10, a β1-associated Collagen Binding Integrin, Expressed on Chondrocytes, J Biol Chem 1998; 273(32):20383-9.
Camper L, Holmvall K, Wängnerud C, Aszódi A, Lundgren-Akerlund E. Distribution of the collagen-binding integrin alpha10beta1 during mouse development, Cell Tissue Res. 2001;306(1):107-16.
Dai X, Li T, Bai Z, Yang Y, Liu X, Zhan J, Shi B. Breast cancer intrinsic subtype classification, clinical use and future trends, Am J Cancer Res. 2015;5(10):2929-2943.
Dai X, Xiang L, Li T, Bai Z. Cancer Hallmarks, Biomarkers and Breast Cancer Molecular Subtypes, Journal of Cancer. 2016;7(10):1281-94.
Einstein DJ, Arai S, Balk SP. Targeting the androgen receptor and overcoming resistance in prostate cancer, Curr Opin Oncol. 2019; 31(3):175-18.
Goswami CP and Nakshatri H. PROGgene: gene expression based survival analysis web application for multiple cancers. J Clin Bioinforma. 2013 Oct 28;3(1):22.
Gyorffy B, Lanczky A, Eklund AC, Denkert C, Budczies J, Li Q, Szallasi Z. An online survival analysis tool to rapidly assess the effect of 22,277 genes on breast cancer prognosis using microarray data of 1809 patients, Breast Cancer Res Treatment, 2010 Oct;123(3):725-31.
Hariri N, Hasteh F, Walavalkar V, Roma AA, and Fadare O. Estrogen receptor, progesterone receptor, and human epidermal growth factor receptor-2 testing in breast cancer: assessing the value of repeated centralized testing in excision specimens, Appl Immunohistochem Mol Morphol. 2019; 27(1):1-7.
Ilic M, Ilic I. Epidemiology of pancreatic cancer. World Journal of Gastroenterology 2016; 44:9694-9705.
Lundgren-Åkerlund E, Aszòdi A. α10β1: a collagen receptor critical in skeletal development, Adv Exp Med Biol. 2014; 819:61-71.
Ma L, Liang Z, Zhou H, Qu L. Applications of RNA Indexes for Precision Oncology in Breast Cancer, Genomics Proteomics Bioinformatics. 2018;16(2):108-119.
Malorni L, Shetty PB, De Angelis C, et al. Clinical and biologic features of triple-negative breast cancers in a large cohort of patients with long-term follow-up. Breast Cancer Res Treat 2012;136:795-804.
Moschos SJ, Drogowski LM, Reppert SL, Kirkwood JM. Integrins and Cancer, Oncology. 2007 Aug;21(9 Suppl 3):13-20.
Rakha EA, El-Sayed ME, Green AR, Lee AH, Robertson JF, Ellis IO Prognostic markers in triple-negative breast cancer. Cancer. 2007 1; 109(1):25-32
Saad F, Shore N, Zhang T, Sharma S, Cho HK, Jacobs IA. Emerging therapeutic targets for patients with advanced prostate cancer. Cancer Treat Rev. 2019;19;76:1-9.
U.S. National Institute of Health, National Cancer Institute. SEER Cancer Statistics Review, 1975-2015.
Varas L, Ohlsson LB, Honeth G, Olsson A, Bengtsson T, Wiberg C, Bockermann R, Järnum S, Richter J, Pennington D, Johnstone B, Lundgren-Akerlund E, Kjellman C. Alpha10 integrin expression is up-regulated on fibroblast growth factor-2-treated mesenchymal stem cells with improved chondrogenic differentiation potential, Stem Cells Dev. 2007;16(6):965-78.
WHO classification of tumors of soft tissue and bone (2013).

## Claims

1. An antibody or antigen-binding fragment thereof, wherein the antibody or antigen-binding fragment thereof is specifically binding to an integrin alpha10 polypeptide extracellular domain, for use in a method of treatment of a cancer selected from the group consisting of pancreatic cancer, sarcoma, squamous cell lung carcinoma, lung adenocarcinoma, large cell lung carcinoma, small-cell lung carcinoma and prostate cancer, or a metastasis of any of the cancers, wherein the integrin alpha10 polypeptide is expressed on the surface of a malignant cell and/or a tumor-associated cell, and wherein the antibody or antigen-binding fragment thereof is capable of inducing cell death and/or inhibiting growth and/or inhibiting proliferation and/or inhibiting migration of cancer cells expressing an integrin alpha10 polypeptide.

2. The antibody or antigen-binding fragment thereof for use according to claim 1, wherein the cancer is pancreatic cancer or a metastasis thereof, and wherein the pancreatic cancer is a grade I, grade II or grade III pancreatic cancer.

3. The antibody or antigen-binding fragment thereof for use according to claim 1 or 2, wherein the pancreatic cancer is a pancreatic ductal adenocarcinoma or a pancreatic neuroendocrine tumor.

4. The antibody or antigen-binding fragment thereof for use according to claim 1, wherein the cancer is sarcoma or a metastasis thereof, and wherein the sarcoma is selected from the group consisting of undifferentiated pleomorphic sarcoma, myxofibrosarcoma, dedifferentiated liposarcoma, atypical lipomatous tumor, myxoinflammatory fibroblastic sarcoma, low grade fibromyxoid sarcoma, sclerosing epithelioid fibrosarcoma, pseudomyogenic hemangioendothelioma and mesenchymal chondrosarcoma.

5. The antibody or antigen-binding fragment thereof for use according to claim 1, wherein the cancer is prostate cancer or a metastasis thereof, and wherein the prostate cancer is a small cell neuroendocrine carcinoma (SCNC).

6. The antibody or antigen-binding fragment thereof for use according to any one of the preceding claims, wherein the malignant cell and/or tumor-associated cell is a cancer associated fibroblast (CAF), a stromal cell, a stem cell and/or a stem-like cell.

7. The antibody or antigen-binding fragment thereof for use according to any one of the preceding claims, wherein the method of treatment comprises inhibiting proliferation, inhibiting self-renewal and/or inhibiting anchorage-independent growth of cancer cells of the cancer.

8. The antibody or antigen-binding fragment thereof for use according to any one of the preceding claims, wherein the antibody is: a) a monoclonal antibody produced by the hybridoma cell line deposited at the Deutsche Sammlung von Mikroorganismen und Zellkulturen GmbH under the accession number DSM ACC2583; or b) a fragment of a), wherein the fragment is capable of binding specifically to the extracellular I-domain of the integrin alpha10 polypeptide chain.

9. The antibody or antigen-binding fragment thereof for use according to any one of the preceding claims, wherein the antibody or antigen-binding fragment comprises a heavy chain variable region comprising a) a CDR-H1 comprising or consisting of the amino acid sequence of SEQ ID NO: 4; b) a CDR-H2 comprising or consisting of the amino acid sequence of SEQ ID NO: 5; and c) a CDR-H3 comprising or consisting of the amino acid sequence of SEQ ID NO: 6; and/or a light chain variable region comprising d) a CDR-L1 comprising or consisting of the amino acid sequence of SEQ ID NO: 7; e) a CDR-L2 comprising or consisting of the amino acid sequence of SEQ ID NO: 8; and f) a CDR-L3 comprising or consisting of the amino acid sequence of SEQ ID NO: 9, or a variant of any one of SEQ ID NO:s 4 to 9, wherein any one amino acid has been altered for another amino acid, with the proviso that no more than 3 amino acids have been so altered, for example wherein 2, or 1 amino acids have been so altered.

10. The antibody or antigen-binding fragment thereof for use according to any one of the preceding claims, wherein the antibody or antigen-binding fragment comprises a heavy chain variable region comprising or consisting of the amino acid sequence of SEQ ID NO: 10, and/or a light chain variable region comprising or consisting of the amino acid sequence of SEQ ID NO: 11.

11. The antibody or antigen-binding fragment thereof for use according to any one of the preceding claims, wherein the antibody or antigen-binding fragment thereof is conjugated to an additional moiety; such as wherein the additional moiety comprises a detectable moiety and/or a cytotoxic moiety.

12. An antibody or antigen-binding fragment thereof, wherein the antibody or antigen-binding fragment thereof is specifically binding to an integrin alpha10 polypeptide extracellular domain, for use in a method of diagnosis of a cancer selected from the group consisting of pancreatic cancer, sarcoma, squamous cell lung carcinoma, lung adenocarcinoma, large cell lung carcinoma, small-cell lung carcinoma and prostate cancer, or a metastasis thereof, wherein the antibody or antigen-binding fragment thereof is used to determine the expression level of an integrin alpha10 polypeptide or fragment thereof on the cell surface, and wherein an expression level higher than a control level, the control level being the average expression level of the integrin alpha10 polypeptide observed in healthy and/or benign tissue of the same tissue type, is indicative of the cancer selected from the group consisting of pancreatic cancer, sarcoma, squamous cell lung carcinoma, lung adenocarcinoma, large cell lung carcinoma, small-cell lung carcinoma and prostate cancer, or the metastasis thereof.

13. A method for detecting a cancer selected from the group consisting of pancreatic cancer, sarcoma, squamous cell lung carcinoma, lung adenocarcinoma, large cell lung carcinoma, small-cell lung carcinoma and prostate cancer, or a metastasis thereof, in a subject, the method comprising the steps of: a. analyzing in a tumor tissue sample the presence of one or more cells having a cancer morphology; b. analyzing in the tissue the presence of an antigen comprising an integrin alpha10 polypeptide or a fragment thereof by using an antibody or antigen-binding fragment thereof specifically binding to an integrin alpha10 polypeptide extracellular domain; and c. determining the expression level of the integrin alpha10 antigen and comparing the expression level to a control level, wherein the control level is the average expression level of the antigen observed in healthy and/or benign tissue of the same tissue type; wherein the presence of one or more cells having a cancer morphology in combination with an expression level of the integrin alpha10 antigen higher than the control level is indicative of the presence of the cancer selected from the group consisting of pancreatic cancer, sarcoma, squamous cell lung carcinoma, lung adenocarcinoma, large cell lung carcinoma, small-cell lung carcinoma and prostate cancer, or the metastasis thereof, in the subject.

14. A method for diagnosis of a cancer selected from the group consisting of pancreatic cancer, sarcoma, squamous cell lung carcinoma, lung adenocarcinoma, large cell lung carcinoma, small-cell lung carcinoma and prostate cancer, or a metastasis thereof, in a subject, the method comprising the steps of: a. analyzing in a tumor tissue sample the presence of one or more cells having a cancer morphology; b. analyzing in the sample the presence of an antigen comprising an integrin alpha10 polypeptide or a fragment thereof by using an antibody or antigen-binding fragment thereof specifically binding to an integrin alpha10 polypeptide extracellular domain; and c. determining the expression level of the integrin alpha10 antigen and comparing the expression level to a control level, wherein the control level is the average expression level of the antigen observed in healthy and/or benign tissue of the same tissue type; wherein the presence of one or more cells having a cancer morphology in combination with an expression level of the integrin alpha10 antigen higher than the control level is indicative of the presence of the cancer selected from the group consisting of pancreatic cancer, sarcoma, squamous cell lung carcinoma, lung adenocarcinoma, large cell lung carcinoma, small-cell lung carcinoma and prostate cancer, or the metastasis thereof, in the subject.

15. A method for determining a prognosis for a cancer selected from the group consisting of pancreatic cancer, sarcoma, squamous cell lung carcinoma, lung adenocarcinoma, large cell lung carcinoma, small-cell lung carcinoma and prostate cancer, or a metastasis thereof for a subject, the method comprising: a. analyzing a tumor tissue sample for the presence of one or more cells having a cancer morphology; b. analyzing in the sample the presence of an antigen comprising an integrin alpha10 polypeptide or a fragment thereof by using an antibody or antigen-binding fragment thereof specifically binding to an integrin alpha10 polypeptide extracellular domain; c. determining the expression level of the integrin alpha10 antigen and comparing the determined expression level to a control level, wherein the control level is the average expression level of the antigen observed in healthy and/or benign tissue of the same tissue type as the sample; and d. determining an unfavorable prognosis of the cancer selected from the group consisting of pancreatic cancer, sarcoma, squamous cell lung carcinoma, lung adenocarcinoma, large cell lung carcinoma, small-cell lung carcinoma and prostate cancer, or the metastasis thereof, when one or more cells having a cancer morphology are present in the tissue in combination with an expression level of the integrin alpha10 antigen higher than the control level.
